# EUROPEAN PATENT APPLICATION

(11) **EP 1 775 283 A1**
(43) Date of publication of application: **18.04.2007**
(21) Application number: 05766175.3
(22) Date of filing: 14.07.2005
(51) Int. Cl.: C07C 237/40, A61K 31/167, A61K 31/44, A61K 31/538, A61K 31/5383, A61P 1/00, A61P 1/04, A61P 9/10, A61P 11/10, A61P 11/06, A61P 13/00, A61P 13/02, A61P 17/00, A61P 25/00, A61P 25/04, A61P 25/06, A61P 27/16, A61P 29/02, A61P 37/08

(54) **3-AMINOBENAMIDE COMPOUND AND VANILLOID RECEPTOR 1 (VR1) ACTIVITY INHIBITOR**

(30) Priority: 14.07.2004 JP 2004207448; 22.07.2004 US 590141 P
(71) Applicant: Japan Tobacco, Inc., Tokyo 105-8422 (JP)
(72) Inventor: YATA, Shinji, Central Pharmaceutical Research, Takatsuki-shi, Osaka 569-1125 (JP); KOGA, Yoshihisa, Central Pharmaceutical Research, Takatsuki-shi, Osaka 569-1125 (JP); WATANABE, Takashi, Central Pharmaceutical Research, Takatsuki-shi, Osaka 569-1125 (JP); MATSUO, Takuya, Central Pharmaceutical Research, Takatsuki-shi, Osaka 569-1125 (JP); SAKATA, Masahiro, Central Pharmaceutical Research, Takatsuki-shi, Osaka 569-1125 (JP); KONDO, Wataru, Central Pharmaceutical Research, Takatsuki-shi, Osaka 569-1125 (JP)
(74) Representative: Vossius & Partner
(86) International application number: PCT/JP2005/013445
(87) International publication number: WO 2006/006740

(57) **Abstract**

The present invention relates to a novel 3-aminobenzamide compound represented by the following formula which effectively inhibits vanilloid receptor subtype 1 (VR1) activity (wherein, for example, R¹ is a C1-6 alkyl group which may be substituted, R² is a hydrogen atom, a C1-6 alkyl group or a C1-6 alkoxy group which may be substituted, R³ is a hydrogen atom or a C1-6 alkyl group, R⁴ is a C1-6 alkyl group, a C1-6 alkoxy group, or a halo C1-6 alkyl group, m is an integer of 1 to 5 and P is a carbon or hetero ring) or a pharmaceutically acceptable salt thereof. The pharmaceutical composition comprising as active ingredients the 3-aminobenzamide compound or a pharmaceutically acceptable salt thereof is useful for treating diseases involved in VR1 activity such aspain, acutepain, chronicpain, neuropathic pain, rheumatoid arthritis pain, and neuralgia.

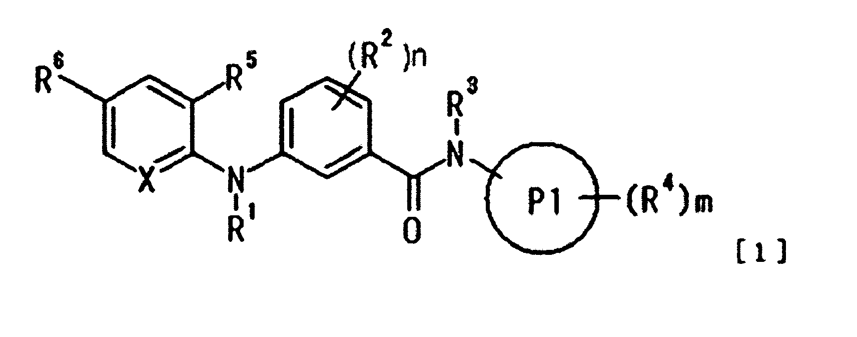

## Description

### TECHNICAL FIELD

The present invention relates to a novel 3-aminobenzamide compound having an inhibitory effect on vanilloid receptor subtype 1 (VR1) activity, and a pharmaceutical composition comprising the compound as an active ingredient, particularly a remedy of a disease associated with pain.

### BACKGROUND ART

Capsaicin, which is the main ingredient of red pepper, is a pungency causing ingredient as well as a pain producing substance. It has been reported that many nociceptive nerves, particularly unmyelinated C fibers have capsaicin sensitivity and it is known that C fibers will selectively drop out when capsaicin is administered to an infant rodent. It has been also reported that there are many sites of action for capsaicin distributed in the skin, cornea, and oral mucosa, and the distribution thereof is also observed in the muscles, joints and internal organs, particularly in the cardiovascular system, respiratory system and bladder urinary tract system, and it is important for autonomic nerve reflex. In addition, capsaicin sensitivity is also observed in the nerves of the preoptic area of the thalamus, and involvement in the regulation of body temperature is presumed. Depolarization by inflow of Na⁺ and Ca²⁺ by capsaicin administration is observed in the nociceptive nerves and discharge of glutamic acid and neuropeptides (mainly Substance P and calcitonin gene-related peptide) from the center side end of the primary afferent fiber of the spinal dorsal horn is resulted. Now that specific binding activity of resiniferatoxin (RTX) which brings about similar effects to that of capsaicin has been observed, and that capsazepine has been revealed as a competitive inhibitor, liposoluble capsaicin is considered to act on receptor protein (see, for example, Szallasi A, Blumberg PM. (1999) Pharmacol. Rev. 51, 159-212).

The capsaicin receptor gene was cloned in 1997 (see, for example, Non-Patent Document 2). It was presumed from its amino acid sequence that it was an ion channel having a six-transmembrane domain. Since capsaicin has a vanillyl group in the structure, it is generically referred to as vanilloids along with its analogs such as RTX, and the cloned receptor was named vanilloid receptor subtype 1 (hereinafter referred to as VR 1; This VR1 may be also referred to as TRPV1 (transient receptor potential vanilloid receptor 1)). Then, electrophysiological functional analysis using the patch clamping method has been performed by making oocytes of Xenopus laevis and human derived cultured cells to express VR1, and it has been revealed that VR1 is directly activated by capsaicin, without mediated by an intracellular second messenger (see, for example, Szallasi A, Blumberg PM. (1999) Pharmacol. Rev. 51, 159-212, and that VR1 is a non-selective cation ion channel having high Ca²⁺ permeability with an outward rectification property (see, for example, Premkumar LS, Agarwal S, Steffen D. (2002) J. Physiol. 545, 107-117).

Although capsaicin is a pain causing substance, it is used as an analgesic agent to mitigate pain in diabetic neuropathy or rheumatic neurosis (see, for example, Szallasi A, Blumberg PM. (1999) Pharmacol. Rev. 51, 159-212). It is understood that such mitigation is resulted from a phenomenon that the sensory nerve end exposed to capsaicin stops answering to pain stimulus, that is, desensitization. Although it is considered that the desensitization mechanism of VR1 involves Ca²⁺-mediated regulation, regulation depending on potential, activity control of VR1 by phosphorylation and dephosphorylation, etc., many points remain unclear.

As well as capsaicin, heat and acid also cause pain and it is known that the capsaicin sensitive nociceptive nerves respond to two or more types of stimulation. It was found that VR1 was directly activated by not only capsaicin but heat stimulation of 43?C or more (see, for example, Yang D, Gereau RW 4th. (2002) J. Neurosci. 22, 6388-6393) . The temperature of 43?C is mostly in agreement with the temperature threshold which causes a pain in humans and animals, suggesting that VR1 participates in nociceptive heat stimulation receptance.

Acidification occurs in an organ in the case of inflammation or ischemia and it is known to cause or enhance pain (see, for example, Bevan S, Geppetti P. (1994) Trends Neurosci. 17, 509-512). It has turned out that when the pH outside cells is reduced within the limits of the acidification which takes place in the case of an organ lesion, VR1 can be directly activated by the acidification (proton) alone, and it is surmised that VR1 is the actual molecule which receives stimulation by acidification in an organ which takes place in the case of inflammation or ischemia (see, for example, Yang D, Gereau RW 4th. (2002) J. Neurosci. 22, 6388-6393).

Immunohistological analysis using a specific antibody has confirmed that the number of unmyelinated C fibers expressing VR1 increases in an inflamed region as compared in a normal region (see Carlton SM, Coggeshall RE. (2001) Neurosci. Lett. 310, 53-56). The enhancement of VR1 expression in submucosal plexus has been actually observed in human inflammatory bowel disease (see, for example, Yiangou Y, Facer P, Dyer NH, Chan CL, Knowles C, Williams NS, Anand P. (2001) Lancet 357, 1338-1339). Such an increase in the amount of VR1 expression causes peripheral sensitization in an inflamed organ and presumably contributes to duration of inflammatory hyperalgesia.

It has been also reported that extracellular ATP, bradykinin and a neuro growth factor which are inflammation related substances increase VR1 activity (see, for example, Tominaga M, Wada M, Masu M. (2001) Proc. Natl. Acad. Sci. USA 98, 6951-6956; Shu X, Mendell LM. (1999) Neurosci. Lett. 274, 159-162; Chuang HH, Prescott ED, Kong H, Shields S, Jordt SE, Basbaum AI, Chao, MV, Julius D. (2001) Nature 411, 957-962, Sugiura T, Tominaga M, Katsuya H, Mizumura K. (2002) J. Neurophysiol. 88, 544-548) and it is said to be a fact without doubt that VR1 involves in pain and hypersensitivity of pain including those caused by inflammation (see, for example, Numazaki M, Tominaga M (2003) Biochemistry 75, 359-371).

The sensory nerve cells in a VR1-deficient mouse responded to none of capsaicin, proton and heat stimulation. It is also reported that in action analysis, VR1-deficient mouse does not show the pain reaction following capsaicin administration, and sensitivity to heat stimulation decreases and inflammatory hyperalgesia is not observed (see, for example, Caterina MJ, Leffler A, Malmberg AB, Martin WJ, Trafton J, Peterson-Zeitz KR, Koltzenburg M, Basbaum AI, Julius D. (2000) Science 288, 306-313 and Davis LB, Gray J, Gunthorpe MJ et al. (2000) Nature 405, 183-187). Thus, it has been confirmed also on an individual level from the analysis of VR1-deficient mouse that VR1 functions as a wide range pain stimulation receptor.

Moreover, as for the relation between vanilloid receptor subtype 1 (VR1) and a disease, it has been reported already that a substance which inhibits VR1 activity is useful as a therapeutical agent of various diseases.

Particularly with regard to a therapeutical agent of pain, there is a report that capsazepine which is known as a VR1 antagonist has exhibited a significant analgesic effect in an animal model (see, for example, Ikeda Y, Ueno A, Naraba H, Oh-ishi S, (2001) Life Science 69, 2911-2919), and use is expected as a new therapeutical agent of pain having an inhibitory effect of VR1 activity.

It has been confirmed with regard to bladder hyperstrain type frequent urination and urinary incontinence that the bladder contraction function of VR1-deficient mouse decreases and there is a report that a compound having a capsaicin-like pharmacological mechanism or a compound having an inhibitory action on VR1, i.e., a compound inhibiting vanilloid receptor subtype 1 (VR1) activity is useful for improving bladder function, for example, as a therapeutical agent of frequent urination, urinary incontinence, etc (see, for example, (2002) Nat. Neurosci. 5, 856-860).

In addition, another reference reports that a substance having an inhibitory effect to the vanilloid receptor (VR1), particularly antagonist of VR1 receptor is useful for preventing and treating diseases related to VR1 activity, particularly urgent urinary incontinence, overactive bladder, chronic pain, neuropathic pain, postoperative pain, rheumatoid arthritis pain, neuralgia, neuropathy, hyperalgesia, nerve damage, ischemic symptom, neurodegenerative, cerebral apoplexy, incontinence, inflammatory disease, urgent urinary incontinence (UUI) and/or conditions and diseases including overactive bladder (see, for example, Japanese Patent Laid-Open No. 2003-192673).

Furthermore, it is also known that diseases relevant to the vanilloid receptor activity may include pain, acute pain, chronic pain, neuropathic pain, postoperative pain, migraine, jointpain, neuropathy, nerve damage, diabetic nervous disease, neurodegenerative disease, neurogenic skin disorder, cerebral apoplexy, bladder hypersensitivity, irritable bowel syndrome, abnormalities in respiratory organs such as asthma and chronic obstructive pulmonary disease, stimulation of skin, eye or mucosa, fever, stomach or duodenal ulcer, inflammatory bowel disease, inflammatory disease, etc (see, for example, National Publication of International Patent Application No. JP2004-506714T2).

Accordingly, it can be said that substances having vanilloid receptor (VR1) antagonistic activity is useful as a therapeutic agent for conditions in which C fibers participates, for example, not to mention pruritus, allergic and allergic rhinitis, overactive bladder type frequent urination and urinary incontinence, apoplexy, irritable bowel syndrome, respiratory ailment such as asthma and chronic obstructive pulmonary disease, dermatitis, mucositis, stomach and duodenal ulcer, inflammatory bowel disease, etc. but also pain, acute pain, chronic pain, neuropathic pain, postoperative pain, migraine, joint pain, neuropathy, nerve damage, diabetic nervous disease, neurodegenerative disease, rheumatoid arthritis pain, neuralgia, neuropathy, hyperalgesia, neurogenic skin disorder, apoplexy, urgent urinary incontinence, ischemic symptom and an inflammatory disease, etc.

Next, compounds considered to relatively resemble the known vanilloid receptor (VR1) antagonist and the compound of present invention are described.

The following compounds are known as compounds having a structure similar to that of the present invention.

For example, JP9059236 discloses that the following piperidine compound is useful as an anti-allergy agent.

However, the piperidine structure of this compound differs from the structure of the present invention. In addition, there is no disclosure that this compound has an inhibitory effect on VR1 receptor activity as in the present invention nor this compound is useful as a therapeutic agent and/or prophylactic agent of pain as in the present invention (see Japanese Patent Laid-open Publication No. 09-059236).

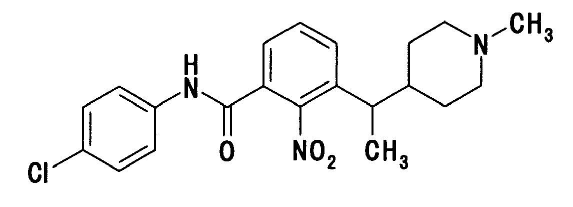

WO04/009552 and WO02/074726 also disclose that the following compound is useful as a PDE4 inhibitor.

However, substituting position of alkoxy group on the phenyl group of this compound is different from the structure of the present invention. In addition, there is no disclosure that this compound has an inhibitory effect on VR1 receptor activity as in the present invention nor this compound is useful as a therapeutic agent and/or prophylactic agent of pain as in the present invention.

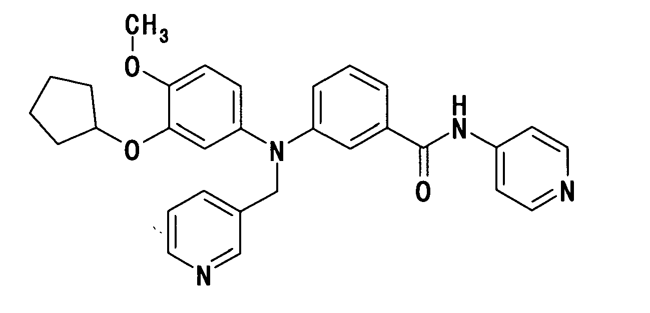

US3773936A also discloses that the following compound is useful as an anti-inflammatory agent.

However, this compound has no substituents other than a hydrogen atom in the phenyl group of N-phenylacetamide structure, and is different from the structure of the present invention. In addition, there is no disclosure that this compound has an inhibitory effect on VR1 receptor activity as in the present invention nor this compound is useful as a therapeutic agent and/or prophylactic agent of pain as in the present invention (for example, see US3773936A, US3678094A, Japanese Patent Publication No. 48-42054).

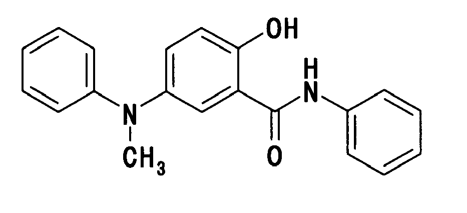

WO02/064545 also discloses that the following benzimidazole compound is useful as an NOS inhibitor.

However, the benzimidazole structure of this compound is different from the structure of the present invention. In addition, there is no disclosure that this compound has an inhibitory effect on VR1 receptor activity as in the present invention nor this compound is useful as a therapeutic agent and/or prophylactic agent of pain as in the present invention.

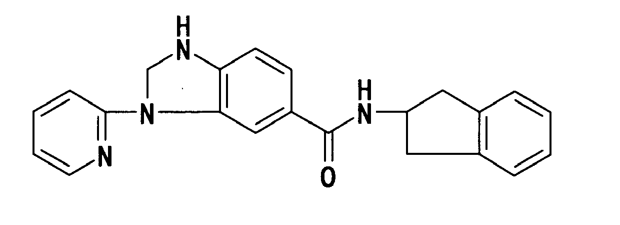

WO97/48697 also discloses that the following indole compound is useful as a PDE4 inhibitor.

However, the indole structure of this compound is different from the structure of the present invention. In addition, there is no disclosure that this compound has an inhibitory effect on VR1 receptor activity as in the present invention nor this compound is useful as a therapeutic agent and/or prophylactic agent of diseases associated with pain as in the present invention.

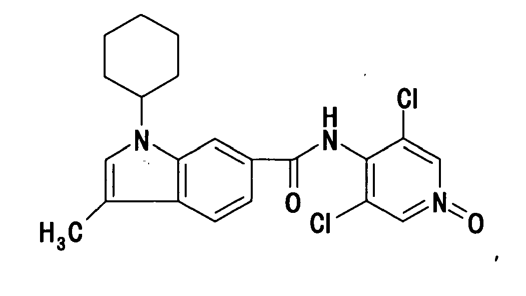

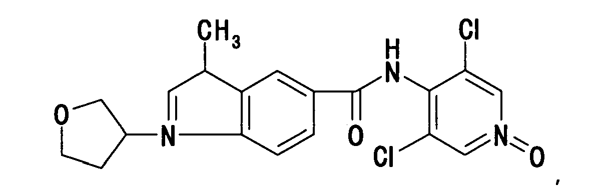

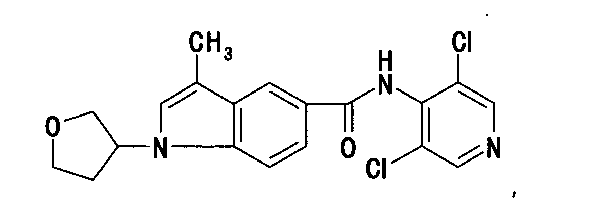

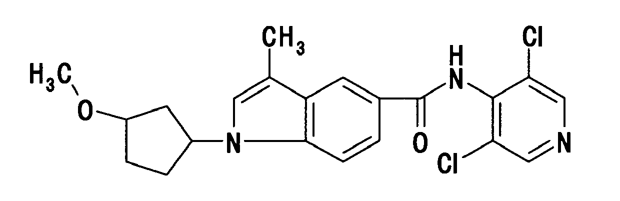

Next, compounds having known inhibitory effect on VR1 receptor activity and considered to relatively resemble the compound of the present invention are described.

WO02/16318 describes a compound represented by the following general formula as a compound exhibiting antagonism to VR1.

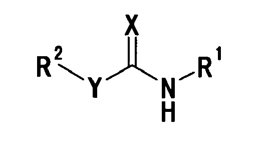

The above-mentioned invention is characterized by the presence of thiourea structure.

However, the above-mentioned invention is characterized by the presence of thiourea structure, and these compounds are different from the structure characteristic in the compound of the present invention where there is a phenyl or pyridyl substituent on an amino group substituted on the third position of benzamide structure.

Furthermore, this reference discloses no specific examples of a compound having benzamide structure as in the present invention and contains no description suggesting the compound of the present invention.

WO03/99284 describes a compound represented by the following general formula as a compound exhibiting antagonism to VR1.

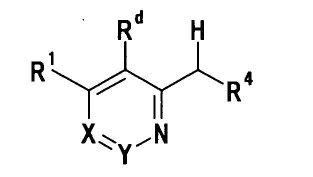

The above-mentioned invention is characterized by the structure where a ring including a pyridine ring is contained and the ring is substituted with an amino group which necessarily has one hydrogen atom.

However, this compound is different from the amino group substituted with an alkyl group in the structure characteristic in the compound of the present invention.

Furthermore, this reference discloses no specific examples of a compound having benzamide structure as in the present invention and contains no description suggesting the compound of the present invention.

Thus the above-mentioned compounds are different from the compound of the present invention in the structure and there is no description suggesting the compound of the present invention.

### DISCLOSURE OF THE INVENTION

As an analgesic agent, narcotic analgesics (morphine etc.), nonnarcotic analgesics (NSAID (nonsteroidal anti-inflammatory drug)), etc. are mainly used now. However, use of narcotic analgesics is severely restricted due to development of resistance/dependency and other serious side effects. It is known well other that an upper gastrointestinal tract disorder and a liver disorder frequently occur during long-term administration of nonnarcotic analgesics, and analgesic agent with a few side effects with higher analgesic effect is eagerly desired. Furthermore, as for diabetes-induced neuropathic pain, postherpetic neuralgia, and neuropathic pain such as trigeminal neuralgia, no effective analgesic agent has been found yet and development of an effective analgesic agent thereof is also expected.

Capsaicin-like compounds which act on VR1 are considered to develop the analgesic effect based on a pharmacological mechanism completely different from those of existing analgesic agents (inhibitionof capsaicin-sensitivenerves), and the efficacy is greatly expected as a therapeutic agent for neuropathic pain and the pain which originates in various conditions such as rheumatic arthritis for which the existing analgesic agents are not effective.

The fact that the final target of various inflammation related substances is VR1 suggests possibility that an agent which acts on VR1 is effective for various inflammatory pains and interstitial cystitis and its efficacy is greatly expected as an analgesic agent which replaces the existing analgesic agents.

Therefore, the purpose of the present invention is to provide a new analgesic agent based on the pharmacological mechanism completely different from those of existing analgesic agents (desensitization of capsaicin-sensitive nerves), i.e., VR1 activity inhibitor.

As a result of intensive study for developing an analgesic agent based on new action mechanism which will replace conventional analgesic agents such as nonnarcotic analgesics, pyrazolone analgesics, non-pyrazolone analgesics and NSAIDS, the present inventors have found out a 3-aminobenzamide compound which has excellent inhibitory effect on VR1, and completed the present invention. The present invention is described in more detail below.
1. A 3-aminobenzamide compound represented by the following general formula [1] or a pharmaceutically acceptable salt thereof: 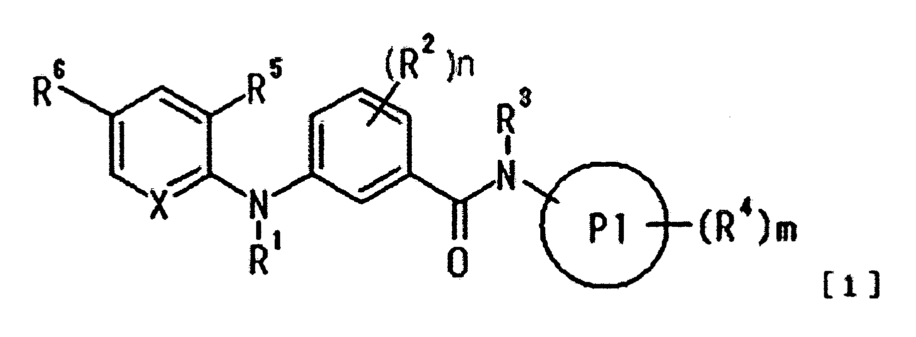 [wherein
   R¹ is
   a C1-6 alkyl group which may be substituted with one or more substituents, which may be the same or different, selected from the following Group A

### [Group A]

1) a halogen atom,
2) a hydroxyl group,
3) a C1-6 alkoxy group,
4) a halo C1-6 alkoxy group,
5) -NR⁷R⁸ (wherein R⁷ and R⁸ are the same or different and each represents
   (a) a hydrogen atom, or
   (b) a C1-6 alkyl group which may be substituted with a hydroxyl group, or
   (c) anitrogen-containing saturated heterocyclic group composed of a monocyclic ring formed by R⁷ and R⁸ together with the adjacent nitrogen atom),
6) -CONR⁷R⁸ (wherein R⁷ and R⁸ are the same as above),
7) -COR⁹ (wherein R⁹ is
   (a) a hydrogen atom,
   (b) a hydroxyl group,
   (c) a C1-6 alkyl group, or
   (d) a C1-6 alkoxy group),
8) -NR⁷¹COR⁹ (wherein R⁹ is the same as above and R⁷¹ is
   (a) a hydrogen atom, or
   (b) a C1-6 alkyl group),
9) -NR⁷¹CONR⁷R⁸ (wherein R⁷, R⁸ and R⁷¹ are the same as above),
10) -NR⁷¹SO₂R¹⁰ (wherein R⁷¹ is the same as above and R¹⁰ is a C1-6 alkyl group),
11) -SO₂R¹⁰ (wherein R¹⁰ is the same as above);
   [wherein the C1-6 alkyl group, C1-6 alkoxy group, halo C1-6 alkoxy group and nitrogen-containing saturated heterocyclic group composed of a monocyclic ring in the above 1) to 11) may be further substituted with one or more substituents selected from the Group A.]
   R² is
   one or more substituents, which may be the same or different, selected from the following Group B

### [Group B]

1) a halogen atom,
2) a hydroxyl group,
3) a C1-6 alkyl group which may be substituted with one or more substituents, which may be the same or different, selected from said Group A,
4) a C1-6 alkoxy group which may be substituted with one or more substituents, which may be the same or different, selected from said Group A,
5) a cycloalkylalkoxy group (said cycloalkylalkoxy group may be substituted with
   (a) one or more substituents, which may be the same or different, selected from said Group A, or
   (b) a C1-6 alkyl group which may be substituted with one or more substituents, which may be the same or different, selected from said Group A),
6) an aralkyl group (said aralkyl group may be substituted with
   (a) one or more substituents, which may be the same or different, selected from said Group A, or
   (b) a C1-6 alkyl group which may be substituted with one or more substituents, which may be the same or different, selected from said Group A),
7) an aralkoxy group (said aralkoxy group may be substituted with
   (a) one or more substituents, which may be the same or different, selected from said Group A, or
   (b) a C1-6 alkyl group which may be substituted with one or more substituents, which may be the same or different, selected from said Group A),
8) -COR¹¹ (wherein R¹¹ is
   (a) a hydroxyl group,
   (b) a C1-6 alkyl group which may be substituted with one or more substituents, which may be the same or different, selected from said Group A,
   (c) a C1-6 alkoxy group which may be substituted with one or more substituents, which may be the same or different, selected from said Group A or a C1-6 alkyl group which may be substituted with said one or more substituents,
   (d) a cycloalkyl group having 3 to 8 carbon atoms which may be substituted with one or more substituents, which may be the same or different, selected from said Group A or a C1-6 alkyl group which may be substituted with said one or more substituents,
   (e) a cycloalkylalkoxy group which may be substituted with one or more substituents, whichmaybe the same or different, selected from said Group A or a C1-6 alkyl group which may be substituted with said one or more substituents,
   (f) an aralkyl group which may be substituted with one or more substituents, which may be the same or different, selected from said Group A or a C1-6 alkyl group which may be substituted with said one or more substituents,
   (g) an aralkoxy group which may be substituted with one or more substituents, which may be the same or different, selected from said Group A or a C1-6 alkyl group which may be substituted with said one or more substituents,
   (h) a saturated or unsaturated carbocyclic group having 3 to 14 carbon atoms which may be substituted with one or more substituents, which may be the same or different, selected from said Group A or a C1-6 alkyl group which may be substituted with said one or more substituents),
9) -NR¹²R¹³ (wherein R¹² and R¹³ are the same or different and each represents
   (a) a hydrogen atom,
   (b) a C1-6 alkyl group which may be substituted with one or more substituents, which may be the same or different, selected from said Group A, or
   (c) anitrogen-containing saturated heterocyclic group composed of a monocyclic ring and is formed by R¹² and R¹³ together with an adjacent nitrogen atom),
10) -CONR¹²R¹³ (wherein R¹² and R¹³ are the same as above),
11) -NR¹²¹COR¹¹ (wherein R¹²¹ is
   (a) a hydrogen atom,
   (b) a C1-6 alkyl group which may be substituted with one or more substituents, which may be the same or different, selected from said Group A, and R¹¹ is the same as above),
12) -NR¹²¹CONR¹²R¹³ (wherein R¹², R¹³ and R¹²¹ are the same as above),
13) -SR¹⁴ (wherein R¹⁴ is
   (a) a C1-6 alkyl group which may be substituted with one or more substituents, which may be the same or different, selected from said Group A, or
   (b) a cycloalkyl group having 3 to 8 carbon atoms which may be substituted with one or more substituents, which may be the same or different, selected from said Group A),
14) -SOR¹⁴ (wherein R¹⁴ is the same as above),
15) -SO₂R¹⁴ (wherein R¹⁴ is the same as above),
16) -SO₂NR¹²R¹³ (wherein R¹² and R¹³ are the same as above),
17) a saturated or unsaturated carbocyclic group having 3 to 14 carbon atoms (wherein the carbocyclic group may be substituted with
   (a) one or more substituents, which may be the same or different, selected from said Group A, or
   (b) a C1-6 alkyl group which may be substituted with one or more substituents, which may be the same or different, selected from said Group A),
18) a saturated or unsaturated heterocyclic group having at least one hetero atom selected from a nitrogen atom, an oxygen atom and a sulfur atom (wherein the heterocyclic group may be substituted with
   (a) one or more substituents, which may be the same or different, selected from said Group A, or
   (b) a C1-6 alkyl group which may be substituted with one or more substituents, which may be the same or different, selected from said Group A), and
19) an aryloxy group (wherein the aryloxy group may be substituted with
   (a) one or more substituents, which may be the same or different, selected from said Group A, or
   (b) a C1-6 alkyl group which may be substituted with one or more substituents, which may be the same or different, selected from said Group A);
   or R¹ and R² may together form a -CH₂-CH₂-O- bond between the adjacent nitrogen atom and carbon atom;
   R³ is
   (1) a hydrogen atom, or
   (2) a C1-6 alkyl group which may be substituted with one or more substituents, which may be the same or different, selected from said Group A;
   m is an integer of 1 to 5;
   n is an integer of 0, 1 to 4; and
   R⁴ is one or more substituents, which may be the same or different, selected from said Group B, and
   when m is two or more
   (1) two R⁴ groups may together form =0, or
   (2) two R⁴ groups together with a carbon atom adjacent to a P1 ring may form 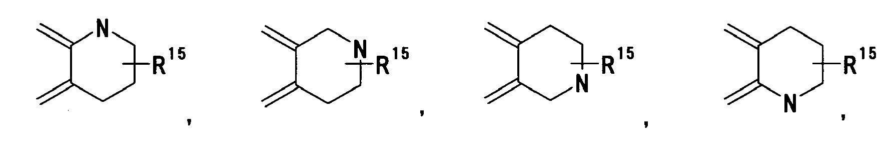 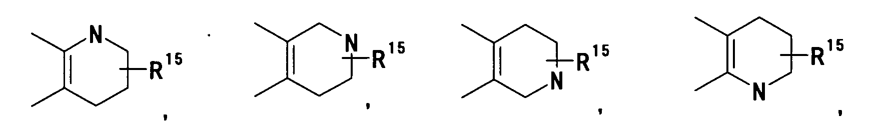 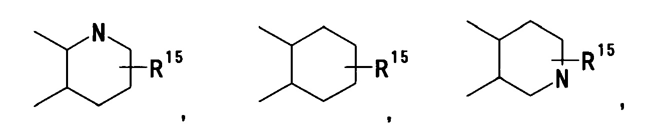 or 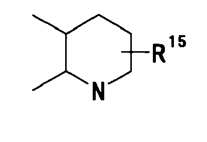 (wherein R¹⁵ is 1 to 4 substituents which are selected from said Group B);
   R⁵ and R⁶ are the same or different and each is
   (1) a hydrogen atom, or
   (2) a substituent selected from said Group B; or
   R² and R⁵ may form a -0- bond together with an adjacent carbon atom;
   X is
   (1) CH or
   (2) N; and
   P1 ring is
   (1) a saturated or unsaturated carbocyclic group having 3 to 14 carbon atoms, or
   (2) a saturated or unsaturated heterocyclic group having at least one hetero atom selected from a nitrogen atom, an oxygen atom and a sulfur atom].

2. The 3-aminobenzamide compound or a pharmaceutically acceptable salt thereof according to the aforementioned 1 wherein R¹ and R² do not form a -CH₂-CH₂-O- together with the adjacent nitrogen atom and carbon atom and R² and R⁵ do not forma -O- together with the adjacent carbon atom in said general formula [1].
3. The 3-aminobenzamide compound or a pharmaceutically acceptable salt thereof according to the aforementioned 1 wherein the compound is represented by the following general formula [2]: 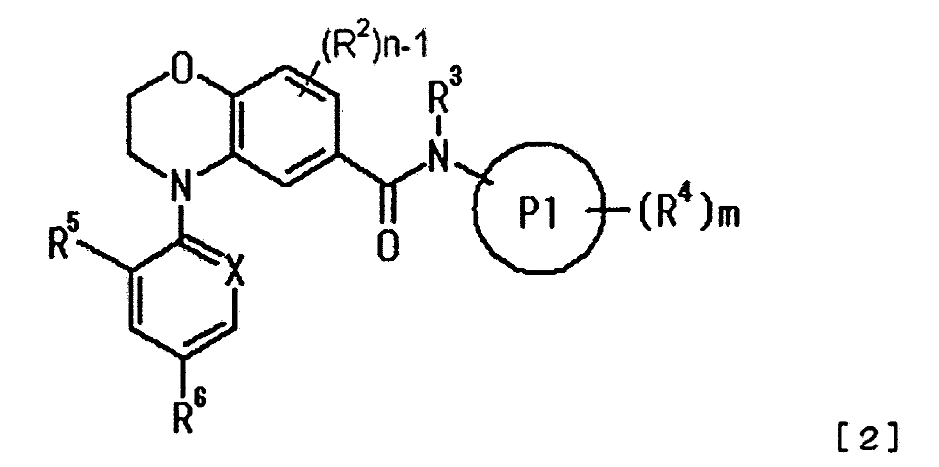 [wherein R², R³, R⁴, R⁵, R⁶, m, n, X, and P1 ring are the same as above provided that n is not 0 in this case.]
4. The 3-aminobenzamide compound or a pharmaceutically acceptable salt thereof according to the aforementioned 1 wherein the compound is represented by the following general formula [3] : 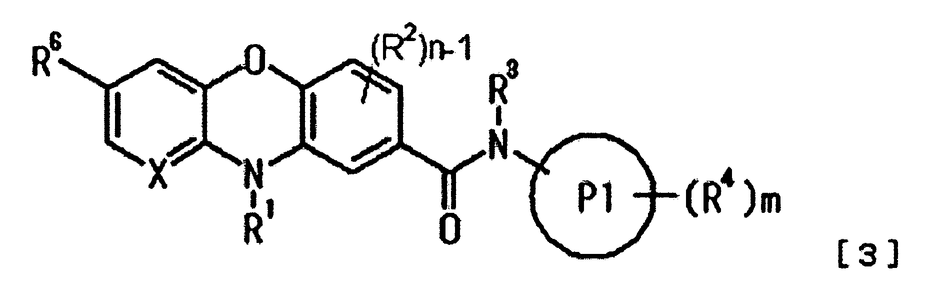 [wherein R¹, R², R³, R⁴, R⁶, m, n, X, and P1 ring are the same as above provided that n is not 0 in this case.]
5. The 3-aminobenzamide compound or a pharmaceutically acceptable salt thereof according to the aforementioned 1 to 4 wherein the carbocyclic group or heterocyclic group of P1 ring is a monocyclic ring.
6. The 3-aminobenzamide compound or a pharmaceutically acceptable salt thereof according to the aforementioned 1, 2, 4 or 5 wherein R¹ is a C1-6 alkyl group which may be substituted with one or more substitution groups selected from a hydroxyl group, a C1-6 alkoxy group, -CONR⁷R⁸, and -COR⁹; n is 0 or n is an integer of 1 to 4 and R² is a halogen atom, a hydroxyl group, a C1 - alkyl group def ined in said Group B, a C1 - 6 alkoxy group defined in said Group B, a carbocyclic group defined in said Group B, or an aralkoxy group defined in said Group B; and R³ is a hydrogen atom.
7. The 3-aminobenzamide compound or a pharmaceutically acceptable salt thereof according to the aforementioned 6 wherein n is 0 or n is an integer of 1 to 4 and R² is a halogen atom, a hydroxyl group, a C1-6 alkyl group, a phenyl group, a phenoxy group or a C1-6 alkoxy group which may be substituted with a substituent selected fromahydroxyl group, a C1-6 alkoxy group, -CONR¹²R¹³ (wherein R¹² and R¹³ are the same as above) and -COR¹¹ (wherein R¹¹ is the same as above).
8. The 3-aminobenzamide compound or a pharmaceutically acceptable salt thereof according to the aforementioned 1 to 7 wherein R⁴ is a halogen atom, a C1-6 alkyl group, a halo C1-6 alkyl group, a C1-6 alkoxy group, a halo C1-6 alkoxy group, or a saturated monocyclic heterocyclic group having a nitrogen atom as a hetero atom.
9. The 3-aminobenzamide compound or a pharmaceutically acceptable salt thereof according to the aforementioned 1 wherein X is CH or N; and R¹ is a C1-6 alkyl group which may be substituted with one or more substituents selected from a hydroxyl group, a C1-6 alkoxy group, -NR⁷R⁸ (wherein R⁷ and R⁸ are the same or different and each is a hydrogen atom or a C1-6 alkyl group which may be substituted with a hydroxyl group), -CONR⁷R⁸ (wherein R⁷ and R⁸ are the same or different and each is a hydrogen atom or a C1-6 alkyl group which may be substituted with a hydroxyl group) and -COR⁹ (wherein R⁹ is a hydroxyl group or a C1-6 alkoxy group); n is an integer of 0, 1 to 2, and R² is a halogen atom, a hydroxyl group, a C1-6 alkyl group, an aryl group, an aryloxy group or a C1-6 alkoxy group (wherein the alkoxy group may be substituted with one or two substituents selected from a hydroxyl group, a C1-6 alkoxy group, -CONR⁷R⁸ (wherein R⁷ and R⁸ are the same or different and each is a hydrogen atom or a C1-6 alkyl group which may be substituted with a hydroxyl group) and -COR⁹ (wherein R⁹ is a hydroxyl group or a C1-6 alkoxy group); R³ is a hydrogen atom; m is an integer of 1 to 3; R⁴ is a halogen atom, a C1-6 alkyl group, a halo C1-6 alkyl group, a C1-6 alkoxy group, a halo C1-6 alkoxy group, -CONR¹²R¹³ (wherein R¹² and R¹³ are the same or different and each is a hydrogen atom or a C1-6 alkyl) or a saturated monocyclic heterocyclic group is a having a nitrogen atom as a hetero atom; R⁵ and R⁶ are the same or different and each is a hydrogen atom, a halogen atom, a C1-6 alkoxy group, a C1-6 alkyl group which may be substituted with a hydroxyl group, or a C1-6 alkoxy group, a halo C1-6 alkyl group, -CONR¹²R¹³ (wherein R¹² and R¹³ are the same or different and each is a hydrogen atom or a C1-6 alkyl) or -COR¹¹ (wherein R¹¹ is a hydroxyl group or a C1-6 alkoxy group) ; and P1 ring is a phenyl group or a pyridyl group.
10. The 3-aminobenzamide compound or a pharmaceutically acceptable salt thereof according to the aforementioned 1 selected from the following group:
   (1) N-(4-tert-butylphenyl)-3-[N-(3-chloropyridin-2-yl)-N-methyl-amino]benzamide hydrochloride,
   (2) N-(4-tert-butylphenyl)-3-[N-(pyridin-2-yl)-N-methyl-amino]benzamide hydrochloride,
   (3) N-(4-tert-butylphenyl)-3-[N-(3-chloropyridin-2-yl)-N-ethyl-amino]benzamide,
   (4) N-(4-tert-butylphenyl)-3-[N-(3-chloropyridin-2-yl)-N-methyl]amino-4-methoxybenzamide,
   (5) N-(4-tert-butylphenyl)-3-[N-(3-chloropyridin-2-yl)-N-methyl]amino-4-phenoxybenzamide,
   (6) N-(4-tert-butylphenyl)-3-[N-(3-chloropyridin-2-yl)-N-methyl-amino]-2-methylbenzamide,
   (7) N-(4-tert-butylphenyl)-3-[N-(3-chloropyridin-2-yl)-N-methyl-amino]-4-methylbenzamide,
   (8) N-(4-tert-butylphenyl)-3-[N-(3-chloropyridin-2-yl)-N-methyl-amino]-4-phenylbenzamide,
   (9) N-(4-tert-butylphenyl)-3-[N-(3-chloropyridin-2-yl)-N-methyl-amino]-4-fluorobenzamide,
   (10) N-(4-tert-butylphenyl)-3-[N-(3-chloropyridin-2-yl)-N-ethyl]amino-4-methoxybenzamide,
   (11) N-(4-tert-butylphenyl)-3-[N-(3-chloropyridin-2-yl)-N-isopropyl]amino-4-methoxybenzamide,
   (12) N-(4-tert-butylphenyl)-4-chloro-3-[N-(3-chloropyridin-2-yl)-N-methyl-amino]benzamide,
   (13) N-(4-tert-butylphenyl)-4-chloro-3-[N-(pyridin-2-yl)-N-methyl-amino]benzamide,
   (14)N-(4-tert-butylphenyl)-3-[N-(3-chloropyridin-2-yl)-N-methyl-amino]-4-methoxymethyloxybenzamide,
   (15) N-(4-tert-butylphenyl)-3-[N-(3-chloropyridin-2-yl)-N-methyl-amino]-4-hydroxybenzamide,
   (16) N-(4-tert-butylphenyl)-3-[N-(3-chloropyridin-2-yl)-N-methyl-amino]-4-isopropoxybenzamide,
   (17) N-(4-tert-butylphenyl)-10-methyl-10H-benzo[b]pyrido[2,3-e][1,4]oxazine-8-carboxamide,
   (18) N-(4-tert-butylphenyl)-3-[N-(3-chloropyridin-2-yl)-N-methyl-amino]-4-(2-hydroxyethyl)oxybenzamide,
   (19) {4-(4-tert-butylphenyl)aminocarbonyl-2-[N-(3-chloropyridin-2-yl)-N-methyl]amino-phenoxy}acetic acid,
   (20) N-(4-tert-butylphenyl)-4-carbamoylmethyloxy-3-[N-(3-chloropyridin-2-yl)-N-methyl-amino]benzamide,
   (21) N-(4-tert-butylphenyl)-3-[N-(3-chloropyridin-2-yl)-N-methyl-amino]-4-(N-methylcarbamoylmethyl)oxybenzamide,
   (22) N-(4-tert-butylphenyl)-3-[N-(3-chloropyridin-2-yl)-N-methyl-amino]-4-(N,N-dimethylcarbamoylmethyl)oxybenzamide,
   (23) N-(4-tert-butylphenyl)-5-chloro-3-[N-(3-chloropyridin-2-yl)-N-methyl]aminobenzamide,
   (24) N-(4-tert-butylphenyl)-2-chloro-5-[N-(3-chloropyridin-2-yl)-N-methyl]aminobenzamide,
   (25) N-(4-tert-butylphenyl)-3-[N-(3-chloropyridin-2-yl)-N-methyl]amino-2-hydroxybenzamide,
   (26) N-(4-tert-butylphenyl)-3-[N-(3-chloropyridin-2-yl)-N-methyl]amino-2-methoxybenzamide,
   (27) N-(4-tert-butylphenyl)-3-[N-(3-chloropyridin-2-yl)-N-methyl-amino]-2-(2-hydroxyethyl)oxybenzamide,
   (28) N-(4-tert-butylphenyl)-3-[N-(3-chloropyridin-2-yl)-N-(2-hydroxyethyl)]amino-4-methoxybenzamide,
   (29) N-(4-tert-butylphenyl)-4-chloro-3-[N-(3-chloropyridin-2-yl)-N-(2-methoxyethyl)amino]benzamide,
   (30) N-(4-tert-butylphenyl)-4-chloro-3-[N-(3-chloropyridin-2-yl)-N-(2-hydroxyethyl)amino]benzamide,
   (31) 4-chloro-3-[N-(3-chloropyridin-2-yl)-N-(2-hydroxyethyl)amino]-N-(4-trifluoromethylphenyl)-benzamide hydrochloride,
   (32) 4-chloro-3-[N-(3-chloropyridin-2-yl)-N-(2-hydroxyethyl)amino]-N-(4-trifluoromethoxyphenyl)-benzamide hydrochloride,
   (33) N-(4-tert-butylphenyl)-3-[N-(2-chlorophenyl)-N-methyl-amino]benzamide, and
   (34) N-(4-tert-butylphenyl)-4-(3-chloropyridin-2-yl)-3,4-dihydro-2H-benzo[1,4]oxazine-6-carboxamide; and
   (35) 4-chloro-3-[N-(3-chloropyridin-2-yl)-N-(2-hydroxyethyl)amino]-N-(4-isobutyloxyphenyl)-benzamide hydrochloride,
   (36) 4-chloro-N-(4-chlorophenyl)-3-[N-(3-chloropyridin-2-yl)-N-(2-hydroxyethyl)amino]-benzamide,
   (37) 4-chloro-3-[N-(3-chloropyridin-2-yl)-N-(2-hydroxyethyl)amino]-N-(4-isopropylphenyl)-benzamide,
   (38) 4-chloro-3-[N-(3-chloropyridin-2-yl)-N-(2-hydroxyethyl)amino]-N-(1-methyl-1,2,3,4-tetrahydroisoquinolin-7-yl)-benzamide,
   (39) 4-chloro-3-[N-(3-chloropyridin-2-yl)-N-(2-hydroxyethyl)amino]-N-(4-chloro-3-trifluoromethylphenyl)-benzamide,
   (40) 4-chloro-3-[N-(3-chloropyridin-2-yl)-N-(2-hydroxyethyl)amino]-N-(2-trifluoromethylpyridin-5-yl)-benzamide,
   (41) 4-chloro-3-[N-(3-chloropyridin-2-yl)-N-(2-hydroxyethyl)amino]-N-(4-isopropyloxyphenyl)-benzamide,
   (42) 4-chloro-3-[N-(3-chloropyridin-2-yl)-N-(2-hydroxyethyl)amino]-N-(5-trifluoromethylpyridin-2-yl)-benzamide,
   (43) 4-chloro-3-[N-(3-chloropyridin-2-yl)-N-(2-hydroxyethyl)amino]-N-(3-fluoro-4-piperidinylphenyl)-benzamide,
   (44) 4-chloro-3-[N-(3-chloropyridin-2-yl)-N-(2-hydroxyethyl)amino]-N-(4-isobutyloxyphenyl)-benzamide,
   (45) 4-chloro-3-[N-(3-chloropyridin-2-yl)-N-(3-hydroxypropyl)amino]-N-(4-tert-butylphenyl)-benzamide,
   (46) N-(3-chloropyridin-2-yl)-N-{2-chloro-5-[N-(4-tert-butylphenyl)carbamoyl]phenyl}-aminoacetic acid,
   (47) 4-chloro-3-[N-(3-chloropyridin-2-yl)-N-(carbamoylmethyl)amino]-N-(4-tert-butylphenyl)-benzamide,
   (48) 4-chloro-3-[N-(3-chloropyridin-2-yl)-N-(N-methylcarbamoylmethyl)amino]-N-(4-tert-butylphenyl)-benzamide,
   (49) 4-chloro-3-[N-(3-chloropyridin-2-yl)-N-(N,N-dimethylcarbamoylmethyl)amino]-N-(4-tert-butylphenyl)-benzamide,
   (50) 3-[N-(3-chloropyridin-2-yl)-N-(2-hydroxyethyl)amino]-N-(4-isobutyloxyphenyl)-4-methoxy-benzamide,
   (51) N-(4-isobutyloxyphenyl)-4-methoxy-3-[N-(5-methylpyridin-2-yl)-N-(2-hydroxyethyl)amino]-benzamide,
   (52) 3-[N-(3-chloropyridin-2-yl)-N-(2-hydroxyethyl)amino]-N-(4-isobutyloxyphenyl)-benzamide,
   (53) N-(4-tert-butylphenyl)-3-[N-(3-chloropyridin-2-yl)-N-(2-hydroxyethyl)amino]-benzamide,
   (54) 3-[N-(3-chloropyridin-2-yl)-N-(2-hydroxyethyl)amino]-N-(4-trifluoromethylphenyl)-benzamide,
   (55) 4-chloro-3-[N-(3-chloropyridin-2-yl)-N-(2-hydroxyethyl)amino]-N-(4-isobutyloxy-3-methylcarbamoylphenyl)-benzamide,
   (56) 4-chloro-3-[N-(3-chloropyridin-2-yl)-N-(2-hydroxyethyl)amino]-N-(3-dimethylcarbamoyl-4-isobutyloxyphenyl)-benzamide,
   (57) N-(4-tert-butylphenyl)-4-chloro-3-{N-(3-chloropyridin-2-yl)-N-[N-(2-hydroxyethyl)carbamoylmethyl]amino}-benzamide,
   (58) 3-[N-(2-aminoethyl)-N-(3-chloropyridin-2-yl)amino]-4-chloro-N-(4-tert-butylphenyl)-benzamide,
   (59) 4-chloro-3-[N-(3-chloropyridin-2-yl)-N-(carbamoylmethyl)amino]-N-(4-trifluoromethylphenyl)-benzamide,
   (60) 4-chloro-3-[N-(3-chloropyridin-2-yl)-N-(carbamoylmethyl)amino]-N-(4-trifluoromethoxyphenyl)-benzamide,
   (61) 4-chloro-3-[N-(3-chloropyridin-2-yl)-N-(carbamoylmethyl)amino]-N-(4-isobutyloxyphenyl)-benzamide,
   (62) 4-chloro-3-[N-(3-chloropyridin-2-yl)-N-(carbamoylmethyl)amino]-N-(3-fluoro-4-piperidinophenyl)-benzamide,
   (63) 3-[N-(3-chloropyridin-2-yl)-N-(carbamoylmethyl)amino]-4-methyl-N-(4-trifluoromethylphenyl)-benzamide,
   (64) 3-[N-(3-chloropyridin-2-yl)-N-(carbamoylmethyl)amino]-4-methyl-N-(4-trifluoromethoxyphenyl)-benzamide,
   (65) 3-[N-(3-chloropyridin-2-yl)-N-(carbamoylmethyl)amino]-N-(4-isobutyloxyphenyl)-4-methoxy-benzamide,
   (66) 4-chloro-3-[N-(3-chloropyridin-2-yl)-N-(N-methylcarbamoylmethyl)amino]-N-(4-trifluoromethylphenyl)-benzamide,
   (67) 4-chloro-3-[N-(3-chloro-5-trifluorofiethylpyridin-2-yl)-N-(carbamoylmethyl)amino]-N-(4-trifluoromethoxyphenyl)-benzamide,
   (68) 4-chloro-3-[N-(3-chloro-5-trifluoromethylpyridin-2-yl)-N-(carbamoylmethyl)amino]-N-(4-trifluoromethylphenyl)-benzamide,
   (69) 4-chloro-3-[N-(3-chloropyridin-2-yl)-N-(carbamoylmethyl)amino]-N-(trifluoromethoxyphenyl)-benzamide,
   (70) 4-chloro-3-[N-(3-chloropyridin-2-yl)-N-(carbamoylmethyl)amino]-N-(trifluoromethylphenyl)-benzamide,
   (71) 4-chloro-3-[N-(3-chloro-5-methoxymethylpyridin-2-yl)-N-(carbamoylmethyl)amino]-N-(trifluoromethoxyphenyl)-benzamide,
   (72) 4-chloro-3-[N-(3-chloro-5-methoxymethylpyridin-2-yl)-N-(carbamoylmethyl)amino]-N-(trifluoromethylphenyl)-benzamide
   (73) ethyl 6-{[5-(4-tert-butylphenylcarbamoyl)-2-chlorophenyl]-methyl-amino}-5-chloronicotinate,
   (74) 6-{[5-(4-tert-butylphenylcarbamoyl)-2-chlorophenyl]-methyl-amino}-5-chloronicotinic acid,
   (75) 6-{[5-(4-tert-butylphenylcarbamoyl)-2-chlorophenyl]-methyl-amino}-5-chloronicotinamide,
   (76) 6-{[5-(4-tert-butylphenylcarbamoyl)-2-chlorophenyl]-methyl-amino}-5-chloro-N-methylnicotinamide,
   (77) 6-{[5-(4-tert-butylphenylcarbamoyl)-2-chlorophenyl]-methyl-amino}-5-chloro-N,N-dimethylnicotinamide,
   (78) N-(4-tert-butylphenyl)-4-chloro-3-[N-(3-chloro-5-hydroxymethylpyridin-2-yl)-N-methyl-amino]benzamide,
   (79) 4-chloro-3-[N-(3-chloro-5-hydroxymethylpyridin-2-yl)-N-methyl-amino]-N-(4-trifluoromethylphenyl)-benzamide,
   (80) 4-chloro-3-[N-(3-chloro-5-hydroxymethylpyridin-2-yl)-N-methyl-amino]-N-(4-chlorophenyl)-benzamide,
   (81) 4-chloro-3-[N-(3-chloro-5-hydroxymethylpyridin-2-yl)-N-methyl-amino]-N-(4-trifluoromethoxyphenyl)-benzamide,
   (82) 3-[N-(3-chloro-5-hydroxymethylpyridin-2-yl)-N-methyl-amino]-4-fluoro-N-(4-trifluoromethylphenyl)-benzamide, and
   (83) 3-[N-(3-chloro-5-hydroxymethylpyridin-2-yl)-N-methyl-amino]-4-fluoro-N-(4-trifluoromethoxyphenyl)-benzamide.
11. A pharmaceutical composition comprising a 3-aminobenzamide compound or a pharmaceutically acceptable salt thereof according to any of the aforementioned 1 to 10 and a pharmaceutically acceptable carrier.
12. A pharmaceutical composition comprising a 3-aminobenzamide compound or a pharmaceutically acceptable salt thereof according to any of the aforementioned 1 to 10 and a pharmaceutically acceptable carrier for treating and/or preventing a disease selected from algia, acute pain, chronic pain, neuropathic pain, rheumatoid arthritis pain, neuralgia, neuropathy, hyperalgesia, migraine, joint pain, acute postherpetic neuralgia, postherpetic neuralgia, chronic postherpetic neuralgia, postoperative pain, cancer pain, inflammatory pain, interstitial cystitis, posttraumatic neuralgia, diabetic neuropathy, neurodegenerative disease, brain apoplexy, ischemic symptom, nerve injury, neurogenic skin disorders, inflammatory disease, pruritus, allergic rhinitis, apoplexy, irritable bowel syndrome, asthma, chronic obstructive pulmonary disease, dermatitis, mucositis, stomach and duodenal ulcer and inflammatory bowel disease, bladder hypersensitivity, and overactive bladder type frequent urination and urinary incontinence.
13. A pharmaceutical composition for treating and/or preventing pain comprising a 3-aminobenzamide compound or a pharmaceutically acceptable salt thereof according to any of the aforementioned 1 to 10 and a pharmaceutically acceptable carrier.
14. The pharmaceutical composition according to the aforementioned 13 wherein the pain is algia, acute pain, chronic pain, neuropathic pain, rheumatoid arthritis pain, neuralgia, neuropathy, hyperalgesia, migraine, joint pain, acute postherpetic neuralgia, postherpetic neuralgia, chronic postherpetic neuralgia, postoperative pain, cancer pain, inflammatory pain, interstitial cystitis, posttraumatic neuralgia, diabetic neuropathy or neurodegenerative disease.
15. An inhibitor of vanilloid receptor subtype 1 (VR1) activity comprising a 3-aminobenzamide compound or a pharmaceutically acceptable salt thereof according to any of the aforementioned 1 to 10 and a pharmaceutically acceptable carrier.
16. A method for treating and/or preventing a disease selected from algia, acute pain, chronic pain, neuropathic pain, rheumatoid arthritis pain, neuralgia, neuropathy, hyperalgesia, migraine, joint pain, acute postherpetic neuralgia, postherpetic neuralgia, chronic postherpetic neuralgia, postoperative pain, cancer pain, inflammatory pain, interstitial cystitis, posttraumatic neuralgia, diabetic neuropathy, neurodegenerative disease, brain apoplexy, ischemic symptom, nerve injury, neurogenic skin disorders, inflammatory disease, pruritus, allergic rhinitis, apoplexy, irritable bowel syndrome, asthma, chronic obstructive pulmonary disease, dermatitis, mucositis, stomach and duodenal ulcer and inflammatory bowel disease, bladder hypersensitivity, and overactive bladder type frequent urination and urinary incontinence characterized in that the method comprises administering a pharmaceutically effective amount of a 3-aminobenzamide compound or a pharmaceutically acceptable salt thereof according to any of the aforementioned 1 to 10.
17. A method for treating and/or preventing pain characterized in that the method comprises administering a pharmaceutically effective amount of a 3-aminobenzamide compound or a pharmaceutically acceptable salt thereof according to any of the aforementioned 1 to 10.
18. The treating and/or preventing method according to the aforementioned 17 wherein the pain is algia, acute pain, chronic pain, neuropathic pain, rheumatoid arthritis pain, neuralgia, neuropathy, hyperalgesia, migraine, joint pain, acute postherpetic neuralgia, postherpetic neuralgia, chronic postherpetic neuralgia, postoperative pain, cancer pain, inflammatory pain, interstitial cystitis, posttraumatic neuralgia, diabetic neuropathy or neurodegenerative disease.
19. A commercial package comprising a pharmaceutical composition according to the aforementioned 11 to 14 and written instructions about this pharmaceutical composition stating that said composition can be used or should be used for treating and/or preventing a disease selected from algia, pain, acute pain, chronic pain, neuropathic pain, rheumatoid arthritis pain, neuralgia, neuropathy, hyperalgesia, migraine, joint pain, acute postherpetic neuralgia, postherpetic neuralgia, chronic postherpetic neuralgia, postoperative pain, cancer pain, inflammatory pain, interstitial cystitis, posttraumatic neuralgia, diabetic neuropathy, neurodegenerative disease, brain apoplexy, ischemic symptom, nerve injury, neurogenic skin disorders, inflammatory disease, pruritus, allergic rhinitis, apoplexy, irritable bowel syndrome, asthma, chronic obstructive pulmonary disease, dermatitis, mucositis, stomach and duodenal ulcer and inflammatory bowel disease, bladder hypersensitivity, and overactive bladder type frequent urination and urinary incontinence.
20. Useofa3-aminobenzamidecompoundorapharmaceutically acceptable salt thereof according to any of the aforementioned 1 to 10 for preparing a pharmaceutical composition according to the aforementioned 12 for treating and/or preventing a disease selected from algia, acute pain, chronic pain, neuropathic pain, rheumatoid arthritis pain, neuralgia, neuropathy, hyperalgesia, migraine, joint pain, acute postherpetic neuralgia, postherpetic neuralgia, chronic postherpetic neuralgia, postoperative pain, cancer pain, inflammatory pain, interstitial cystitis, posttraumatic neuralgia, diabetic neuropathy, neurodegenerative disease, brain apoplexy, ischemic symptom, nerve injury, neurogenic skin disorders, inflammatory disease, pruritus, allergic rhinitis, apoplexy, irritable bowel syndrome, asthma, chronic obstructive pulmonary disease, dermatitis, mucositis, stomach and duodenal ulcer and inflammatory bowel disease, bladder hypersensitivity, and overactive bladder type frequent urination and urinary incontinence.
21. Useofa3-aminobenzamidecompoundorapharmaceutically acceptable salt thereof according to any of the aforementioned 1 to 10 for preparing a pharmaceutical composition for treating and/or preventing pain according to the aforementioned 13.
22. The use of a 3-aminobenzamide compound or a pharmaceutically acceptable salt thereof according to the aforementioned 21 wherein the pain is algia, acute pain, chronic pain, neuropathic pain, rheumatoid arthritis pain, neuralgia, neuropathy, hyperalgesia, migraine, joint pain, acute postherpetic neuralgia, postherpetic neuralgia, chronic postherpetic neuralgia, postoperative pain, cancer pain, inflammatory pain, interstitial cystitis, posttraumatic neuralgia, diabetic neuropathy or neurodegenerative disease.
23. A pharmaceutical composition comprising a 3-aminobenzamide compound or a pharmaceutically acceptable salt thereof according to any of the aforementioned 1 to 10 and a pharmaceutically acceptable carrier wherein the composition is to be used in combination with one or more agents selected from the group consisting of an anti-virus agent, an antidepressant, an anticonvulsant, an antiarrhythmic drug, a local anesthetic, an anesthetic drug, an N-methyl-D-aspartate receptor antagonist, adrenal-cortex steroid, a nerve block, a nonsteroidal antiinflammatory analgesic, narcotics, an antagonist analgesic, ?₂ adrenaline-receptor agonist, a medicine for external application, a calcium channel antagonist, and a potassium channel opening drug.
24. Use of a 3-aminobenzamide compound or a pharmaceutically acceptable salt thereof according to any of the aforementioned 1 to 10 for preparing a pharmaceutical composition according to the aforementioned 23.
25. A method for treating and/or preventing a disease selected from algia, acute pain, chronic pain, neuropathic pain, rheumatoid arthritis pain, neuralgia, neuropathy, hyperalgesia, migraine, joint pain, acute postherpetic neuralgia, postherpetic neuralgia, chronic postherpetic neuralgia, postoperative pain,cancer pain,inflammatory pain, posttraumatic neuralgia, diabetic neuropathy, neurodegenerative disease, brain apoplexy, ischemic symptom, nerve injury, neurogenic skin disorders, inflammatory disease, interstitial cystitis, pruritus, allergic rhinitis, apoplexy, irritable bowel syndrome, asthma, chronic obstructive pulmonary disease, dermatitis, mucositis, stomach and duodenal ulcer and inflammatory bowel disease, bladder hypersensitivity, and overactive bladder type frequent urination and urinary incontinence characterized in that one or more agents selected from the group consisting of an anti-virus agent, an antidepressant, an anticonvulsant, an antiarrhythmic drug, a local anesthetic, an anesthetic drug, an N-methyl-D-aspartate receptor antagonist, adrenal-cortex steroid, a nerve block, a nonsteroidal antiinflammatory analgesic, narcotics, an antagonist analgesic, ?₂ adrenaline-receptor agonist, a medicine for external application, a calcium channel antagonist, and a potassium channel opening drug are used in combination with a pharmaceutically effective amount of an inhibitor of vanilloid receptor subtype 1 (VR1) activity.
26. The medical treatment method and/or the prevention method according to the aforementioned 25 wherein inhibitor of vanilloid receptor 1 type (VR1) activity is a 3-aminobenzamide compound or a pharmaceutically acceptable salt thereof according to any of the aforementioned 1 to 10.
27. A method for treating and/or preventing pain characterized in that the method uses administration of an inhibitor of vanilloid receptor subtype 1 (VR1) activity in combination with stimulation-produced analgesia selected from acupuncture, transcutaneous electroacupuncture stimulation therapy, transcutaneous electrical nerve stimulation therapy, silver spike point (SSP) therapy, peripheral nerve stimulation therapy, spinal cord electrical stimulation therapy, electroconvulsive therapy, laser therapy and low frequency therapy.
28. The treating and/or preventing method according to the aforementioned 27 wherein the inhibitor of vanilloid receptor subtype 1 (VR1) activity is a 3-aminobenzamide compound or a pharmaceutically acceptable salt thereof according to any of the aforementioned 1 to 10.
29. A method for treating and/or preventing postoperative pain characterized in that an inhibitor of vanilloid receptor subtype 1 (VR1) activity is administered after performing a surgical operation selected from cicatrectomy, nerve freezing solidification, peripheral nerve excision, spinal cord dorsal root excision, sympathectomy, spinal cord dorsal root entry zone destruction, cordotomy, and frontal lobe excision.
30. The treating and/or preventing method according to the aforementioned 29 wherein the inhibitor of vanilloid receptor subtype 1 (VR1) activity is a 3-aminobenzamide compound or a pharmaceutically acceptable salt thereof according to any of the aforementioned 1 to 10.

The 3-aminobenzamide compound of the present invention effectively inhibits vanilloid receptor subtype 1 (VR1) activity, and therefore it is effective in the medical treatment and/or prevention of diseases such as pain, acute pain, chronic pain, neuropathic pain, rheumatoid arthritis pain, neuralgia, neuropathy, hyperalgesia, migraine, joint pain, acute postherpetic neuralgia, postherpetic neuralgia, chronic postherpetic neuralgia, postoperative pain, cancer pain, inflammatory pain, interstitial cystitis, posttraumatic neuralgia, diabetic neuropathy, neurodegenerative disease, cerebral apoplexy, ischemic symptom, nerve injury, neurogenic skin disorder, inflammatory disease, pruritus, allergic rhinitis, apoplexy, irritable bowel syndrome, asthma, chronic obstructive pulmonary disease, dermatitis, mucositis, stomach and duodenal ulcer and inflammatory bowel disease, bladder hypersensitivity, and overactive bladder type frequent urination and urinary incontinence. Particularly, it is effective as a therapeutic agent and preventive agent of diseases accompanied with pain condition such as pain, acute pain, chronic pain, neuropathic pain, rheumatoid arthritis pain, neuralgia, neuropathy, hyperalgesia, migraine, joint pain, acute postherpetic neuralgia, postherpetic neuralgia, chronic postherpetic neuralgia, postoperative pain, cancer pain, inflammatory pain, interstitial cystitis, posttraumatic neuralgia, diabetic neuropathy and neurodegenerative disease. In addition, effects by different mechanism from the conventional analgesics are also expected.

### BEST MODE FOR CARRYING OUT THE INVENTION

The definition of each term used in this specification is as follows.

A "C1-6 alkyl group" represents a linear or branched alkyl group having 1 to 6 carbon atoms, and specifically includes a methyl group, an ethyl group, a propyl group, an isopropyl group, a butyl group, an isobutyl group, a sec-butyl group, a tert-butyl group, a pentyl group, an isopentyl group, a tert-pentyl group, a hexyl group, etc. A "C1-6 alkyl group" preferable as R¹, R², R⁴, R⁵ and R⁶ is a C1-4 alkyl group.

A "halogen atom" is a fluorine atom, a chlorine atom, a bromine atom or an iodine atom, and a fluorine atom and a chlorine atom are preferred. A "halogen atom" preferable as R², R⁴, R⁵ and R⁶ is a chlorine atom and a fluorine atom.

A "halo C1-6 alkyl group" is a "C1-6 alkyl group" of the above-mentioned definition substituted with "halogen atom" of the above-mentioned definition, and preferably a halogenated alkyl group in which the alkyl group thereof is a linear or branched alkyl group having 1 to 4 carbon atoms. Specifically, it includes a fluoromethyl group, a difluoromethyl group, a trifluoromethyl group, a bromomethyl group, a chloromethyl group, a 1,2-dichloromethyl group, a 2,2-dichloromethyl group, a 2,2,2-trifluoroethyl group, etc.

A "C1-6 alkoxy group" is an alkoxy group in which the alkyl part thereof is a "C1-6 alkyl group" of the above-mentioned definition. Specifically, it includes a methoxy group, an ethoxy group, a propoxy group, an isopropyloxy group, a butoxy group, an isobutyloxy group, a tert-butyloxy group, a pentyloxy group, a hexyloxy group, etc. Preferred is an alkoxy group in which the alkyl part thereof is a linear or branched chain alkyl group having 1 to 4 carbon atoms. A "C1-6 alkoxy group" preferable as R² and R⁴ is a C1-4 alkoxy group.

A "carbocyclic group" or a "saturated or unsaturated carbocyclic group having 3 to 14 carbon atoms" is a cyclic hydrocarbon group having 3 to 14 carbon atoms, preferably 3 to 8 carbon atoms, which is saturated or unsaturated with a double bond in a part and specifically means an aryl group, a cycloalkyl group, a cycloalkenyl group or a condensed carbocyclic in which these rings are condensed.

Here, an "aryl group" is an aromatic hydrocarbon group having 6 to 14 carbon atoms, and specifically includes a phenyl group, a naphthyl group, a biphenyl group, an anthryl group, an indenyl group, a pentalenyl group, an azulenyl group, a fluorenyl group, a phenanthryl group, etc. Preferably, it is a phenyl group.

Here, a "cycloalkyl group" is a saturated cycloalkyl group having 3 to 8 carbon atoms, and specifically includes a cyclopropyl group, a cyclobutyl group, a cyclopentyl group, a cyclohexyl group, a cycloheptyl group, and a cyclooctyl group.

A "cycloalkenyl group" is a cycloalkenyl group having 3 to 8 carbon atoms, and preferably contains at least one, preferably one or two double bonds. Specifically it includes a cyclopropenyl group, a cyclobutenyl group, a cyclopentenyl group, a cyclopentadienyl group, a cyclohexenyl group, cyclohexadienyl groups (2,4-cyclohexadien-1-yl group, 2,5-cyclohexadien-1-yl group, etc.), a cycloheptenyl group, a cyclooctenyl group, etc.

A "condensed carbocyclic group" in which the "aryl group, " "cycloalkyl group," and "cycloalkenyl group" are condensed specifically includes an indenyl group, an indanyl group, a 1,4-dihydronaphthyl group, a 1,2,3,4-tetrahydronaphthyl group (a 1,2,3,4-tetrahydro-2-naphthyl group, a 5,6,7,8-tetrahydro-2-naphthyl group, etc.), a perhydronaphthyl group, etc.

A "carbocyclic group" preferable as P1 ring and R² is an aryl group, and more preferably a phenyl group.

An "aralkyl group" is an arylalkyl group in which the aryl part thereof is an aryl group as mentioned above, particularly a phenyl group and the alkyl part thereof is a "C1-6 alkyl group" of the above-mentioned definition, and specifically it includes a benzyl group, a phenethyl group, a 3-phenyl propyl group, a 4-phenylbutyl group, a 6-phenyl hexyl group, etc.

A "aralkoxy group" is an arylalkoxy group in which the aryl part thereof is an aryl group as mentioned above, particularly a phenyl group and the alkoxy part thereof is a "C1-6 alkoxy group" of the above-mentioned definition, and, specifically it includes a benzyloxy group, a 3-phenylpropyloxy group, a 4-phenylbutyloxy group, a 6-phenylhexyloxy group, etc. An "aralkoxy group" preferable as R² is a benzyloxy group.

A "cycloalkylalkoxy group" is a cycloalkylalkoxy group in which the cycloalkyl part thereof is a "cycloalkyl group" of the above-mentioned definition and the alkoxy part thereof is a "C1-6 alkoxy group" of the above-mentioned definition, and, specifically it includes a cyclopropylmethoxy group, a cyclobutylmethoxy group, a cyclopentylmethoxy group, a cyclohexylmethoxy group, etc.

An "aryloxy group" is an aryloxy group in which the aryl part thereof is an "aryl group" of the above-mentioned definition and it is specifically a phenoxy group, a naphthyloxy group and a biphenyloxy group.

A "heterocyclic group" or a "saturated or unsaturated "heterocyclic group having at least one hetero atom selected from a nitrogen atom, an oxygen atom and a sulfur atom" means a saturated or unsaturated (including partial unsaturation and complete unsaturation) 5-membered or 6-membered heterocyclic monocyclic ring containing at least one, preferably 1 to 4 hetero atoms selected from a nitrogen atom, an oxygen atom and a sulfur atom besides the carbon atoms, or a condensed ring of these heterocyclic rings or a condensed ring of these heterocyclic rings and a carbocyclic selected from benzene, cyclopentane and cyclohexane.

As "a saturated monocyclic heterocyclic group", a pyrrolidinyl group, a tetrahydrofuryl group, a tetrahydrothienyl group, an imidazolidinyl group, a pyrazolidinyl group, 1,3-dioxanyl group, 1,3-oxathiolanyl group, an oxazolidinyl group, a thiazolidinyl group, a piperidinyl group, a piperazinyl group, a tetrahydropyranyl group, tetrahydrothiopyranyl group, dioxanyl group, morpholinyl group, thiomorpholinyl group, 2-oxopyrrolidinyl group, 2-oxopiperidinyl group, 4-oxopiperidinyl group, 2,6-dioxopiperidinyl group etc. is mentioned. A "nitrogen-containing saturated heterocyclic group" means "the saturated monocyclic heterocyclic group" which have at least one nitrogen atom as an atom which constitutes a ring.

A "unsaturated monocyclic heterocyclic group" includes a pyrrolyl group, a furyl group, a thienyl group, an imidazolyl group, a 1,2-dihydro-2-oxoimidazolyl group, a pyrazolyl group, a diazolyl group, an oxazolyl group, an isoxazolyl group, a thiazolyl group, an isothiazolyl group, a 1,2,4-triazolyl group, a 1,2,3-triazolyl group, a tetrazolyl group, a 1,3,4-oxadiazolyl group, a 1,2,4-oxadiazolyl group, a 1,3,4-thiadiazolyl group, a 1,2,4-thiadiazolyl group, a furazanyl group, a pyridyl group, a pyrimidinyl group, a 3,4-dihydro-4-oxopyrimidinyl group, a pyridazinyl group, a pyrazinyl group, a 1,3,5-triazinyl group, an imidazolinyl group, a pyrazolinyl group and an oxazolinyl group (a 2-oxazolinyl group, a 3-oxazolinyl group and a 4-oxazolinyl group), an isooxazolinyl group, a thiazolinyl group, an isothiazolinyl group, a pyranyl group, a 2-oxopyranyl group, a 2-oxo-2,5-dihydrofuranyl group, a 1,1-dioxo-1H-isothiazolyl group. An "unsaturated monocyclic heterocyclic group" preferable as a P1 ring is a piperidinyl group, a thiazolyl group, a pyridyl group and a quinolyl group, andparticularlypreferable is a pyridyl group.

A "condensed heterocyclic ring" includes an indolyl group (for example, a 4-indolyl group, a 7-indolyl group, etc.), an isoindolyl group, a1,3-dihydro-1,3-dioxo isoindolyl group, a benzofuranyl group (for example, a 4-benzofuranyl group, a 7-benzofuranyl group, etc.), an indazolyl group, an isobenzofuranyl group, a benzothiophenyl group (for example, a 4-benzothiophenyl group, a 7-benzothiophenyl group, etc.) a benzooxazolyl group (for example, a 4-benzooxazolyl group, a 7-benzooxazolyl group, etc.), a benzimidazolyl group (for example, a 4-benzimidazolyl group, a 7-benzimidazolyl group, etc.), a benzothiazolyl group (for example, a 4-benzothiazolyl group, a 7-benzothiazolyl group, etc.), an indolidinyl group, a quinolyl group, an iso quinolyl group, a 1,2-dihydro-2-oxoquinolyl group, a quinazolinyl group, a quinoxalinyl group, a cinnolinyl group, a phthalazinyl group, a quinolidinyl group, a puryl group, a pteridinyl group, an indolinyl group, an isoindolinyl group, a 5,6,7,8-tetrahydroquinolyl group, a 1,2,3,4-tetrahydroquinolyl group, a 2-oxo-1,2,3,4-tetrahydroquinolyl group, a benzo [1,3] dioxolyl group, a 3,4-methylene dioxypyridyl group, a 4,5-ethylene dioxypyrimidinyl group, a chromenyl group, a chromanyl group, an isochromanyl group, etc. A "condensed heterocyclic group" preferable as a P1 ring is a 1,2,3,4-tetrahydroquinolyl group.

The phrase "which may be substituted with one or more substituents" means that substitution can be made with at least one substituent and at most the acceptable largest number of substituents. For example, it means that substitution can be made with 1 to 3 substituents in the case of a methyl group, and means that substitution can be made with 1 to 5 substituents in the case of an ethyl group.

A "C1-6 alkyl group which may be substituted with one or more substituents, which may be the same or different, selected from Group A" means, for example, a methyl group which may be substituted with 1 to 3 substituents and an ethyl group which may be substituted with 1 to 5 substituents, etc.

A "nitrogen-containing saturated heterocyclic group composed of a monocyclic ring formed by R⁷ and R⁸ together with the adjacent nitrogen atom" means a heterocyclic ring composed of a saturated 5- or 6-membered monocyclic ring having at least one nitrogen atom, such as a piperidino group, a morpholino group, a piperazino group and a pyrrolidino group.

The sentence "two R⁴ groups may together form =0" means that when m is two or more, two R⁴ groups of them together form =0.

The sentence " the C1-6 alkyl group, C1-6 alkoxy group, halo C1-6 alkoxy group, and the nitrogen-containing saturated heterocyclic ring composed of a monocyclic ring in the above 1) to 11) may be further substituted with one or more substituents selected from Group A" means, for example, that an alkyl group in R⁷, R⁸ or R⁹ may be further substituted with a halogen atom or a hydroxyl group.

The sentence "R¹ and R² may together form -CH₂-CH₂-O- bond between the adjacent nitrogen atom and carbon atom" means that a -CH₂-CH₂-O- bond is formed between the nitrogen atom adjacent to R¹ and the carbon atom of benzene ring adjacent to R² and it forms a 2H-benzo [1,4] oxazine ring together with the adjacent benzene ring.

The number m is an integer of 1 to 5, preferably 1 to 3 and more preferably 1 to 2.

The number n is an integer of or 1 to 4, and preferably 0 or 1 to 2.

The sentence "R⁵ and R⁶ are the same or different, and each represents 1 or 2 substituents selected from (1) hydrogen atom or (2) said Group B" means that both R⁵ and R⁶ are hydrogen atoms, or one of them is a hydrogen atom and the other is a substituent selected from Group B, or they are the same or different two substituents selected from Group B.

In the general formula [1], X is preferably -N=; m is preferably 1 to 3 and particularly preferably 1 to 2; P1 ring is preferably an aromatic carbocyclic group composed of a monocyclic ring such as a phenyl group or a heterocyclic group such as a pyridyl group or a condensed heterocyclic group such as 1,2,3,4-tetrahydroquinolyl group; R¹ is preferably a C1-6 alkoxy group which may be preferably substituted with one or more substituents selected from a hydroxyl group, a C1-6 alkoxy group, -NR⁷R⁸, -CONR⁷R⁸ (wherein R⁷ and R⁸ are preferably C1-6 alkyl groups which may be substituted with a hydrogen atom or a hydroxyl group) and -COR⁹ (wherein R⁹ is a hydroxyl group or a C1-6 alkoxy group); n is preferably an integer of 0, 1 to 2; R² is preferably a halogen atom, a hydroxyl group, a C1-6 alkyl group defined in the above-mentioned Group B, a C1-6 alkoxy group defined in the above-mentioned Group B, a carbocyclic group defined in the above-mentioned Group B, or an aralkoxy group defined in the above-mentioned Group B, and particularly preferably a halogen atom, a hydroxyl group, a C1-4 alkyl group, a phenyl group, a phenoxy group or a C1-4 alkoxy group (wherein this alkoxy group may be substituted with a hydroxyl group, a C1-4 alkoxy group, -CONR⁷R⁸ (wherein R⁷ and R⁸ are preferably hydrogen atoms or C1-6 alkyl groups) or -COR⁹ (wherein R⁹ is preferably a hydroxyl group)); R³ is preferably a hydrogen atom or a C1-4 alkyl group, particularly a hydrogen atom; R⁴ is preferably a halogen atom, a C1-4 alkyl group, a halo C1-4 alkyl group, a C1-4 alkoxy group, a halo C1-4 alkoxy group, and -CONR¹²R¹³ (wherein R¹² and R¹³ are preferably hydrogen atoms or C1-6 alkyl groups) or a nitrogen-containing saturated 6-membered heterocyclic group composed of a monocyclic ring.

The "pharmaceutically acceptable salt" may be any kind of salt as long as it forms a nontoxic salt with a compound represented by the above-mentioned general formula [1], and can be obtained by reacting it with, for example, an inorganic acid such as hydrochloric acid, sulfuric acid, phosphoric acid, or hydrobromic acid; an organic acid such as oxalic acid, malonic acid, citric acid, fumaric acid, lactic acid, malic acid, succinic acid, tartaric acid, acetic acid, trifluoroacetic acid, gluconic acid, ascorbic acid, methylsulfonic acid, or benzylsulfonic acid; an inorganic base such as sodium hydroxide, potassium hydroxide, calcium hydroxide, magnesium hydroxide, or ammonium hydroxide; an organic base such as methylamine, diethylamine, triethylamine, triethanolamine, ethylenediamine, tris(hydroxymethyl)methylamine, guanidine, choline, or cinchonine; or an amino acid such as lysine, arginine or alanine. A hydrated compound, hydrate and solvate of each compound are also included in the present invention.

In addition, various isomers exist for the compound represented by the above-mentioned general formula [1]. For example, E isomer and Z isomer exist as geometric isomers, and when an asymmetric carbon atom exists, enantiomers and diastereomers exist as stereoisomers based on these, and tautomers may exist. Therefore, all of these isomers and the mixtures thereof are included in the range of the present invention. In addition, the present invention also encompasses prodrug compounds of these compounds and metabolite compounds as equivalent compounds besides the compound represented by the above-mentioned general formula [1].

A "prodrug" is a derivative of the compound of the present invention having a group which may be decomposed chemically or metabolically and after administered to a living body, it goes through a chemical change to a compound which has an activity as a drug and exhibits original pharmacological effect, and complexes and salts not by a covalent bond are included.

A prodrug is used for improving absorption upon oral administration or targeting to a target site. Moieties to be modified for forming a prodrug include reactive functional groups such as a hydroxyl group, a carboxyl group, an amino group, and a thiol group in the compound of the present invention. Specific examples of the modifying group for a hydroxyl group include an acetyl group, a propionyl group, an isobutyryl group, a pivaloyl group, a benzoyl group, a 4-methylbenzoyl group, a dimethylcarbamoyl group, a sulfo group, etc. Specific examples of the modifying group for a carboxyl group include an ethyl group, a pivaloyloxymethyl group, a 1-(acetyloxy)ethyl group, a1-(ethoxycarbonyloxy)ethyl group, a 1-(cyclohexyloxycarbonyloxy)ethyl group, a carboxylmethyl group, a methyl (5-methyl-2-oxo-1,3-dioxol-4-yl) group, a phenyl group, an o-tolyl group, etc. Specific examples of the modifying group for an amino group include a hexylcarbamoyl group, a 3-methylthio-1-(acetylamino)propylcarbonyl group, a 1-sulfo-1-(3-ethoxy-4-hydroxyphenyl)methyl group, a methyl(5-methyl-2-oxo-1,3-dioxol-4-yl) group, etc.

A "pharmaceutical composition" encompasses a combination drug with another drugs, etc., besides the so-called "composition" which comprises an active ingredient as a drug and a combinational agent, etc. Needless to say, the pharmaceutical composition of the present invention may be used in combination with any kind of other drugs as long as it is permitted in the medical scene. Therefore, it can also be said that this pharmaceutical composition is a pharmaceutical composition for the combined use with other drugs.

A "pain" means every type of pain condition no matter what the condition is (for example, no matter whether it is a dull pain or a sharp pain, chronic or acute, etc.), no matter which disease causes the pain (for example, no matter whether the pain is resulted from rheumatism, or the pain resulted from cancer, etc.). Therefore, the "pain" as used herein encompasses, in addition to the so-called "pain, " acute pain, chronic pain, neuropathic pain, rheumatoid arthritis pain, neuralgia, neuropathy, hyperalgesia, migraine, joint pain, acute postherpetic neuralgia, postherpetic neuralgia, chronic postherpetic neuralgia, postoperative pain, cancer pain, inflammatory pain, interstitial cystitis, posttraumatic neuralgia, diabetic neuropathy, and neurodegenerative disease.

An "inhibitor of vanilloid receptor subtype 1 (VR1) activity" means a substance which inhibits the function of the vanilloid receptor subtype 1 as an ion channel, and eliminates or attenuates the activity. Specifically, it includes vanilloid receptor subtype 1 antagonist, etc. The vanilloid receptor subtype 1 antagonist means a substance which inhibits the effect of the agonist which acts on the vanilloid receptor subtype 1, thereby inhibiting the function of the vanilloid receptor subtype 1 as an ion channel. The inhibitor of the present invention has not to compete with the agonist but may also inhibit the function as a VR1 ion channel. Specifically, agonists which act on the vanilloid receptor subtype 1 include capsaicin, capsaicin derivatives, acid stimulation (proton), heat stimulation, etc., the inhibitor of vanilloid receptor subtype 1 (VR1) activity may be a substance which inhibits the Ca²⁺ inflow into the cell caused by agonist stimulation of capsaicin, acidstimulation (proton) or heat stimulation.

The pharmaceutical composition of the present invention can be administered to human as well as other mammals (mouse, rat, hamster, rabbit, cat, dog, cow, horse, sheep, monkey, etc.). Therefore, the pharmaceutical composition of the present invention is useful also as a drug for animal not to mention for human.

When the compound of the present invention is used as a pharmaceutical preparation, it can be mixed with a pharmaceutically acceptable carrier usually known in itself, excipient, diluent, extender, disintegrating agent, stabilizer, preservative, buffer, emulsifier, flavor, colorant, sweetener, thickner, corrigent, dissolution auxiliary agent, and other additive agents, specifically water, plant oil, alcohol such as ethanol or benzyl alcohol, carbohydrates such as polyethylene glycol, glycerol triacetate, gelatin, lactose and starch, magnesium stearate, talc, lanolin, vaseline, etc. to prepare a drug in the form such as tablet, pill, powder, granule, suppository, injection agent, eye-drops, liquid medicine, capsule agent, troche, aerosol agent, elixir agent, suspension, emulsion and syrup for systemic or local administration by oral or parenteral route.

Although the dosage varies depending on age, weight, condition, therapeutical effect, administration methods, etc., it is usually administered at a dose in the range of 0.01 mg to 1 g per dose, 1 time to several times per day, to adults, in the form of an oral preparate or injection preparation such as an intravenous injection, etc.

"Preventing" means suppressing the onset of neuralgia or chronicity of neuralgia prophylactically. Specifically included is prophylactically suppressing the onset of acute postherpetic neuralgia, onset of postherpetic neuralgia, transition to postherpetic neuralgia from acute herpetic pain, chronicity of postherpetic neuralgia, onset of postoperative pain, chronicity of postoperative pain, onset of symptoms of cancer pain, chronicity of cancer pain, onset of symptoms of inflammatory pain, onset of interstitial cystitis, chronicity of inflammatory pain, onset of posttraumatic neuralgia or chronicity of posttraumatic neuralgia.

The compound and pharmaceutical composition of the present invention can be used together with another agent or two or more other agents following a general method currently performed in the usual medical site.

A "combination drug" means a drug characterized in that it is a compounded agent containing pharmaceutical compositions or agents which can be combined, a drug characterized in that it is a kit containing pharmaceutical compositions or agents which can be combined, a drug characterized in that pharmaceutical compositions or agents which can be combined are respectively administered in the same or different administration route.

Although there are various compounds which can be used in combination with the compound of the present invention, particularly preferred are an anti-virus agent, an antidepressant, an anticonvulsant, an antiarrhythmic drug, a local anesthetic, an anesthetic drug, aN-methyl-D-aspartate receptor antagonist, adrenal cortical steroid, a nerve block, a nonsteroidal antiinflammatory analgesic, narcotics, an antagonist analgesic, an ?₂-adrenaline receptor agonist, a stimulation analgesic method, drugs for external application, a calcium channel antagonist, and a potassium channel opener.

The anti-virus agent specifically includes vidarabine, acyclovir, ganciclovir, zidovudine, didanosine, amantadine, and idoxuridine, interferon, etc.

The antidepressant specifically includes amitriptyline, imipramine, clomipramine, trimipramine, lofepramine, dosulepin, desipramine, amoxapine, nortriptyline, fluoxetine, fluvoxamine, maprotiline, mianserin, setiptiline, trazodone, etc.

The anticonvulsant specifically includes gabapentin, pregabalin, phenobarbital, primidone, phenytoin, mephenytoin, nirvanol, ethotoin, trimethadione, ethosuximide, acetylpheneturide, carbamazepine, zonisamide, acetazolamide, diazepam, clonazepam, nitrazepam, diphenylhydantoin, valproic acid, baclofen, etc.

The antiarrhythmic drug specifically includes quinidine, disopyramide, procainamide, ajmaline, prajmalium, cibenzoline, lidocaine, mexiletine, aprindine, tonicaid, phenytoin, flecainide, pilcicainide, propafenone, propranolol, amiodarone, verapamil, bepridil, etc.

The local anesthetic specifically includes lidocaine, mexiletine, cocaine, procaine, bupivacaine, mepivacaine, prilocaine, tetracaine, dibucaine, ethyl aminobenzoate, etc.

The anesthetic drug specifically includes benzodiazepine, diazepam, midazolam, thiopental, thiamylal, propofol, baclofen, droperidol, sufentanil, etc. are mentioned. The N-methyl-D-aspartate receptor antagonist specifically includes ketamine, dextromethorphan, memantine, amantadine, etc. are included.

The adrenal cortical steroid specifically includes cortisol, cortisone, prednisolone, triamcinolone, dexamethasone, betamethasone, paramethasone, fluocinolone acetonide, fluocinonide, beclomethasone, fludrocortisone, etc.

The nerve block specifically includes stellate ganglion block, epidural ganglion block, brachial plexus ganglion block, nerve root block, thoracic/lumbar sympathetic ganglion, trigger point block, subarachnoid ganglion block, trigeminal nerve block, sympathetic nerve block, local infiltration block, peripheral nerve block, etc.

The nonsteroidal antiinflammatory analgesic specifically includes celecoxib, rofecoxib, etodolac, meloxicam, nimesulid, sodium diclofenac, mefenamic acid, zaltoprofen, sodium loxoprofen, sulindac, nabumetone, diflunisal, piroxicam, ibuprofen, naproxen, fenoprofen, acetylsalicylic acid, tolmetin, indomethacin, flurbiprofen, oxaprozin, ketoprofen, mofezolac, acetaminophen, ketorolac, zomepirac, nitroaspirin, tiaprofen, ampiroxicam, tiaramide, epirizole, etc.

The narcotics specifically include morphine, fentanyl, oxycodone, methadon, codeine, cocaine, pethidine, opium, ipecac, etc.

The antagonist analgesic specifically includes pentagyn, buprenorphine, nalorphine, cyclazocine, butorphanol, etc.

The ?₂-adrenaline receptor agonist specifically includes clonidine, dexmedetomidine, tizanidine, guanfacine, guanabenz, etc.

The medicine for external application specifically includes capsaicin cream etc.

The stimulation analgesic method specifically includes acupuncture, a percutaneous electricity needle stimulation therapy, a percutaneous electricity nerve stimulation therapy, a silver spike point (SSP) treatment, a peripheral nerve stimulus, a spine electricity stimulus, an electric spasm treatment, laser surgery, a low-frequency therapy, etc.

In addition, the compound of the present invention can be used following the general method usually performed in the art by administration after performing a surgical operation to prevent or treat pain. Although various surgical operations can be performed in combination with the compound of the present invention, cicatrectomy, nerve freezing, peripheral nerve excision, spinal dorsal root excision, sympathectomy, spinal cord dorsal root entry zone destruction, cordotomy, and frontal lobe excision are particularly preferable.

Although application of the compound of the present invention has been described mainly as a use for preventing or treating pain, the compound of the present invention can be applied to the conditions in which C fibers participates, for example, pruritus, allergic and allergic rhinitis, overactive bladder type frequent urination and urinary incontinence, apoplexy, irritable bowel syndrome, respiratory ailment such as asthma and a chronic obstructive pulmonary disease, dermatitis, mucositis, stomach and duodenal ulcer, inflammatory bowel disease, etc.

Next, the preparation methods of the compound represented by the general formula (1) according to the present invention are specifically explained. However, it is needless to say that the present invention is not limited to these preparation methods. In preparing the compound of the present invention, the reactions can be performed in any order starting from the part in which the reaction is more easy to be performed suitably. In addition, substituent conversion (conversion or further modification of substituent) step may be suitably inserted between the respective steps. In order to promote progress of the reaction, reagents other than the illustrated reagents can be used suitably. The compounds used as raw material compounds for which preparation method is not described are commercially available, or compounds which can be prepared easily combining known synthetic reactions.

All the compounds obtained at each step can be isolated and purified following a usual method but they can be used in the next step without performing isolation and/or purification depending on the case. 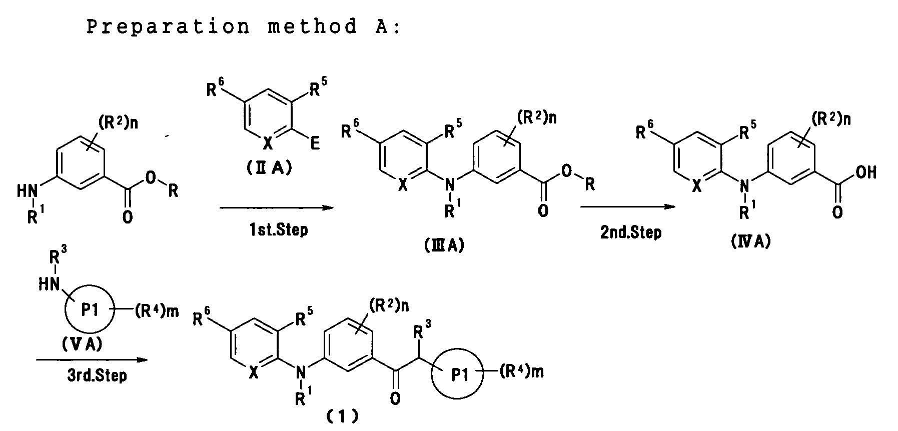 (wherein R represents an alkyl group and forms an ester easily derived to a carboxylic acid by hydrolysis or catalytic hydrogenation reaction. E represents a halogen atom or a sulfonyloxy group such as 3-nitrobenzenesulfonyloxy group, p-toluenesulfonyloxy group, benzenesulfonyloxy group, p-bromobenzenesulfonyloxygroup, methanesulfonyloxygroup or trifluoromethanesulfonyloxy group. Although R¹ is the same as above, when a reactant substituent is included, for example, in the case of a hydroxyl group, a group substituted with a protecting group such as a tetrahydropyranyl group and a tert-butyldimethylsilyl group are also included. Each of the other symbols is the same as above respectively)

### First Step:

This is a reaction for obtaining a compound (IIIA) by the palladium catalyzed Buchwald/Hartwig type amination reaction from a compound (IA) and a compound (IIA).

The compound (IIIA) can be obtained by reacting the compound (IA) with the compound (IIA) in toluene, 1,4-dioxane, tetrahydrofuran or the like or a mixed solvent of these, using a mixture of palladium catalyst such as palladium acetate, bis(diphenylphosphino)ferrocene palladium chloride (II), tris(dibenzylideneacetone)dipalladium and 2,2'-'bis(diphenylphosphino)-1,1'-binaphthyl together with a base such as sodium carbonate, tripotassium phosphate (K₃PO₄), potassium carbonate, cesium carbonate, sodium bicarbonate, potassium bicarbonate, potassium tert-butoxide and sodium tert-butoxide.

### Second Step:

This is a step for obtaining a carboxylic acid (IVA) by removing R in the compound (IIIA).

WhenRisamethyl, ethyl, propyl group, etc., the compound (IVA) can be obtained by hydrolyzing the compound (IIIA) in water, methanol, ethanol, propanol, tetrahydrofuran, etc., or a mixed solvent thereof using sodium hydroxide, potassium hydroxide, lithium hydroxide, potassium carbonate, sodium carbonate etc., or a base of these aqueous solutions.

When R is a tert-butyl group, the compound (IVA) can be obtained by reacting the compound (IIIA) without solvent or in water,methanol, ethanol, propanol, tetrahydrofuran, etc., or a mixed solvent thereof using an acid such as hydrochloric acid and trifluoroacetic acid.

When R is a benzyl, paramethoxy benzyl group, etc., the compound (IVA) can be obtained by reacting in methanol, ethanol, propanol, tetrahydrofuran, etc., or a mixed solvent thereof using hydrogen or ammonium formate, etc. in the presence of a palladium carbon catalyst, etc.

### Third Step:

This is a step for obtaining a compound (1) by condensation reaction of (IVA) and (VA). The condensation reaction includes a method by using a condensing agent and a method via an acid chloride, etc.

When a direct condensation reaction is performed using a condensing agent, the compound (1) can be obtained by reacting a compound (IVA) with the compound (VA) using a condensing agent such as dicyclohexylcarbodiimide, 1-ethyl-3-(3-dimethylaminopropyl)-carbodiimide in N,N-dimethylformamide, methylenechloride, chloroform, etc., or a mixed solvent thereof. In this case, it is preferable to add an additive such as hydroxybenzotriazole and N-hydroxysuccinic acid imide.

When a reaction via an acid chloride is performed, the compound (1) can be obtained by reacting the compound (IVA) with thionyl chloride, oxalyl chloride, etc. in chloroform, methylene chloride, tetrahydrofuran, pyridine, etc., or a mixed solvent thereof to obtain an acid chloride of the compound (IVA) and reacting this with the compound (VA) in the presence of a base such as triethylamine and pyridine, in toluene, chloroform, tetrahydrofuran, etc., or a mixed solvent thereof.

### Substitution group conversion process:

When a substituent conversion reaction for R¹, R², R⁵ or R⁶ is to be included in the middle of the preparing process, it can be inserted after the first step or the third step.

For example, when R¹ is an acetoxyethyl group, R¹ can be converted to a hydroxyethyl group by adding an aqueous solution of sodium hydroxide, potassium hydroxide, lithium hydroxide, etc., and reacting in tetrahydrofuran, methanol, etc., or a mixed solvent thereof after the first step is ended.

For example, when R² is a methoxyethyloxy group, R² can be converted to a hydroxyl group by adding hydrochloric acid and reacting in tetrahydrofuran, methanol, etc., or a mixed solvent thereof after the first step or the third step is ended.

For example, when R² is a hydroxyl group, R² can be converted to an alkoxy group by reacting with an alkyl halide, etc. in the presence of a base such as sodium carbonate, triethylamine, in N,N-dimethylformamide and acetone, etc., after the first step or the third step is ended.

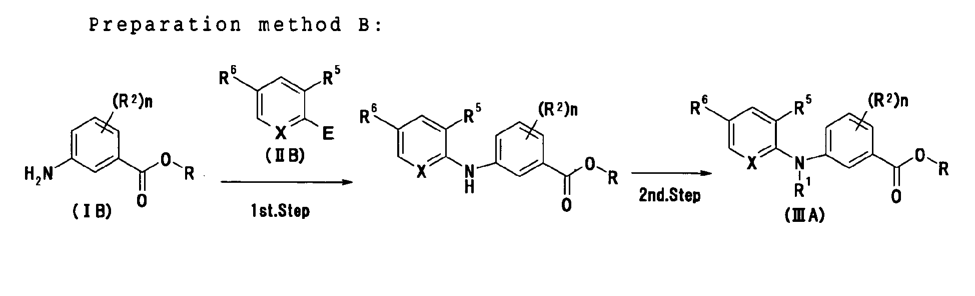

This is an alternative method of preparing (IIIA) in the preparation method A.

The symbols in the formula are the same as above.

### First Step:

This is a reaction for obtaining a compound (IIIB) by the palladium catalyzed Buchwald/Hartwig type amination reaction from a compound (IB) and a compound (IIB).

The compound (IIIB) can be obtained by reacting the compound (IB) with the compound (IIB) in toluene, 1,4-dioxane, tetrahydrofuran or the like or a mixed solvent of these, using a mixture of palladium catalyst such as palladium acetate, bis(diphenylphosphino)ferrocene palladium chloride (II), tris(dibenzylideneacetone)dipalladium and 2,2'-bis(diphenylphosphino)-1,1'-binaphthyl together with a base such as sodium carbonate, tripotassium phosphate (K₃PO₄), potassium carbonate, cesium carbonate, sodium bicarbonate, potassium bicarbonate, potassium tert-butoxide and sodium tert-butoxide.

### Second Step:

This is a step to carry out N-alkylation of the compound (IIIB).

The compound (IIIA) can be obtained by reacting an alkylation reagent such as alkyl halide, alkyl p-toluenesulfonate, alkyl methanesulfonate, alkyl trifluoromethanesulfonate and dialkyl sulfuric acid with the compound (IIIB) in N,N-dimethylformamide, dimethylsulfoxide, tetrahydrofuran, acetone, and water, etc., or a mixed solvent thereof in the presence of a base such as sodium hydride, sodium hydroxide, potassium hydroxide, sodium carbonate, potassium carbonate, sodium bicarbonate, potassium bicarbonate, triethylamine, N,N-diisopropylethylamine, N-methylmorpholine, pyridine and 4-dimethylaminopyridine at 0?C to refluxing temperature. 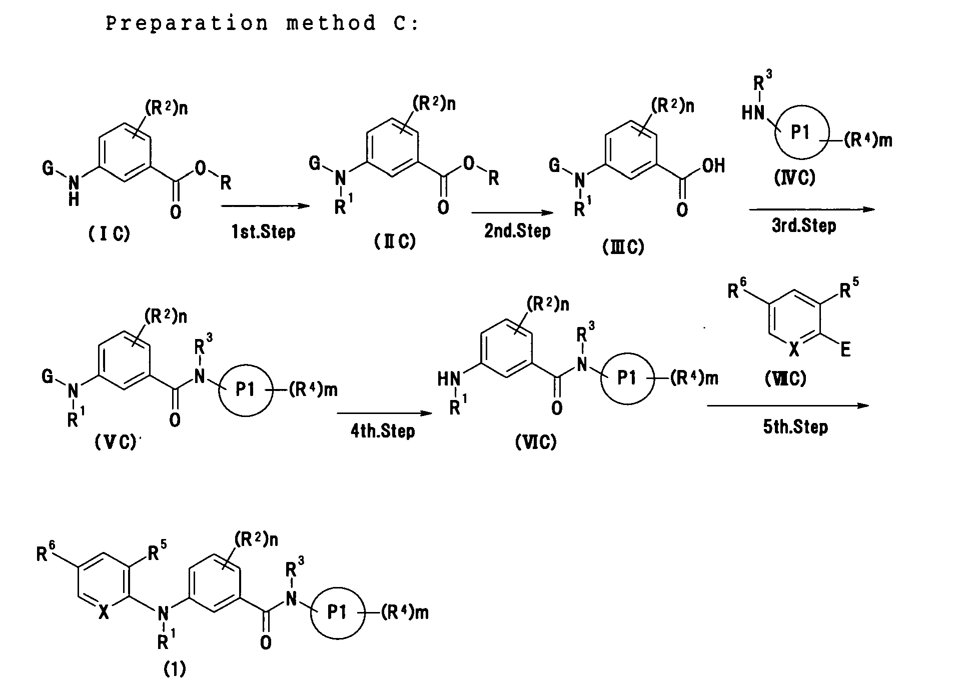 (wherein G means an amino protecting group which is generally used in organic synthesis reaction and can be easily attached and detached, i.e., a tert-butoxy carbonyl group, a benzyloxycarbonyl group, ap-methoxybenzyloxycarbonylgroup, a chloroacetyl group, etc. and a protecting group is selected and used so that it may be convenient under the conditions of each step or in performing substituent conversion. The other symbols are the same as above respectively)

### First Step:

This is a step to carry out N-alkylation of the compound (IC) .

The compound (IIC) can be obtained by reacting an alkylation reagent such as alkyl halide, alkyl p-toluenesulfonate, alkyl methanesulfonate, alkyl trifluoromethanesulfonate and dialkyl sulfuric acid with the compound (IC) in N,N-dimethylformamide, dimethylsulfoxide, tetrahydrofuran, acetone, and water, etc., or a mixed solvent thereof in the presence of a base such as sodium hydride, sodium hydroxide, potassium hydroxide, sodium carbonate, potassium carbonate, sodium bicarbonate, potassium bicarbonate, triethylamine, N,N-diisopropylethylamine,
N-methylmorpholine, pyridine and 4-dimethylaminopyridine at 0?C to refluxing temperature.

### Second Step:

This is a step for obtaining a carboxylic acid (IIIC) by removing R in the compound (IIC).

When R is a methyl, ethyl, propyl group, etc., G is a tert-butoxy carbonyl group, a benzyloxycarbonyl group, a p-methoxybenzyloxycarbonylgroup, a chloroacetyl group, etc. The compound (IIIC) can be obtained by reacting by hydrolyzing the compound (IIC) using sodium hydroxide,potassium hydroxide, lithium hydroxide,potassium carbonate, and sodium carbonate, etc. , or a base of these solutions in water, methanol, ethanol, propanol, and tetrahydrofuran, etc., or a mixed solvent thereof.

When R is a tert-butyl group, G is a benzyloxycarbonyl group, a p-methoxybenzyloxycarbonyl group, a chloroacetyl group, etc. The compound (IIIC) can be obtained by reacting the compound (IIC) without solvent or in water, methanol, ethanol, propanol, tetrahydrofuran and chloroform, etc., or a mixed solvent thereof using an acid such as hydrochloric acid and trifluoroacetic acid.

When R is a benzyl, paramethoxy benzyl group, etc., G is a tert-butoxy carbonyl group, a chloroacetyl group, etc. The compound (IIIC) can be obtained by reacting the compound (IIC) in methanol, ethanol, propanol, tetrahydrofuran, etc., or a mixed solvent thereof using hydrogen or ammonium formate, etc. in the presence of a palladium carbon catalyst, etc.

### Third Step:

This is a step for obtaining a compound (VC) by condensation reaction of (IIIC) and (IVC). The condensation reaction includes a method by using a condensing agent and a method via an acid chloride, etc.

When a direct condensation reaction is performed using a condensing agent, the compound (VC) can be obtained by reacting the compound (IIIC) with the compound (IVC) using a condensing agent such as dicyclohexylcarbodiimide, 1-ethyl-3-(3-dimethylaminopropyl)-carbodiimide in N,N-dimethylformamide, methylenechloride, chloroform, etc., or a mixed solvent thereof. In this case, it is preferable to add an additive such as hydroxybenzotriazole and N-hydroxysuccinic acid imide.

When a reaction via an acid chloride is performed, G is a benzyloxycarbonyl group, a p-methoxybenzyloxycarbonyl group, a chloroacetyl group, etc. The compound (VC) can be obtained by reacting the compound (IIIC) with thionyl chloride, oxalyl chloride, etc. in chloroform, methylene chloride, tetrahydrofuran, etc., or a mixed solvent thereof to obtain an acid chloride of the compound (IIIC) and reacting this with the compound (IVC) in the presence of a base such as triethylamine and pyridine, in toluene, chloroform, tetrahydrofuran, etc., or a mixed solvent thereof.

### Fourth Step:

This is a step to remove the protecting group G.

When G is a tert-butoxy carbonyl group, the compound (VIC) can be obtained by reacting the compound (VC) without solvent orinwater, methanol, ethanol, propanol, tetrahydrofuran and chloroform, etc., or a mixed solvent thereof using an acid such as hydrochloric acid and trifluoroacetic acid.

When G is a benzyloxycarbonyl group or a p-methoxybenzyloxycarbonyl group, the compound (VIC) can be obtained by reacting the compound (VC) in methanol, ethanol, propanol, tetrahydrofuran, etc., or a mixed solvent thereof using hydrogen or ammonium formate, etc. in the presence of a palladium carbon catalyst, etc.

When G is a chloroacetyl group, the compound (VIC) can be obtained by reacting the compound (VC) with thiourea in water, methanol, ethanol, propanol, tetrahydrofuran, etc., or a mixed solvent thereof.

### Fifth Step:

This is a reaction for obtaining a compound (1) by the palladium catalyzed Buchwald/Hartwig type amination reaction from a compound (VIC) and a compound (VIIC).

The compound (1) can be obtained by reacting the compound (VIC) with the compound (VIIC) in toluene, 1,4-dioxane, tetrahydrofuran or the like or a mixed solvent of these, using a mixture of palladium catalyst such as palladium acetate, bis(diphenylphosphino)ferrocene palladium chloride (II), tris(dibenzylideneacetone)dipalladium and 2,2'-'bis(diphenylphosphino)-1,1'-binaphthyl together with a base such as sodium carbonate, tripotassium phosphate (K₃PO₄), potassium carbonate, cesium carbonate, sodium bicarbonate, potassium bicarbonate, potassium tert-butoxide and sodium tert-butoxide.

### Example 1

### Preparation of N-(4-tert-butylphenyl)-3-[N-(3-chloropyridin-2-yl)-N-methyl-amino]benzamide hydrochloride salt:

### Step 1:

### Preparation of methyl 3-(tert-butoxycarbamide)benzoate:

Methyl 3-aminobenzoate (3.02 g) was dissolved in tetrahydrofuran (30 mL), triethylamine (2.79 mL) and di-tert-butyl dicarbonate (4.47 g) were added, and the mixture was stirred at room temperature for 1 hour. The reaction mixture was partitioned between ethyl acetate and water, and the ethyl acetate layer was washed with a saturated brine, dried over anhydrous sodium sulfate and concentrated. The obtained residue was purified by silica gel chromatography (n-hexane: ethyl acetate = 2:1) to obtain the title compound (2.86 g).

### Step 2:

### Preparation of methyl 3-(N-tert-butoxycarbonyl-N-methyl)aminobenzoate:

Sodium hydride (60%) (530 mg) was suspended in tetrahydrofuran (5 mL), and a solution of methyl 3-(tert-butoxycarbamide)benzoate (2.86 g), which was obtained in the preceding step, in N,N-dimethylformamide (20 mL) was dropwised under ice-cooled stirring, and the mixture was then stirred at room temperature for 1 hour. The reaction mixture was ice-cooled, methyl iodide (1.12 mL) was added and the mixture was stirred for 30 minutes at room temperature. The reaction mixture was partitioned between water-ethyl acetate and the ethyl acetate layer was washed with a saturated brine, dried with a saturated brine and concentrated to obtain the residue containing the title compound.

### Step 3:

### Preparation of 3-(N-tert-butoxycarbonyl-N-methyl)aminobenzoic acid:

The residue containing methyl 3-(N-tert-butoxycarbonyl-N-methyl)aminobenzoate obtained in the preceding step was dissolved in methanol (15 mL), 4 M sodium hydroxide (5 mL) was added, and the mixture was stirred for 30 minutes at 70?C. The reaction mixture was allowed to cool, 1M hydrochloric acid (20 mL) was added, and partitioned between ethyl acetate and water after neutralization. The ethyl acetate layer was washed with a saturated brine, dried over anhydrous sodium sulfate and concentrated to obtain the residue containing the title compound.

### Step 4:

### Preparation of 3-[(N-tert-butoxycarbonyl-N-methyl)amino]-N-(4-tert-butylphenyl)benzamide:

The residue containing 3-(N-tert-butoxycarbonyl-N-methyl)aminobenzoate obtained in the preceding step was dissolved in N,N-dimethylformamide (15 mL), 4-tert-butylaniline (1.96 g), 1-hydroxybenzotriazole (2.02 g) and 1-ethyl-3-(3-dimethylaminopropyl)-carbodiimide hydrochloride salt (2. 53 g) were added in this order, and the mixture was stirred at room temperature for 15 hours. Water was added to the reaction mixture and agitated, and obtained white solid was filtered and dried to obtain the title compound (4.87 g) .

### Step 5:

### Preparation of N-(4-tert-butylphenyl)-3-(N-methyl/amino)benzamide:

3-[(N-tert-butoxycarbonyl-N-methyl)amino]-N-(4-tert-butylphenyl) benzamide (4.87 g) obtained in the preceding step was dissolved in chloroform (15 mL) and trifluoroacetic acid (5 mL) was adding and the mixture was stirred for 6 hours at room temperature. The reaction mixture was concentrated and partitioned between a saturated sodium bicarbonate aqueous solution and ethyl acetate. The ethyl acetate layer was washed with a saturated brine, dried over anhydrous sodium sulfate and concentrated. Theobtainedresiduewaspurifiedbysilica gel chromatography (n-hexane: ethyl acetate = 2:1) to obtain the title compound (2.92 g).

### Step 6:

### Preparation of N-(4-tert-butylphenyl)-3-[N-(3-chloropyridin-2-yl)-N-methyl-amino]benzamide hydrochloride salt:

N-(4-tert-butylphenyl)-3-methylamino-benzamide (687 mg) obtained in the preceding step was dissolved in toluene (6 mL), 2,3-dichloropyridine (300 mg), tris(dibenzylideneacetone) dipalladium (92 mg), 2,2'-bis(diphenylphosphino)-1,1'-binaphthyl (151 mg), and sodium tert-butoxide (390 mg) were added in this order, and the mixture was stirred at 70?C for 6 hours and at 90?C for further 12 hours. The reaction mixture was partitioned between ethyl acetate and water after standing to cool, and the ethyl acetate layer was washed with a saturated brine, dried over anhydrous sodium sulfate and concentrated. The obtained residue was purified by silica gel chromatography (hexane: tetrahydrofuran = 2:1, chloroform: methanol =19:1), and the title compound (265 mg) in pale yellow solid precipitated by the addition of 4N hydrochloric acid / ethyl acetate was obtained.

### Example 2

### Preparation of N-(4-tert-butylphenyl)-3-[N-(pyridin-2-yl)-N-methyl-amino]benzamide hydrochloride salt:

The similar reaction was performed in the step 6 of Example 1 using 2-bromopyridine instead of 2,3-dichloropyridine to obtain the title compound (373 mg).

### Example 3

### Preparation of N-(4-tert-butylphenyl)-3-[N-(3-chloropyridin-2-yl)-N-ethyl-amino]benzamide:

### Step 1:

### Preparation of methyl 3-(3-chloropyridin-2-yl)amino-benzoate:

Methyl 3-aminobenzoate (1 g) was suspended in toluene (10 mL), 2,3-dichloropyridine (890 mg), tris(dibenzylideneacetone) dipalladium (275 mg), 2,2'-bis(diphenylphosphino)-1,1'-binaphthyl (450 mg) and cesium carbonate (2.94 g) were added in this order, and the mixture was stirred overnight at 80?C. After the reaction mixture was allowed to cool, ethyl acetate-water was added, insoluble substances were filtered off, the reaction liquid was then partitioned, and the obtained ethyl acetate layer was washed with a saturated brine, dried over anhydrous sodium sulfate and concentrated. The obtained residue was purified by silica gel chromatography (hexane: ethyl acetate system) to obtain the title compound (320 mg).

### Step 2:

### Preparation of methyl 3-[N-(3-chloropyridin-2-yl)-N-ethyl aminobenzoate:

Methyl 3-(3-chloropyridin-2-yl)aminobenzoate (300 mg) obtained in the preceding step was dissolved in N,N-dimethylformamide (5 mL), and 60% sodium hydride (46 mg) was added under ice-cooled stirring, and then ethyl iodide (0,092 mL) was added, and the mixture was stirred at room temperature for 3 hours. After a small amount of acetic acid was added to the reaction mixture to stop the reaction, the reaction mixture was concentrated and partitioned between ethyl acetate and water. The ethyl acetate layer was washed with a saturated brine, dried over anhydrous sodium sulfate and concentrated. Theobtainedresiduewaspurifiedbysilica gel chromatography (hexane: ethyl acetate =5:1, hexane-acetone = 8:1) to obtain the title compound (80 mg).

### Step 3:

### Preparation of 3-[N-(3-chloropyridin-2-yl)-N-ethyl]aminobenzoic acid:

Methyl 3-[N-(3-chloropyridin-2-yl)-N-ethyl]aminobenzoate (80 mg) obtained in the preceding step was dissolved in methanol (1 mL), 4 M sodium hydroxide (0.5 mL) was added, and the mixture was stirred at 60?C for 3 hours. The reaction mixture was concentrated after standing to cool, and partitioned between ethyl acetate and water. The obtained aqueous layer was acidified with a diluted hydrochloric acid, and this was extracted with ethyl acetate. The ethyl acetate layer was dried over anhydrous sodium sulfate and concentrated to obtain the title compound (60 mg).

### Step 4:

### Preparation of N-(4-tert-butylphenyl)-3-[N-(3-chloropyridin-2-yl)-N-ethyl-amino]benzamide:

3-[N-(3-chloropyridin-2-yl)-N-ethyl]aminobenzoicacid (60 mg) obtained in the preceding step was subjected to the same reaction as in the step 4 of Example 1 to obtain the title compound (30 mg) in pale yellow solid.

### Example 4

### Preparation of N-(4-tert-butylphenyl)-3-[N-(3-chloropyridin-2-yl)-N-methyl]amino-4-methoxybenzamide:

### Step 1:

### Preparation of methyl 3-(3-chloropyridin-2-yl)amino-4-methoxybenzoate:

A similar reaction was performed using methyl 3-amino-4-methoxybenzoate (399 mg) and palladium acetate instead of tris (dibenzylideneacetone) dipalladium in the step 1 of Example 3 to obtain the title compound (273 mg).

### Step 2:

### Preparation of methyl 3-[N-(3-chloropyridin-2-yl)-N-methyl]amino-4-methoxybenzoate:

Methyl 3-(3-chloropyridin-2-yl)amino-4-methoxybenzoate (273 mg) obtained in the preceding step was used and the same reaction was performed using methyl iodide instead of ethyl iodide in the step 2 of Example 3 to obtain the title compound (260 mg) in white solid.

### Step 3:

### Preparation of N-(4-tert-butylphenyl)-3-[N-(3-chloropyridin-2-yl)-N-methyl]amino-4-methoxybenzamide:

Methyl 3-[N-(3-chloropyridin-2-yl)-N-methyl]amino-4-methoxybenzo ate (259 mg) obtained in the preceding step was used and a similar reaction as in the steps 3-4 of Example 3 was performed to obtain the title compound (297 mg) in white solid.

### Example 5

### Preparation of N-(4-tert-butylphenyl)-3-[N-(3-chloropyridin-2-yl)-N-methyl]amino-4-phenoxybenzamide:

The title compound (351 mg) was obtained by the same method as in Example 4 using methyl 3-amino-4-phenoxybenzoate (537 mg).

### Example 6

### Preparation of N-(4-tert-butylphenyl)-3-[N-(3-chloropyridin-2-yl)-N-methyl-amino]-2-methylbenzamide:

The title compound was obtained by the same method as in Example 4 using methyl 3-amino-2-methylbenzoate.

### Example 7

### Preparation of N-(4-tert-butylphenyl)-3-[N-(3-chloropyridin-2-yl)-N-methyl-amino]-4-methylbenzamide:

The title compound was obtained by the same method as in Example 4 using methyl 3-amino-4-methylbenzoate.

### Example 8

### Preparation of N-(4-tert-butylphenyl)-3-[N-(3-chloropyridin-2-yl)-N-methyl-amino]-4-phenylbenzamide:

The title compound was obtained by the same method as in Example 4 using methyl 3-amino-4-phenylbenzoate.

### Example 9

### Preparation of N-(4-tert-butylphenyl)-3-[N-(3-chloropyridin-2-yl)-N-methyl-amino]-4-fluorobenzamide:

The title compound was obtained by the same method as in Example 4 using methyl 3-amino-4-fluorobenzoate.

### Example 10

### Preparation of N-(4-tert-butylphenyl)-3-[N-(3-chloropyridin-2-yl)-N-ethyl]amino-4-methoxybenzamide:

Methyl 3-(3-chloropyridin-2-yl)amino-4-methoxybenzoate (293 mg) obtained by the method of the step 1 of Example 4 was used and the same reaction as in steps 2-4 of Example 3 was performed, and the title compound (331 mg) was obtained.

### Example 11

### Preparation of N-(4-tert-butylphenyl)-3-[N-(3-chloropyridin-2-yl)-N-isopropyl]amino-4-methoxybenzamide:

Methyl 3-(3-chloropyridin-2-yl)amino-4-methoxybenzoate (293 mg) obtained by the method of the step 1 of Example 4 was used and isopropyl iodide was used instead of ethyl iodide in the step 2 of Example 3, and the same reaction as in the subsequent steps 3-4 of Example 3 was performed to obtain the title compound (38 mg).

### Example 12

### Preparation of N-(4-tert-butylphenyl)-4-chloro-3-[N-(3-chloropyridin-2-yl)-N-methyl-amino]benzamide

The title compound (293 mg) was obtained by the same method as in Example 4 using methyl 3-amino-4-chlorobenzoate (611 mg).

### Example 13

### Preparation of N-(4-tert-butylphenyl)-4-chloro-3-[N-(pyridin-2-yl)-N-methyl-amino]benzamide:

Methyl 3-amino-4-chlorobenzoate (611 mg) was used and 2-bromopyridine was used instead of 2,3-dichloropyridine in the step 1 of Example 4, and the same reaction as in the subsequent steps 2-3 of Example 4 was performed to obtain the title compound (377 mg).

### Example 14

### Preparation of N-(4-tert-butylphenyl)-3-[N-(3-chloropyridin-2-yl)-N-methyl-amino]-4-methoxymethyloxybenzamide:

The title compound (1.76g) was obtained by the same method as in Example 4 using methyl 3-amino-4-methoxymethyloxybenzoate (5.25 g).

### Example 15

### Preparation of N-(4-tert-butylphenyl)-3-[N-(3-chloropyridin-2-yl)-N-methyl-amino]-4-hydroxybenzamide:

N-(4-tert-butylphenyl)-3-[N-(3-chloropyridin-2-yl)-N -methyl-amino]-4-methoxymethyloxybenzamide (1.48 g) obtained in Example 14 was dissolved in tetrahydrofuran (10 mL), methanol (10 mL), 6 M hydrochloric acid (10 mL) was added, and the mixture was stirred at 60?C for 2 hours. The reaction mixture was ice-cooled and weakly alkalified with a saturated sodium bicarbonate aqueous solution, and white solid obtained after stirring for 1 hour at room temperature was filtered and dried to obtain the title compound (1.24 g).

### Example 16

### Preparation of N-(4-tert-butylphenyl)-3-[N-(3-chloropyridin-2-yl)-N-methyl-amino]-4-iso propoxybenzamide:

N-(4-tert-butylphenyl)-3-[N-(3-chloropyridin-2-yl)-N -methyl-amino]-4-hydroxybenzamide (146 mg) obtained in Example 15 was dissolved in N,N-dimethylformamide (3 mL), potassium carbonate (74 mg) and isopropyl bromide (0.037 mL) were added, and the mixture was stirred at 80?C for 13 hours. The reaction mixture was allowed to cool, water was added, and the obtained white solid was filtered and purified by silica gel chromatography (hexane: tetrahydrofuran system) to obtain Example 16 compound (66 mg).

### Example 17

### Preparation of N-(4-tert-butylphenyl)-10-methyl-10H-benzo[b]pyrido[2,3-e][1,4] oxazine-8-carboxamide

When the method of the above Example was performed, Example 17 compound (20 mg) was obtained simultaneously with Example 16 compound (66 mg).

### Example 18

### Preparation of N-(4-tert-butylphenyl)-3-[N-(3-chloropyridin-2-yl)-N-methyl-amino]-4-(2-hydroxyethyl)oxybenzamide:

### Step 1:

### Preparation of N-(4-tert-butylphenyl)-3-[N-(3-chloropyridin-2-yl)-N-methyl-amino]-4-(2-acetoxyethyl)oxybenzamide:

N-(4-tert-butylphenyl)-3-[N-(3-chloropyridin-2-yl)-N -methyl-amino]-4-hydroxybenzamide (205 mg) obtained in Example 15 was dissolved in N,N-dimethylformamide (2 mL). Potassium carbonate (345 mg) and 2-bromoethyl acetate (0.417 mL) were added and the mixture was stirred at room temperature for 14 hours. The reaction mixture was partitioned between water-ethyl acetate, washed with a saturated brine, dried over anhydrous sodium sulfate and concentrated. The obtained residue was purified by silica gel chromatography (hexane: ethyl acetate = 1:1) to obtain amorphous white title compound (229 mg).

### Step 2:

### Preparation of N-(4-tert-butylphenyl)-3-[N-(3-chloropyridin-2-yl)-N-methyl-amino]-4-(2-hydroxyethyl)oxybenzamide:

N-(4-tert-butylphenyl)-3-[N-(3-chloropyridin-2-yl)-N -methyl-amino]-4-(2-acetoxyethyl)oxybenzamide (291 mg) obtained in the preceding step was dissolved in methanol (1.5 mL), tetrahydrofuran (1.5 mL) and water (1 mL), lithium hydroxide hydrate (42 mg) was added, and the mixture was stirred at room temperature for 1 hour. The reaction mixture was diluted with water and extracted with ethyl acetate. The ethyl acetate layer was washed with a saturated brine, washed with anhydrous sodium sulfate and concentrated to obtain the title compound (200 mg).

### Example 19

### Preparation of {4-(4-tert-butylphenyl)aminocarbonyl-2-[N-(3-chloropyridin-2-yl)-N-methyl]amino-phenoxy}acetic acid:

### Step 1:

### Preparation of N-(4-tert-butylphenyl)-3-[N-(3-chloropyridin-2-yl)-N-methyl-amino]-4-(methoxycarbonylmethyl)oxybenzamide:

N-(4-tert-butylphenyl)-3-[N-(3-chloropyridin-2-yl)-N -methyl-amino]-4-hydroxybenzamide (820 mg) obtained in Example 15 was dissolved in N,N-dimethylformamide (8 mL), potassium carbonate (1.38 g) and methyl bromoacetate (1.53 g) were added, and the mixture was stirred at room temperature for 18 hours. The reaction mixture was partitioned between water-ethyl acetate, washed with a saturated brine, dried over anhydrous sodium sulfate and concentrated. The residue was purified by silica gel chromatography (hexane: ethyl acetate = 1:1) to obtain oily white title compound (1.04 g).

### Step 2:

### Preparation of {4-(4-tert-butylphenyl)aminocarbonyl-2-[N-(3-chloropyridin-2-yl)-N-methyl]amino-phenoxy}acetic acid:

N-(4-tert-butylphenyl)-3-[N-(3-chloropyridin-2-yl) -N-methyl-amino]-4-(methoxycarbonylmethyl)oxybenzamide (1.04 g) obtained in the preceding step was dissolved in methanol (5 mL), tetrahydrofuran (mL) and water (3 mL), lithium hydroxide hydrate (210 mg) was added, and the mixture was stirred at room temperature for 1 hour. 1 M hydrochloric acid (5 mL) was added to the reaction mixture and the mixture was concentrated. The concentrate was diluted with water and extracted with ethyl acetate. The ethyl acetate layer was washed with a saturated brine, dried over anhydrous sodium sulfate and concentrated to obtain the title compound (816 mg).

### Example 20

### Preparation of N-(4-tert-butylphenyl)-4-carbamoylmethyloxy-3-[N-(3-chloropyridin-2-yl)-N-methyl-amino]benzamide:

{4-(4-tert-butylphenyl)aminocarbonyl-2-[N-(3-chlorop yridin-2-yl)-N-methyl]amino-phenoxy}acetic acid (200 mg)obtained in Example 19 was dissolved in N,N-dimethylformamide (2 mL), ammonium chloride (114 mg), 1-hydroxybenzotriazole (72 mg), 1-ethyl-3-(3-dimethylaminopropyl)-carbodiimide hydrochloride salt (90 mg) and triethylamine (0.357 mL) were added in this order, and the mixture was stirred at room temperature for 48 hours. Water was added to the reaction mixture and the mixture was extracted with ethyl acetate. The obtained ethyl acetate layer was sequentially washed with a saturated sodium bicarbonate aqueous solution, 5% citric acid aqueous solution and a saturated brine, dried over anhydrous sodium sulfate and concentrated. The obtained residue was purified by silica gel chromatography (chloroform: methanol = 9:1) and suspended and washed with hexane, white solid was filtered and dried to obtain the title compound (88 mg).

### Example 21

### Preparation of N-(4-tert-butylphenyl)-3-[N-(3-chloropyridin-2-yl)-N-methyl-amino]-4-(N-methylcarbamoylmethyl)oxybenzamide:

Methylamine hydrochloride salt was used instead of ammonium chloride in the same method as in Example 20 to obtain the title compound.

### Example 22

### Preparation of N-(4-tert-butylphenyl)-3-[N-(3-chloropyridin-2-yl)-N-methyl-amino]-4-(N,N-dimethylcarbamoylmethyl)oxybenzamide:

Dimethylamine hydrochloride salt was used instead of ammonium chloride in the same method as in Example 20 to obtain the title compound.

### Example 23

### Preparation of N-(4-tert-butylphenyl)-5-chloro-3-[N-(3-chloropyridin-2-yl)-N-methyl]aminobenzamide:

The title compound was obtained by the same method as in Example 4 using methyl 3-amino-5-chlorobenzoate.

### Example 24

### Preparation of N-(4-tert-butylphenyl)-2-chloro-5-[N-(3-chloropyridin-2-yl)-N-methyl]aminobenzamide:

The title compound was obtained by the same method as in Example 4 using methyl 5-amino-2-chlorobenzoate.

### Example 25

### Preparation of N-(4-tert-butylphenyl)-3-[N-(3-chloropyridin-2-yl)-N-methyl]amino-2-hydroxybenzamide:

### Step 1:

### Preparation of 2-methoxymethyloxy-3-nitrobenzoic acid:

3-Nitrosalicylic acid (2.5 g) was dissolved in N,N-dimethylformamide (25 mL), and potassium carbonate (3.5 g) was added. After stirred at room temperature for 30 minutes, the mixture was ice-cooled, methoxymethyl chloride (1.15 mL) was added, and the mixture was stirred overnight while allowed to return to room temperature. The reaction mixture was partitioned between water-ethyl acetate, and the obtained ethyl acetate layer was sequentially washed with water and a saturated brine, dried over anhydrous sodium sulfate and concentrated to obtain the title compound (2.64 g).

### Step 2:

### Preparation of methyl 3-amino-2-methoxymethyloxybenzoate:

The 2-methoxymethyloxy-3-nitrobenzoic acid (2.64 g) obtained in the preceding step was dissolved in tetrahydrofuran (20 mL) and ethyl acetate (10 mL), 5% palladium/activated carbon (0.25 g) was added, and the mixture was stirred at room temperature under hydrogen atmosphere for 2 hours. The reaction mixture was filtered, and the filtrate was concentrated to obtain the title compound (2.38 g).

### Step 3:

### Preparation of methyl 3-(3-chloropyridin-2-yl)amino-2-methoxymethyloxybenzoate:

Methyl 3-amino-2-methoxymethyloxy benzoate (2.38 g) obtained in the preceding step was suspended in toluene (20 mL), 2,3-dichloropyridine (1.62 g), palladium acetate (123 mg), 2,2'-bis(diphenylphosphino)-1,1'-binaphthyl (341 mg) and cesium carbonate (5.35 g) were added in this order, and the mixture was agitated at 80?C for 15 hours. The reaction mixture was allowed to cool, ethyl acetate-water was added, and after insoluble substances were filtered off, the mixture was partitioned, and the obtained ethyl acetate layer was washed with a saturated brine, dried over anhydrous sodium sulfate and concentrated. The obtained residue was purified by silica gel chromatography (hexane: ethyl acetate = 4:1) to obtain the title compound (3.46 g).

### Step 4:

### Preparation of 3-(3-chloropyridin-2-yl)amino-2-hydroxybenzoic acid:

Methyl 3-(3-chloropyridin-2-yl)amino-2-methoxymethyloxybenzoate. (3.46 g) obtained in the preceding step was dissolved in tetrahydrofuran (7.5 mL) and methanol (7.5 mL), 4 M sodium hydroxide (4 mL) was added, and the mixture was stirred at 60?C for 1 hour. The reaction mixture was allowed to cool, 1 M hydrochloric acid was added under stirring so that the pH was adjusted to 4, and the mixture was then concentrated. Water was added to the concentrate, the mixture was stirred at room temperature for 1 hour, and the obtained white solid was filtered and dried to obtain the title compound (1.31 g).

### Step 5:

### Preparation of N-(4-tert-butylphenyl)-3-[N-(3-chloropyridin-2-yl)-N-methyl]amino-2-hydroxybenzamide:

3-(3-Chloropyridin-2-yl)amino-2-hydroxybenzoic acid(1.31 g) obtained in the preceding step was subjected to the same reaction as in the step 4 of Example 1 to obtain the title compound (1.19 g) in white solid.

### Example 26

### Preparation of N-(4-tert-butylphenyl)-3-[N-(3-chloropyridin-2-yl)-N-methyl]amino-2-methoxybenzamide:

N-(4-tert-butylphenyl)-3-[N-(3-chloropyridin-2-yl)-N -methyl]amino-2-hydroxybenzamide (150 mg) obtained in Example 25 was dissolved in N,N-dimethylformamide (1.5 mL), potassium carbonate (101 mg) and methyl iodide (0.046 mL) were added, and the mixture was stirred at room temperature for 4 hours. The reaction mixture was partitioned between water-ethyl acetate, and the ethyl acetate layer was washed with a saturated brine, dried over anhydrous sodium sulfate and concentrated to obtain pale yellow resin-like title compound (151 mg).

### Example 27

### Preparation of N-(4-tert-butylphenyl)-3-[N-(3-chloropyridin-2-yl).-N-methyl-amino]-2-(2-hydroxyethyl)oxybenzamide:

The title compound (145 mg) was obtained by the same method as in Example 18 using N-(4-tert-butylphenyl)-3-[N-(3-chloropyridin-2-yl)-N-meth yl]amino-2-hydroxybenzamide (200 mg) obtained in Example 25.

### Example 28

### Preparation of N-(4-tert-butylphenyl)-3-[N-(3-chloropyridin-2-yl)-N-(2-hydroxyethyl)]amino-4-methoxybenzamide] :

Methyl 3-(3-chloropyridin-2-yl)amino-4-methoxybenzoate (200 mg) obtained in the method of the step 1 of Example 4 was used and the same reaction was performed using tert-butyldimethylsilylethyl bromide instead of ethyl iodide in the step 2 of Example 3, the same reaction as in the subsequent steps 3 - 4 of Example 3 was performed to obtain the title compound (162 mg).

### Example 29

### Preparation of N-(4-tert-butylphenyl)-4-chloro-3-[N-(3-chloropyridin-2-yl)-N-(2-methoxy ethyl)amino]benzamide:

### Step 1:

### Preparation of methyl 4-chloro-3-(3-chloropyridin-2-yl)aminobenzoate:

The title compound (5.5 g) was obtained using 3-amino-4-chlorobenzoic acid methyl (7 g) by the same method as in the step 1 of Example 4.

### Step 2:

### Preparation of N-(4-tert-butylphenyl)-4-chloro-3-[N-(3-chloropyridin-2-yl)-N-(2-methoxy ethyl)amino]benzamide:

Methyl4-chloro-3-(3-chloropyridin-2-yl)aminobenzoate (600 mg) obtained in the preceding step was used and the same reaction was performed using methoxyethyl bromide instead of ethyl iodide in the step 2 of Example 3, the same reaction as in the subsequent steps 3-4 of Example 3 was performed to obtain the title compound (250 mg).

### Example 30

### Preparation of N-(4-tert-butylphenyl)-4-chloro-3-[N-(3-chloropyridin-2-yl)-N-(2-hydroxyethyl)amino]benzamide:

Methyl4-chloro-3-(3-chloropyridin-2-yl)aminobenzoate obtained in the step 1 of Example 29 was used and the same reaction was performed using 2-bromoethyl acetate instead of ethyl iodide in the step 2 of Example 3, and the same reaction as in the subsequent steps 3-4 of Example 3 was performed to obtain the title compound.

### Example 31

### Preparation of 4-chloro-3-[N-(3-chloropyridin-2-yl)-N-(2-hydroxyethyl)amino]-N-(4-trifluoromethylphenyl)-benzamide hydrochloride salt:

### Step 1:

### Preparation of methyl 4-chloro-3-{N-(3-chloropyridin-2-yl)-N-[2-(tetrahydropyran-2-yl)oxyethyl]amino}benzoate:

Methyl4-chloro-3-(3-chloropyridin-2-yl)aminobenzoate (2.15 g) obtained by the method of the step 1 of Example 29 was dissolved in (30 mL), 60% sodium hydride (320 mg) and 2-bromoethyl-(tetrahydropyran-2-yl)ether were added in this order under ice-cooled stirring, and the mixture was stirred at 60?C for 6 hours. After the reaction mixture was diluted and neutralized with acetic acid, the mixture was extracted with ethyl acetate. The ethyl acetate layer was washed with a saturated brine, dried over anhydrous sodium sulfate and concentrated. The residue was purified by silica gel chromatography (hexane: ethyl acetate = 8:1) to obtain oily title compound (1.9 g).

### Step 2:

### Preparation of 4-chloro-3-{N-(3-chloropyridin-2-yl)-N-[2-(tetrahydropyran-2-yl)oxyethyl]amino}benzoic acid:

Methyl 4-chloro-3-{N-(3-chloropyridin-2-yl)-N-[2-(tetrahydropyra n-2-yl)oxyethyl]amino}benzoate (1.9 g) obtained in the preceding step was dissolved in methanol (20 mL), 4 M sodium hydroxide (1.5 mL) was added, and the mixture was stirred at 60?C for 1 hour. 4 M sodium hydroxide (1.5 mL) was then added, and the mixture was stirred at the same temperature further for 1 hour. After the reaction mixture was concentrated and the concentrate was diluted with water, the mixture was adjusted to pH 5 with hydrochloric acid and extracted with ethyl acetate. The ethyl acetate layer was washed with a saturated brine, dried over anhydrous sodium sulfate and concentrated. Hexane was added to the residue and precipitated white solid was filtered and dried to obtain the title compound (1.48 g).

### Step 3:

### Preparation of 4-chloro-3-{N-(3-chloropyridin-2-yl)-N-[2-(tetrahydropyran-2-yl)oxyethyl]amino}-N-(4-trifluoromethylphenyl)-benzamide:

4-chloro-3-{N-(3-chloropyridin-2-yl)-N-[2-(tetrahydr opyran-2-yl)oxyethyl]amino}benzoicacid (250 mg) obtained in the preceding step and 4-trifluoromethyl aniline were subjected to the same condensation reaction as in the step 4 of Example 1 to obtain the title compound (120 mg).

### Step 4:

### Preparation of 4-chloro-3-[N-(3-chloropyridin-2-yl)-N-(2-hydroxyethyl)amino]-N-(4-trifluoromethylphenyl)benzamide hydrochloride salt:

4-chloro-3-{N-(3-chloropyridin-2-yl)-N-[2-(tetrahydr opyran-2-yl)oxyethyl]amino}-N-(4-trifluoromethylphenyl)-b enzamide (120 mg) obtained in the preceding step was dissolved in methanol (2 mL), 6 M hydrochloric acid (1 mL) was added, and the mixture was stirred at room temperature for 1 hour. The reaction mixture was partitioned between a saturated sodium bicarbonate aqueous solution and ethyl acetate, and the obtained ethyl acetate layer was washed with a saturated brine, dried over anhydrous sodium sulfate and concentrated. The obtained residue was purified by silica gel chromatography (hexane: ethyl acetate = 3:1) and, after concentration, white solid precipitated by the addition of 4 N-HCl/ethyl acetate was separated by filtration and dried to obtain the title compound (45 mg).

### Example 32

### Preparation of 4-chloro-3-[N-(3-chloropyridin-2-yl)-N-(2-hydroxyethyl)amino]-N-(4-trifluoromethoxyphenyl)benzamide hydrochloride salt:

4-chloro-3-{N-(3-chloropyridin-2-yl)-N-[2-(tetrahydr opyran-2-yl)oxyethyl]amino}benzoic acid (200 mg) obtained at the step 2 of Example 31 and 4-trifluoromethoxy aniline were used and the same reaction as in the subsequent steps 3-4 of Example 31 was performed to obtain the title compound (30 mg).

### Example 33

### Preparation of N-(4-tert-butylphenyl)-3-[N-(2-chlorophenyl)-N-methyl-amino]benzamide:

### Step 1:

### Preparation of ethyl 3-(2-chlorophenyl)aminobenzoate: 2-bromo-1-chlorobenzene (383 mg) and ethyl

3-aminobenzoate (661 mg) were dissolved in toluene (6 mL), tris(dibenzylideneacetone) dipalladium (18 mg), 2,2'-bis(diphenylphosphino)-1,1'-binaphthyl (12 mg), potassium tert-butoxide (450 mg) were added in this order, and the mixture was refluxed under stirring for 16 hours. The reaction mixture was diluted with diethyl ether after allowed to cool, insoluble substances was filtered off, and the filtratewasconcentrated. The residue was purified by silica gel chromatography (hexane: ethyl acetate = 5:1) to obtain colorless oily title compound (156 mg).

### Step 2:

### Preparation of ethyl 3-[N-(2-chlorophenyl)-N-methyl]aminobenzoate:

Ethyl 3-(2-chlorophenyl)aminobenzoate (156mg) obtained in the preceding step was dissolved in N,N-dimethylformamide (2 mL), 60% sodium hydride (10 mg) was added, the mixture was stirred at room temperature until foaming was stopped, then ethyl iodide (0.028 mL) was added, and the mixture was stirred for 1 hour. The reaction mixture was partitioned between water and ethyl acetate, and the obtained ethyl acetate layer was sequentially washed with water and a saturated brine, dried over anhydrous magnesium sulfate and concentrated to obtain yellow oily title compound (171 mg).

### Step 3:

### Preparation of N-(4-tert-butylphenyl)-3-[N-(2-chlorophenyl)-N-methyl-amino]benzamide:

Ethyl 3-[N-(2-chlorophenyl)-N-methyl]aminobenzoate (170 mg) obtained in the preceding step was used and the same reaction as in the subsequent steps 3-4 of Example 3 was performed to obtain 26 mg of title compound in white solid.

### Example 34

### Preparation of N-(4-tert-butylphenyl)-4-(3-chloropyridin-2-yl)-3,4-dihydro-2H-benzo[1,4]oxazine-6-carboxamide:

### Step 1:

### Preparation of methyl 4-hydroxy-3-nitrobenzoate:

4-Hydroxy-3-nitrobenzoic acid (10 g) was dissolved in methanol (100 mL), concentrated sulfuric acid (1.5 mL) was added, and the mixture was refluxed under stirring for 5 hours. The reaction mixture was concentrated and partitioned between water and ethyl acetate, and the ethyl acetate layer was washed with a saturated brine, dried over anhydrous magnesium sulfate and concentrated to obtain the title compound (7.6 g).

### Step 2:

### Preparation of methyl 4-ethoxycarbonylmethyloxy-3-nitrobenzoate:

Methyl 4-hydroxy-3-nitrobenzoate (7.6 g) obtained in the preceding step was dissolved in N,N-dimethylformamide (100 mL), potassium carbonate (5.4 g) and bromoethyl acetate (4.3 mL) were added, and the mixture was stirred at 110?C for 1.5 hours. The reaction mixture was concentrated and partitioned between water and ethyl acetate. The ethyl acetate layer was washed with water, dried over anhydrous magnesium sulfate and concentrated. Hexane was added to the concentrated residue and precipitated solid was separated by filtration and dried to obtain the title compound (10.14 g).

### Step 3:

### Preparation of methyl 3-oxo-3,4-dihydro-2H-benzo[1,4]oxazine-6-carboxylate:

Methyl 4-ethoxycarbonylmethyloxy-3-nitrobenzoate (10 g) obtained in the preceding step was dissolved in methanol (50 mL) and tetrahydrofuran (50 mL), 5% palladium carbon (2 g) was added, and the mixture was stirred at room temperature under hydrogen atmosphere . The reaction mixture was filtered and the filtrate was concentrated and purified by silica gel chromatography (hexane: ethyl acetate system) to obtain the title compound (4 g).

### Step 4:

### Preparation of methyl 3,4-dihydro-2H-benzo[1,4]oxazine-6-carboxylate:

Methyl 3-oxo-3,4-dihydro-2H-benzo[1,4]oxazine-6-carboxylate (3 g) obtained in the preceding step was dissolved in tetrahydrofuran (50 mL), and 1 M borane / tetrahydrofuran solution (17.4 mL) was added, and the mixture was refluxed under stirring for 1.5 hours. The reaction mixture was ice-cooled, 6 M hydrochloric acid was added under stirring until pH was adjusted to 2, stirring was then continued for 1 hour. The reaction mixture was partitioned by ethyl acetate and sodium bicarbonate aqueous solution, and the ethyl acetate layer was washed with a saturated brine, dried over anhydrous magnesium sulfate and concentrated. The residue was purified by silica gel chromatography (hexane: ethyl acetate = 3:1) to obtain the title compound (2 g).

### Step 5:

### Preparation of methyl 4-(3-chloropyridin-2-yl)-3,4-dihydro-2H-benzo[1,4]oxazine-6-carboxylate:

Methyl 3,4-dihydro-2H-benzo[1,4]oxazine-6-carboxylate (1 g) obtained in the preceding step was dissolves in toluene (10 mL). 2,3-dichloropyridine (1.5 g), tris(dibenzylideneacetone) dipalladium (356 mg), 2,2'-bis(diphenylphosphino)-1,1'-binaphthyl (242 mg) and cesium carbonate (1.42.53 g) were added in this order, and the mixture was heated at 80?C under stirring overnight. After the reaction mixture was allowed to cool, ethyl acetate and water were added, insoluble substances were filtered off, and the obtained ethyl acetate layer was washed with a saturated brine, dried over anhydrous magnesium sulfate and concentrated. The obtained residue was purified by silica gel chromatography (hexane: tetrahydrofuran = 3:1) to obtain the oily title compound (250 mg).

### Step 6:

### Preparation of 4-(3-chloropyridin-2-yl)-3,4-dihydro-2H-benzo[1,4]oxazine-6-carboxylic acid:

Methyl 4-(3-chloropyridin-2-yl)-3,4-dihydro-2H-benzo[1,4]oxazine -6-carboxylate (240 mg) obtained in the preceding step was dissolved in methanol (2.5 mL), 4 M lithium hydroxide aqueous solution (0.3 mL) was added, and the mixture was heated under stirring at 60?C for 1.5 hours. The mixture was allowed to cool and neutralized with 1 M hydrochloric acid (4 mL), water (20 mL) was added, and the precipitated solid was filtered and dried to obtain the title compound (180 mg).

### Step 7:

### Preparation of N-(4-tert-butylphenyl)-4-(3-chloropyridin-2-yl)-3,4-dihydro-2H-benzo[1,4]oxazine-6-carboxamide:

4-(3-Chloropyridin-2-yl)-3,4-dihydro-2H-benzo[1,4]ox azine-6-carboxylic acid (170 mg) obtained in the preceding step was dissolved in N,N-dimethylformamide (1.7 mL), 4-tert-butylaniline (0.094 mL), 1-hydroxybenzotriazole (90 mg) and 1-ethyl-3-(3-dimethylaminopropyl)carbodiimide hydrochloride salt (112 mg) were added in this order, and the mixture was stirred at room temperature overnight. After water and a saturated sodium bicarbonate aqueous solution were added to the reaction mixture, the mixture was extracted with ethyl acetate. The obtained ethyl acetate layer was washed with a saturated brine, dried over anhydrous magnesium sulfate and concentrated. Theobtainedresiduewaspurifiedbysilica gel chromatography (hexane: ethyl acetate system), diisopropyl ether was added, and the precipitated solid was dried to obtain the title compound (45 mg).

### Example 35

### Preparation of 4-chloro-3-[N-(3-chloropyridin-2-yl)-N-(2-hydroxyethyl)amino]-N-(4-isobutyloxyphenyl)benzamide hydrochloride salt:

4-Chloro-3-{N-(3-chloropyridin-2-yl)-N-[2-(tetrahydr opyran-2-yl)oxy ethyl]amino}benzoic acid (150 mg) obtained in the step 2 of Example 31 and 4-isobutyloxyaniline were used and the same reaction as in the subsequent steps 3-4 of Example 31 was performed to obtain the title compound (40 mg).

### Example 36

### Preparation of 4-chloro-N-(4-chlorophenyl)-3-[N-(3-chloropyridin-2-yl)-N-(2-hydroxyethyl)amino]-benzamide:

4-Chloro-3-{N-(3-chloropyridin-2-yl)-N-[2-(tetrahydr opyran-2-yl)oxyethyl]amino}benzoicacid (200 mg) obtained in the step 2 of Example 31 and 4-chloroaniline were used and the same reaction as in the subsequent steps 3-4 of Example 31 was performed to obtain the title compound (205 mg).

### Example 37

### Preparation of 4-chloro-3-[N-(3-chloropyridin-2-yl)-N-(2-hydroxyethyl)amino]-N-(4-isopropylphenyl)-benzamide:

4-Chloro-3-{N-(3-chloropyridin-2-yl)-N-[2-(tetrahydr opyran-2-yl)oxyethyl]amino}benzole acid (210 mg) obtained in the step 2 of Example 31 and 4-isopropylaniline were used and the same reaction as in the subsequent steps 3-4 of Example 31 was performed to obtain the title compound (110 mg).

### Example 38

### Preparation of 4-chloro-3-[N-(3-chloropyridin-2-yl)-N-(2-hydroxyethyl)amino]-N-(1-methyl-1,2,3,4-tetrahydroisoquinolin-7-yl)benzamide:

4-Chloro-3-{N-(3-chloropyridin-2-yl)-N-[2-(tetrahydr opyran-2-yl)oxyethyl]amino}benzoic acid (200 mg) obtained in the step 2 of Example 31 and 1-methyl-1,2,3,4-tetrahydroisoquinolin-7-ylamine were used and the same reaction as in the subsequent steps 3-4 of Example 31 was performed to obtain the title compound (89 mg).

### Example 39

### Preparation of 4-chloro-3-[N-(3-chloropyridin-2-yl)-N-(2-hydroxyethyl)amino]-N-(4-chloro-3-trifluoromethylphenyl)benzamide :

4-Chloro-3-{N-(3-c,hloropyridin-2-yl)-N-[2-(tetrahydr opyran-2-yl)oxyethyl]amino}benzoic acid (200 mg) obtained in the step 2 of Example 31 and 4-chloro-3-trifluoromethylaniline were used and the same reaction as in the subsequent steps 3-4 of Example 31 was performed to obtain the title compound (175 mg).

### Example 40

### Preparation of 4-chloro-3-[N-(3-chloropyridin-2-yl)-N-(2-hydroxyethyl)amino]-N-(2-trifluoromethylpyridine-5-yl)benzamide:

4-Chloro-3-{N-(3-chloropyridin-2-yl)-N-[2-(tetrahydr opyran-2-yl)oxyethyl]amino}benzoicacid (200 mg) obtained in the step 2 of Example 31 and 3-fluoro-4-piperidinoaniline were used and the same reaction as in the subsequent steps 3-4 of Example 31 was performed to obtain the title compound (105 mg).

### Example 41

### Preparation of 4-chloro-3-[N-(3-chloropyridin-2-yl)N-(2-hydroxyethyl)amino]-N-(4-isopropyloxyphenyl)benzamide:

4-Chloro-3-{N-(3-chloropyridin-2-yl)-N-[2-(tetrahydr opyran-2-yl)oxyethyl]amino}benzoic acid (200 mg) obtained in the step 2 of Example 31 and 4-isopropyloxyaniline were used and the same reaction as in the subsequent steps 3-4 of Example 31 was performed to obtain the title compound (13 mg).

### Example 42

### Preparation of 4-chloro-3-[N-(3-chloropyridin-2-yl)-N-(2-hydroxyethyl)amino]-N-(5-trifluoromethylpyridin-2-yl)-benzamide:

### Step 1:

### Preparation of 4-chloro-3-{N-(3-chloropyridin-2-yl)-N-[2-(tetrahydropyran-2-yl)oxyethyl]amino}-N-(2-trifluoromethylpyridin-5-yl)-benzamide:

4-Chloro-3-{N-(3-chloropyridin-2-yl)-N-[2-(tetrahydr opyran-2-yl)oxyethyl]amino}benzoic acid (200 mg) obtained in the step 2 of Example 31 was dissolved in pyridine (2 mL), thionyl chloride (0.04 mL) was added, and the mixture was stirred at room temperature for 1 hour. Subsequently, 2-trifluoromethylpyridin-5=yl amine (79 mg) was added, and the mixture was stirred for 2 hours. The reaction mixture was diluted with ethyl acetate, sequentially washed with potassium bisulfate aqueous solution, sodium hydroxide aqueous solution, and a saturated brine, dried over anhydrous magnesium sulfate and concentrated. The residue was purified by silica gel chromatography (hexane: ethyl acetate = 1:1) to obtain the title compound (120 mg).

### Step 2:

### Preparation of 4-chloro-3-[N-(3-chloropyridin-2-yl)-N-(2-hydroxyethyl)amino]-N-(2-trifluoromethylpyridin-5-yl)benzamide :

4-Chloro-3-{N-(3-chloropyridin-2-yl)-N-[2-(tetrahydr opyran-2-yl)oxyethyl]amino}-N-(2-trifluoromethylpyridin-5 -yl)-benzamide (120 mg) obtained in the preceding step was dissolved in tetrahydrofuran (2 mL), 6 M hydrochloric acid was added, and the mixture was stirred overnight at room temperature. The reaction mixture was neutralized with 2M sodium hydroxide and extracted with ethyl acetate. The ethyl acetate layer was sequentially washed with water and a saturated brine, dried over anhydrous magnesium sulfate and concentrated. The obtained solid was purified by silica gel chromatography (hexane: ethyl acetate = 3:2) to obtain the title compound (5.8 mg).

### Example 43

### Preparation of 4-chloro-3-[N-(3-chloropyridin-2-yl)-N-(2-hydroxyethyl)amino]-N-(3-fluoro-4-piperidinylphenyl)-benzamide:

5-Trifluoromethylpyridin-2-ylamine (53 mg) was used instead of 2-trifluoromethylpyridin-5ylamine in the step 1 of Example 42 to obtain the title compound (79 mg) by the same subsequent method.

### Example 44

### Preparation of 4-chloro-3-[N-(3-chloropyridin-2-yl)-N-(2-hydroxyethyl)amino]-N-(4-isobutyloxyphenyl)-benzamide:

4-Chloro-3-{N-(3-chloropyridin-2-yl)-N-[2-(tetrahydropyran-2-yl)oxyethyl]amino}benzoicacid (180 mg) obtained in the step 2 of Example 31 and 4-isobutyloxyaniline were used and the same reaction as in the subsequent steps 3-4 of Example 31 was performed to obtain the title compound (31 mg).

### Example 45

### Preparation of 4-chloro-3-[N-(3-chloropyridin-2-yl)-N-(3-hydroxypropyl)amino]-N-(4-tert-butylphenyl)-benzamide;

### Step 1:

### Preparation of 4-chloro-3-{N-(3-chloropyridin-2-yl)-N-[3-(tetrahydropyran-2-yl)oxy propyl]amino}benzoic acid:

4-Chloro-3-(3-chloropyridin-2-yl)aminobenzoate (6.5 g) was used in the same method as in the step 1 of Example 31, and 3-bromopropyl-(tetrahydropyran-2-yl)ether instead of 2-bromoethyl-(tetrahydropyran-2-yl)ether was used, and the same reaction was performed followed by the same reaction as in the step 2 of Example 31 to obtain the title compound (4.7 g).

### Step 2:

### Preparation of 4-chloro-3-[N-(3-chloropyridin-2-yl)-N-(3-hydroxypropyl)amino]-N-(4-tert-butylphenyl)-benzamide:

4-Chloro-3-{N-(3-chloropyridin-2-yl)-N-[3-(tetrahydr opyran-2-yl)oxy propyl]amino}benzoic acid (280 mg) obtained in the preceding step and tert-butylaniline were used, and subjected to the same reaction as in the steps 3-4 of Example 31 to obtain the title compound (245 mg).

### Example 46

### Preparation of N-(3-chloropyridin-2-yl)-N-{2-chloro-5-[N-(4-tert-butylphenyl)carbamoyl]phenyl}-aminoacetic acid:

### Step 1:

### Preparation of tert-butyl 4-chloro-3-nitrobenzoate:

4-chloro-3-nitrobenzoic acid (5.0 g) was suspended in chloroform (50 mL), oxalyl chloride (3.24 mL), N,N-dimethylformamide (one drop) was added, and the mixture was stirred at room temperature for 2 hours. The reaction mixture was concentrated, the tetrahydrofuran (50mL) was added, a tetrahydrofuran (30 mL) solution of potassium tert-butoxide (4.17 g) was added under ice-cooling, and the mixture was stirred at room temperature for 1.5 hours. A saturated sodium bicarbonate aqueous solution was added to the reaction mixture and the mixture was extracted with ethyl acetate. The ethyl acetate layer was washed with a saturated brine, dried over anhydrous magnesium sulfate and concentrated to obtain the title compound (6.35 g).

### Step 2:

### Preparation of tert-butyl 3-amino-4-chlorobenzoate:

tert-Butyl 4-chloro-3-nitrobenzoate (6.35 g) obtained in the preceding step was dissolved in methanol (65 mL), activated carbon (3 g), iron(III) chloride hexahydrate (666 mg) and subsequently hydrazine hydrate (5.97 mL) were added, and the mixture was refluxed under stirring for 1 hour. The reaction mixture was filtered and the filtrate was concentrated. The concentrate was partitioned between ethyl acetate and water, and the ethyl acetate layer was sequentially washed with water and a saturated brine, and then dried over anhydrous magnesium sulfate and concentrated. The residue was purified by silica gel chromatography (hexane: ethyl acetate = 8:1) to obtain the title compound (4.14 g) in white solid.

### Step 3:

### Preparation of tert-butyl 4-chloro-3-(3-chloropyridin-2-yl)aminobenzoate:

tert-Butyl 3-amino-4-chlorobenzoate (1.48 g) obtained in the preceding step was subjected to the same reaction as in the step 1 of Example 3 to obtain the title compound (3.13 g) in white solid.

### Step 4:

### Preparation of tert-butyl 4-chloro-3-[N-(3-chloropyridin-2-yl)-N-ethoxycarbonylmethyl]aminobenzoate:

tert-Butyl 4-chloro-3-(3-chloropyridin-2-yl)aminobenzoate (1.62 g) obtained in the preceding step was dissolved in N,N-dimethylformamide (15 mL), sodium hydride (229 mg) and bromoethyl acetate (0.635 mL) were added in this order, and the mixture was stirred at 60?C for 2 hours . Water was added to the reaction mixture, extracted with ethyl acetate, and the obtained ethyl acetate layer was sequentially washed with water and a saturated brine, dried over anhydrous magnesium sulfate and concentrated. The residue was purified by silica gel chromatography (hexane: ethyl acetate = 8:1) to obtain the title compound (1.55 g) in white solid.

### Step 5:

### Preparation of 4-chloro-3-[N-(3-chloropyridin-2-yl)-N-ethoxycarbonylmethyl]aminobenzoic acid:

Tert-butyl 4-chloro-3-[N-(3-chloropyridin-2-yl)-N-ethoxycarbonylmeth yl] aminobenzoate (500 mg) obtained in the preceding step was dissolved in chloroform (1 mL), trifluoroacetic acid (1 mL) was added, and the mixture was stirred at room temperature for 6 hours. The reaction mixture was concentrated, and the residue containing the title compound was subjected to a subsequent step without purification.

### Step 6:

### Preparation of 4-chloro-3-[N-(3-chloropyridin-2-yl)-N-(ethoxycarbonylmethyl)amino]-N-(4-tert-butylphenyl)benzamide:

The residue containing 4-chloro-3-[N-(3-chloropyridin-2-yl)-N-ethoxycarbonylmeth yl]aminobenzoic acid obtained in the preceding step was dissolved in N,N-dimethylformamide (5 mL), 4-tert-butylaniline (192 mg), 1-hydroxybenzotriazole (216 mg) and 1-ethyl-3-(3-dimethylaminopropyl)-carbodiimide hydrochloride salt (270 mg) were added in this order, and the mixture was stirred for two days at room temperature. Water was added to reaction mixture, the mixture was extracted with ethyl acetate, and this was sequentially washed with a saturated sodium bicarbonate aqueous solution and a saturated brine, dried over anhydrous magnesium sulfate and concentrated. The obtained residue was purified by silica gel chromatography (hexane: ethyl acetate = 7:1) to obtain amorphous white title compound (408 mg).

### Step 7:

### Preparation of N-(3-chloropyridin-2-yl)-N-{2-chloro-5-[N-(4-tert-butylphenyl)carbamoyl]phenyl}aminoacetic acid:

4-Chloro-3-[N-(3-chloropyridin-2-yl)-N-(ethoxycarbon ylmethyl)amino]-N-(4-tert-butylphenyl)-benzamide (408 mg) obtained in the preceding step was dissolved in tetrahydrofuran (2 mL) and ethanol (2 mL), 1 M sodium hydroxide (1.5 mL) was added, and the mixture was stirred at room temperature for 3 hours. The reaction mixture was concentrated after neutralization with 1 M hydrochloric acid. Water was added to the residue, the mixture was stirred, and the precipitated white solid was dried to obtain the title compound (372 mg) .

### Example 47

### Preparation of 4-chloro-3-[N-(3-chloropyridin-2-yl)-N-(carbamoylmethyl)amino]-N-(4-tert-butylphenyl)-benzamide:

N-(3-chloropyridin-2-yl)-N-{2-chloro-5-[N-(4-tert-butylphenyl)carbamoyl]phenyl}aminoacetic acid (100 mg) obtained in Example 46 was dissolved in N,N-dimethylformamide (2 mL), ammonium chloride (57 mg), triethylamine (0.177 mL), 1-hydroxybenzotriazole (39 mg) and 1-ethyl-3-(3-dimethylaminopropyl)-carbodiimide hydrochloride salt (49 mg) were added in this order, and the mixture was stirred at room temperature for 24 hours. Water was added to reaction mixture, the mixture was extracted with ethyl acetate, and this was sequentially washed with a saturated sodium bicarbonate aqueous solution and a saturated brine, dried over anhydrous magnesium sulfate and concentrated. The obtained residue was purified by silica gel chromatography (chloroform: methanol = 20:1) to obtain the title compound (56 mg) in light yellow solid.

### Example 48

### Preparation of 4-chloro-3-[N-(3-chloropyridin-2-yl)-N-(N-methylcarbamoylmethyl)amino]-N-(4-tert-butylphenyl)benzamide:

N-(3-chloropyridin-2-yl)-N-{2-chloro-5-[N-(4-tert-bu tylphenyl)carbamoyl]phenyl}aminoacetic acid (100 mg) obtained in Example 46 was dissolved in N,N-dimethylformamide (2 mL), 1-hydroxybenzotriazole (39 mg) and 1-ethyl-3-(3-dimethylaminopropyl)-carbodiimide hydrochloride salt (49 mg) were added in this order, and the mixture was stirred at room temperature for 2 hours. After the reaction mixture was ice-cooled and 40% methylamine solution (0.038 mL) was added, the mixture was stirred at room temperature for 2 hours. Water was added to the reaction mixture, the mixture was extracted with ethyl acetate, and this was sequentially washed with a saturated sodium bicarbonate aqueous solution and a saturated brine, dried over anhydrous magnesium sulfate and concentrated. The obtained residue was purified by silica gel chromatography (chloroform: methanol = 20:1) to obtain the title compound (26 mg) in light yellow solid.

### Example 49

### Preparation of 4-chloro-3-[N-(3-chloropyridin-2-yl)-N-(N,N-dimethylcarbamoylmethyl)amino]-N(4-tert-butylphenyl)benzamide:

N-(3-Chloropyridin-2-yl)-N-{2-chloro-5-[N-(4-tert-bu tylphenyl)carbamoyl]phenyl}aminoacetic acid (150 mg) obtained in Example 46 was dissolved in N, N-dimethylformamide (3 mL), 1-hydroxybenzotriazole (58 mg) and 1-ethyl-3-(3-dimethylaminopropyl)carbodiimide hydrochloride salt (73 mg) were added in this order, and the mixture was stirred at room temperature for 30 minutes. After the reaction mixture was ice-cooled and 50% dimethylamine solution (0.066 mL) was added, the mixture was stirred at room temperature for 1 hour. Water was added to the reaction mixture, the mixture was extracted with ethyl acetate, and this was sequentially washed with a saturated sodium bicarbonate aqueous solution and a saturated brine, dried over anhydrous magnesium sulfate and concentrated. The obtained residue was purified by silica gel chromatography (hexane: ethyl acetate = 3:2) to obtain the title compound (136 mg) in light yellow solid.

### Examples 50-56

Compounds of Examples 50-56 shown in the following table were obtained in the same method as described in the above-mentioned general methods A to C and/or the methods described in the above-mentioned Examples 28-32.

### Examples 57-72

Compounds of Examples 57-72 shown in the following table were obtained in the same method as described in the above-mentioned general methods A to C and/or the methods described in the above-mentioned Examples 46-49.

### Example 73

### Preparation of 6-{[5-(4-tert-butylphenylcarbamoyl)-2-chlorophenyl]-methyl-amino}-5-chloronicotinic acid ethyl

### Step 1:

### Preparation of ethyl 6-[(5-tert-butoxycarbonyl-2-chlorophenyl)amino]-5-chloronicotinate:

Ethyl 5,6-dichloronicotinate (1.64 g), 3-amino-4-chlorobenzoic acid tert-butyl ester (1.70 g) was dissolved in toluene (15 mL), palladium acetate (84 mg), 2,2'-bis(diphenylphosphino)-1,1'-binaphthyl (232 mg), and cesium carbonate (3.65 g) were added in this order, and the mixture was stirred at 80?C for 20 hours. After reaction mixture was allowed to cool, ethyl acetate-water was added. Insoluble substances were filtered off and the reaction mixture was partitioned, and the obtained ethyl acetate layer was washed with a saturated brine, dried over anhydrous magnesium sulfate and concentrated. The obtained residue was purified by silica gel chromatography (hexane: ethyl acetate system) to obtain the title compound (2.37 g).

### Step 2:

### Preparation of ethyl 6-[(5-tert-butoxycarbonyl-2-chlorophenyl)-methyl-amino]-5-chloronicotinate:

Ethyl 6-[(5-tert-butoxycarbonyl-2-chlorophenyl)amino]-5-chloron icotinate (1.37 g) obtained in the preceding step was dissolved in N,N-dimethylformamide (15 mL), sodium hydride (60%) (160 mg) was added while ice-cooled, the mixture was stirred at room temperature for 1 hour, methyl iodide (0.311 mL) was added, and the mixture was stirred for 1 hour. A saturated sodium bicarbonate aqueous solution was added to the reaction mixture, and the mixture was extracted with ethyl acetate. The ethyl acetate layer was washed with a saturated brine, dried over anhydrous sodium sulfate and concentrated. The obtained residue was purified by silica gel chromatography to obtain the title compound (1.17 g).

### Step 3:

### Preparation of 4-chloro-3-(3-chloro-5-ethoxycarbonylpyridin-2-yl-)-methyl-aminobenzoic acid

Ethyl 6-[(5-tert-butoxycarbonyl-2-chlorophenyl)amino]-5-chloron icotinate (1.17 g) obtained in the preceding step was dissolved in chloroform (5 mL), trifluoroacetic acid (5 mL) was added and the mixture was stirred at room temperature for 5 hours. The reaction mixture was concentrated, water was added to the residue, the solid obtained by neutralization with a saturated sodium bicarbonate aqueous solution was separated by filtration and dried to obtain the title compound (965 mg) .

### Step 4:

### Preparation of ethyl 6-{[5-(4-tert-butylphenylcarbamoyl)-2-chlorophenyl]-methyl-1-amino}-5-chloronicotinate

4-chloro-3-(3-chloro-5-ethoxycarbonylpyridin-2-yl)-m ethyl-aminobenzoic acid (965 mg) obtained in the preceding step was dissolved in N,N-dimethylformamide (10 mL). 4-tert-butylaniline (423 mg), 1-hydroxybenzotriazole (476 mg) and 1-ethyl-3-(3-dimethylaminopropyl)-carbodiimide hydrochloride salt (595 mg) were added in this order, and the mixture was stirred at room temperature for 4 hours. Water was added to the reaction mixture and the mixture was extracted with ethyl acetate. The ethyl acetate layer was washed with a saturated sodium bicarbonate aqueous solution and a saturated saline solution, dried over anhydrous sodium sulfate and concentrated to obtain the title compound (1.33 g).

### Example 74

### Preparation of 6-{[5-(4-tert-butylphenylcarbamoyl)-2-chlorophenyl]-methyl-amino}-5-chloronicotinic acid

Ethyl 6-{[5-(4-tert-butylphenylcarbamoyl)-2-chlorophenyl]-methy 1-amino}-5-chloronicotinate (1.30 g) obtained in Example 73 was dissolved in tetrahydrofuran (5 mL) and methanol (5 mL), 4N sodium hydroxide (2 mL) was added, and the mixture was stirred at room temperature for 1.5 hours. The reaction mixture was concentrated after neutralization with 1N hydrochloride. Water was added to the residue and the precipitated solid was separated by filtration and dried. The obtained solid was dissolved in ethyl acetate and the mixture was concentrated after insoluble substances were filtered off. Diethyl ether was added to the residue to make a suspension, and the solid was separated by filtration and dried to obtain the title compound (1.03 g).

### Example 75

### Preparation of 6-{[5-(4-tert-butylphenylcarbamoyl)-2-chlorophenyl]-methyl-amino}-5-chloronicotinamide

6-{[5-(4-tert-butylphenylcarbamoyl)-2-chlorophenyl]-methyl-amino}-5-chloronicotinic acid (200 mg) obtained in Example 74 was dissolved in chloroform (5 mL), oxalyl chloride (0.055 mL), N,N-dimethylformamide (one drop) were added, and the mixture was stirred at room temperature for 1 hour. The reaction mixture was concentrated and dissolved in tetrahydrofuran (5 mL), 28% ammonia aqueous solution (1 mL) was then added, and the mixture was stirred at room temperature for 1 hour. The reaction mixture was partitioned between water and ethyl acetate, and the ethyl acetate layer was washed with a saturated brine, then dried over anhydrous sodium sulfate and concentrated. The residue was purified by silica gel chromatography to obtain the title compound (46 mg).

### Example 76

### Preparation of 6-{[5-(4-tert-butylphenylcarbamoyl)-2-chlorophenyl]-methyl-amino}-5-chloro-N-nicotinamide

The title compound (106 mg) was obtained from 6-{[5-(4-tert-butylphenylcarbamoyl)-2-chlorophenyl]-methy 1-amino}-5-chloronicotinic acid (200 mg) obtained in Example 74 in the same method as in Example 75.

### Example 77

### Preparation of 6-{[5-(4-tert-butylphenylcarbamoyl)-2-chlorophenyl]-methyl-amino}-5-chloro-N,N-dimethyl nicotinamide

The title compound (143 mg) was obtained from 6-{[5-(4-tert-butylphenylcarbamoyl)-2-chlorophenyl]-methy 1-amino}-5-chloronicotinic acid (200 mg) obtained in Example 74 in the same method as in Example 75.

### Example 78 to Example 83

Compounds of Example 78 to Example 83 shown in the following tables were obtained by the same method as described in the above-mentioned general methods A to C and/or the methods described in the above-mentioned Examples 1-28.

### Test Example

The assay for evaluation of VR1 inhibition by the inventive compounds will be described below. The assay was intended to evaluate in vitro inhibition of Ca²⁺ entry in cells caused by proton, one of the VR1 agonists.

Test Example 1: inhibition of Ca²⁺ entry in cells

Rat glioma (C6BU1) cells stably expressing human VR1 were suspended in 20 mM MES buffer (at pH 6.8, contg. 20 mM 2-morpholinoethanesulfonate (referred to as MES hereinafter), 115 mM NaCl, 5 mM KCl, 1 mM MgCl₂ and 14 mM D-glucose) to make a cell density of 1?10⁶ cells/mL. A fluorescent dye, Fura 2-AMsolution (DojindoCorporate, Cat. No. 343-05401) was added to the suspension to make a 5 ?M concentration thereof. Further, Pluronic F-127 (Wako Pure Chemical Industries, Ltd., Cat. No. P6866) was added to make a 0.1% content thereof. Then, the suspension was incubated at 37 ?C for 30 min. The cells were harvested and washed three times with 20 mM MES buffer. The cells were suspended again to make a cell density of 5?10⁵ cells/mL. A 500-?L portion of the suspension was taken with a cuvette (MC MEDICAL, INC., Cat. No. SSR3121), to which 10 ?L of 20 mM MES buffer containing 250 mM CaCl₂ was added to incorporate Ca²⁺ into the cells. At the same time, 5 ?L of a test compound solution (in a range of 100 ?M to 10 nM in DMSO) was also added to provide a final concentration thereof in a range of 1 ?M to 0.1 nM. Alternatively, 5 ?L of DMSO was added as control to provide a final concentration of 1% DMSO. The suspension was set in an intracellular ionometer (CAF-110; JASCO) 10 min after those additions. The cells were stimulated with protons by addition of 60 ?L of 20 mM MES buffer at pH 1.1 to the suspension to set its pH at 5.5. The activity of the test compound was determined as a difference between the minimum of fluorescence intensity before agonist stimulation and its maximum after the stimulation. The value of IC₅₀ was derived from percentage of inhibition by the test compound compared with the control.

The results are shown in Table 1 to Table 11 along with the structural formulae of Example compounds.

In the Tables, "++" indicates that IC50 value is 100 nM or less, and "+" indicates that IC50 value is 100 nM to 1000 nM.

**[Table 1]**

| Ex. | Molucular Structure | m.w. | NMR | in vitro IC50 |
|---|---|---|---|---|
| 1 | 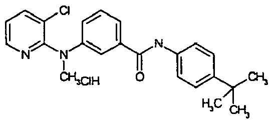 | 430.38 | (400 MHz, DMSO-D6) 1.27 (s, 9 H) 3.42 (s, 3 H) 6.98 (dd, J=8.12, 2.32 Hz, 1 H) 7.21 (dd, J=7.88, 4.64 Hz. 1 H) 7.32 - 7.40 (m, 3 H) 7.55 (d, J=7.65 Hz, 1 H) 7.60 - 7.67 (m, 2 H) 7.91 (dd, J=7.88, 1.62 Hz, 1 H) 8.41 (dd, J=4.64, 1.62 Hz, 1 H) 10.15 (s, 1 H) | ++ |
| 2 | 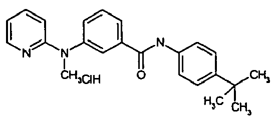 | 395.93 | (400 MHz, DMSO-D6) 1.28 (s, 9 H) 3.59 (s, 3 H) 6.85 (d. J=9.04 Hz, 1 H) 7.01 (t, J=6.61 Hz, 1 H) 7.33-7.42 (m, 2 H) 7.64 - 7.73 (m, 4 H) 7.84 - 7.95 (m, 1 H)8.02-8.13(m, 3H) 10.36 (s, 1 H) | + |
| 3 | 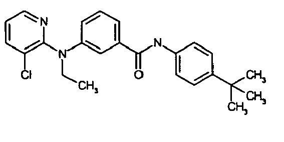 | 407.94 | (300 MHz, DMSO-D6) 1.19(t, J=6.97 Hz, 3H) 1.28 (s, 9 H) 4.01 (q, J=6.97 Hz, 2 H) 6.99 (dd, J=8.07, 2.20 Hz, 1 H) 7.18 (dd, J=7.70, 4.77 Hz, 1 H) 7.33 - 7.42 (m, 4 H) 7.56 (d, J=8.07 Hz, 1 H) 7.66 (d, J=8.80 Hz, 2 H) 7,88 (dd, J=7.89, 1.65 Hz, 1 H) 8.41 (dd, J=4.59, 1.65 Hz, 1H) 10,14 (s, 1H) | ++ |
| 4 | 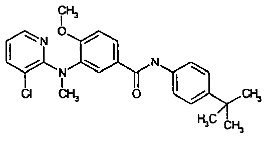 | 423.94 | (400 MHz, CHLOROFORM-D) 1.31 (s, 9 H) 3.38 (s, 3 H) 3.89 (s, 3 H) 6.83 (dd, J=7.65, 4.87 Hz, 1 H) 7.00 (d, J=8.58 Hz, 1 H) 7.31 - 7.40 (m, 2 H) 7.44 - 7.54 (m, 4H) 7.61 - 7.71 (m, 2 H) 8.24 (dd, J=4.87,1.62 Hz, 1 H) | ++ |
| 5 | 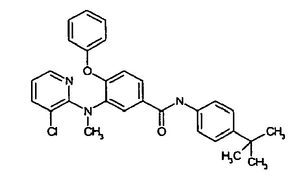 | 486.01 | (400 MHz, DMSO-D6) 1.28 (s, 9 H) 3.38 (s, 3 H) 6.80 - 6.91 (m, 2 H) 6.91 - 7.00 (m, 2 H) 7.10 - 7.17 (m, 1 H) 7.30 - 7.40 (m, 4 H) 7.62 - 7.67 (m, 2 H) 7.68-7.72 (m, 1 H) 7.74 - 7.84 (m, 2 H) 8.22 (dd, J=4.46, 1.62 Hz, 1H) 10.13 (s, 1H) | ++ |
| 6 | 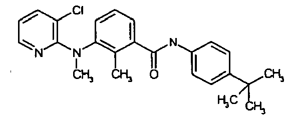 | 407.94 | (300 MHz, DMSO-D6) 1.28 (s, 9 H) 2.33 (s, 3 H) 3.19 (s, 3H) 6.87 (d, *J*=7.70 Hz, 1 H) 6.99 (dd, J=7.70, 4.77 Hz, 1 H) 7.22 (t, J=7.70 Hz, 1 H) 7.28 - 7.41 (m, 3 H) 7.63 - 7.73 (m, 3 H) 8.30 (dd, *J*=4.59, 1.65 Hz, 1 H) 10.26 (s, 1H) | ++ |
| 7 | 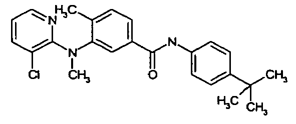 | 407.94 | (300 MHz, DMSO-D6) 1.27 (s, 9 H) 2.30 (s, 3 H) 3.23 (s, 3 H) 6.99 (dd, J=7.70, 4.77 Hz, 1 H) 7.34 (d, J=8.80 Hz, 2 H) 7.38 - 7.47 (m, 2 H) 7.61 (d, J=8.80 Hz. 2 H) 7.66 - 7.72 (m, 1 H) 7.79 (dd, J=7.89, 1.65 Hz, 1 H) 8.27 - 8.33 (m, 1H) 10.06 (s, 1H) | ++ |

**[Table 2]**

| Ex. | Molucular Structure | m.w. | NMR | in vitro IC50 |
|---|---|---|---|---|
| 8 | 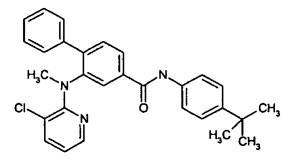 | 470.01 | (400 MHz, DMSO-D6) 1.28 (s, 9 H) 3.07 (s, 3 H) 6.88 (dd, J=7.77, 4.75 Hz, 1 H) 7.29-7.41 (m, 5 H) 7.50 (d, J=7.88 Hz, 1 H) 7.55 - 7.63 (m, 4 H) 7.64 - 7.68 (m, 2H) 7.90 (dd, J=8.00, 1.74 Hz, 1 H) 8.19 (dd, J=4.75, 1.51 Hz, 1H) 10.21 (s, 1 H) | ++ |
| 9 | 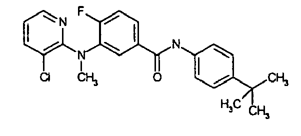 | 411.91 | (400 MHz, DMSO-D6) 1.27 (s, 9 H) 3.39 (s, 3 H) 7.10 (dd, J=7.77, 4.75 Hz, 1 H) 7.31 - 7.40 (m, 3 H) 7.58-7.66 (m, 2 H) 7.68 - 7.72 (m, 1 H) 7.78 - 7.84 (m, 2 H) 8.32 (dd, J=4.75, 1.74 Hz, 1 H) 10.17 (s, 1H) | ++ |
| 10 | 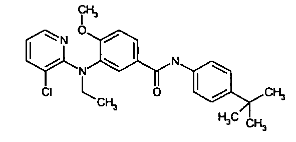 | 437.97 | (400 MHz, DMSO-D6) 1.14 (t. J=6.96 Hz, 3 H) 1.27 (s, 9 H) 3.80 (s, 3 H) 3.87 (q, J=6.96 Hz, 2 H) 6.90 (dd, J=7.65, 4.87 Hz, 1 H) 7.18 (d, J=8.58 Hz, 1 H) 7.31 - 7.41 (m, 2 H) 7.56 (d, J=2.09 Hz, 1 H) 7.59-7.70 (m, 3 H) 7.88 (dd, J=8.58, 2.09 Hz, 1 H) 8.24 (dd, J=4.87, 1.62 Hz, 1 H) 10.01 (s, 1 H) | ++ |
| 11 | 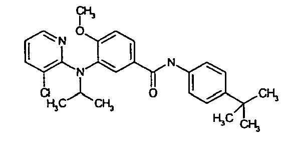 | 452.00 | (400 MHz, DMSO-D6) 1.20 (d, J=6.73 Hz, 6 H) 1.28 (s, 9 H) 3.74 (s, 3H) 4.69 - 4.77 (m, 1 H) 6.85 (dd, J=7.65, 4.64 Hz, 1H) 7.17 (d, J=8.58 Hz, 1 H) 7.31-7.38 (m, 2 H) 7.55 (dd, J=7.77, 1.74 Hz, 1 H) 7.61-7.66 (m, 3 H) 7.94 (dd, J=8.58, 2.32 Hz, 1 H) 8.23 (dd, J=4.75, 1.74 Hz, 1 H) 10.02 (s, 1 H) | ++ |
| 12 | 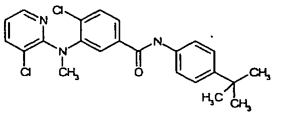 | 428.36 | (400 MHz, DMSO-D6) 1.27 (s, 9 H) 3.32 (s, 3 H) 7.05 (dd, J=7.65, 4.64 Hz, 1 H) 7.33 - 7.38 (m, 2 H) 7.60-7.67 (m, 2 H) 7.66 (d, J=2.09 Hz, 1 H) 7.69 (d, J=8.35 Hz, 1 H) 7.76 (dd, J=7.65, 1.62 Hz, 1 H) 7-85 (dd, J=8.35, 2.09 Hz, 1 H) 8.32 (dd, J=4.75, 1.51 Hz, 1H) 10.22 (s, 1 H) | ++ |
| 13 | 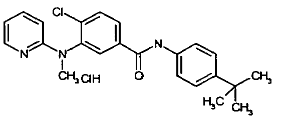 | 430.38 | (400 MHz, DMSO-D6) 1.28 (s, 9 H) 3.52 (s, 3 H) 6.72 (brs, 1H) 6.97 - 7.04 (m, 1 H) 7.35 - 7.41 (m, 2 H) 7.67-7.71 (m, 2 H) 7.85 - 7.91 (m, 2 H) 8.07 - 8.17 (m, 2 H) 8.22 - 8.26 (m, 1 H) 10.41 (s, 1 H) | + |
| 14 | 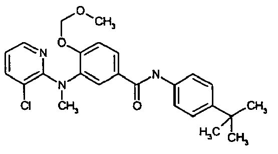 | 453.97 | (400 MHz, CHLOROFORM-D) 1.31 (s, 9 H) 3.41 (s, H) 3.42 (s, 3 H) 5.21 (s, 2 H) 6.84 (dd, J=7.77, 4.75 Hz, 1 H) 7.23 (d, J=8.58 Hz, 1 H) 7.32 - 7.43 (m. 2 H) 7.44 - 7.53 (m, 4 H) 7.59 - 7.68 (m, 2 H) 8.24 (dd, J=4.75, 1.74 Hz, 1 H) | 3 ++ |

**[Table 3]**

| Ex. | Molucular Structure | m.w. | NMR | in vitro IC50 |
|---|---|---|---|---|
| 15 | 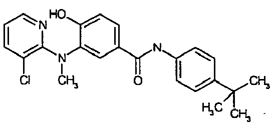 | 409.91 | (400 MHz, DMSO-D6) 1.26 (s, 9 H) 3.25 (s, 3 H) 6.92 - 6.96 (m, 2 H) 7.30 - 7.41 (m, 2 H) 7.49 (d, J=2.09 Hz, 1H) 7.59 - 7.68 (m, 3 H) 7.73 (dd, J=8.46, 2.20 Hz, 1H) 8.26 (dd, J=4.87, 1.62 Hz, 1 H) 9.89 (s, 1 H) 10.18(brs, 1 H) | ++ |
| 16 | 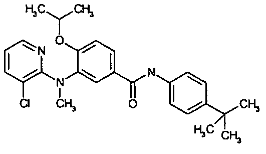 | 452.00 | (400 MHz, CHLOROFORM-D) 1.12- 1.19 (m, 6H) 1.29 (s, 9H) 3.38 (s, 3 H) 4.48 - 4.58 (m, 1 H) 6.77-6.82 (m, 1H) 6.88 (d, J=8.38 Hz, 1 H) 7.34 (d, J=8.58 Hz, 2 H) 7.45 - 7.63 (m, 6 H) 7.55 - 7.65 (m, 1 H) | ++ |
| 17 | 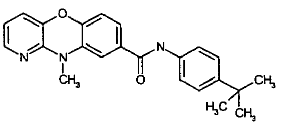 | 373.45 | (400 MHz, CHLOROFORM-D) 1.32 (s, 9 H) 3.28 (s, 3 H) 6.56 (dd, J=7.65. 5.10 Hz, 1 H) 6.67 (d, J=8.12 Hz, 1H) 6.79 (dd, J=7.65, 1.39 Hz, 1 H) 7.08 - 7.19 (m, 2H) 7.39 (d, J=8-81 Hz, 2 H) 7.54 (d, J=8.81 Hz, 2 H) 7.63 -7.74 (m, 2 H) | ++ |
| 18 | 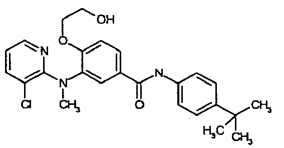 | 453.97 | (400 MHz, DMSO-D6) 1.27 (s, 9 H) 3.32 (s, 3 H) 3.51 (q, J=5.45 Hz. 2 H) 4.03 (t, J=5.45 Hz, 2 H) 4.74 (t, J=5.45 Hz, 1 H) 6.96 (dd, J=7.65, 4.64 Hz, 1 H) 7.17 (d, J=8.58 Hz, 1 H) 7.28 - 7.37 (m, 2 H) 7.56 (d, J=2.32 Hz, 1 H) 7.61 - 7.66 (m, 2 H) 7.67 (dd, J=7.88, 1.62 Hz. 1 H) 7.83 (dd, J=8.58, 2.32 Hz, 1 H) 8.26 (dd. J=4.87, 1.62 Hz, 1 H) 10.00 (s, 1 H) | ++ |
| 19 | 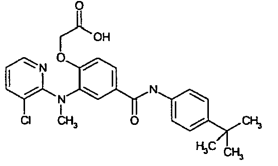 | 467.95 | (400 MHz, DMSO-D6) 1.26 (s, 9 H) 3.32 (s, 3 H) 4.84 (s, 2 H) 6.96 (dd, J=7.77, 4.75 Hz, 1 H) 7.12 (d, J=8.58 Hz, 1 H) 7.33 (d, J=8.81 Hz, 2 H) 7.45 (d, J=2.09 Hz, 1 H) 7.58 - 7.68 (m, 3H) 7.82 (dd, J=8.70. 2.20 Hz, 1 H) 8.28 (dd, J=4.75, 1.51 Hz, 1 H) 10.00 (s, 1 H) 13.08 (brs. 1 H) | |
| 20 | 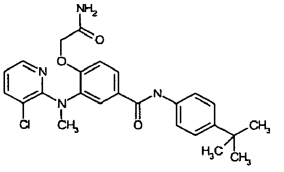 | 466.97 | (400 MHz, DMSO-06) 1.24 (s, 9 H) 3.29 (s, 3 H) 4.48 (s, 2 H) 6.73 (brs, 1 H) 6.94 (dd, J=7.65, 4.87 Hz, 1H) 7.08 (d, J=8.81 Hz, 1H) 7.29 - 7.33 (m, 2 H) 7.43 (brs, 1 H) 7.58 - 7.62 (m, 3 H) 7.69 (dd, J=7.77, 1.51 Hz, 1 H) 7.82 (dd, J=8.70, 2.20 Hz, 1 H) 8.28 (dd, J=4.75,1.51 Hz, 1 H) 10.00 (s, 1 H) | ++ |

**[Table 4]**

| Ex. | Molucular Structure | m.w. | NMR | in vitro IC50 |
|---|---|---|---|---|
| 21 | 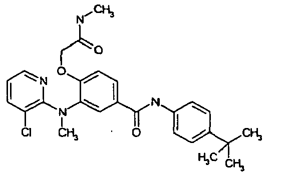 | 480.99 | (400 MHz, DMSO-D6) 1.27 (s, 9 H) 2.63 (d, J=4.64 Hz, 3H) 3.37 (s, 3 H) 4.53 (s, 2 H) 6.99 (dd, J=7.65, 4.87 Hz, 1 H) 7.13 (d, J=8.58 Hz, 1 H) 7.15. 7.20 (m, 1 H) 7.32-7.37 (m, 2 H) 7.61-7.66 (m, 3 H) 7.70 (dd, J=7.65, 1.62 Hz, 1 H) 7.83 (dd, J=8.70, 2.20 Hz, 1 H) 8.30 (dd, J=4.87,1.62 Hz, 1 H) 10.04 (s, 1 H) | ++ |
| 22 | 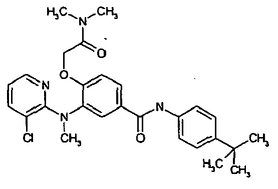 | 495.02 | (400 MHz, DMSO-D6) 1.26 (s, 9 H) 2.28 (s, 3 H) 2.97 (s, 3 H) 3.34 (s, 3 H) 4.96 (s, 2 H) 6.95 (dd. J=7.65, 4.87 Hz, 1 H) 7.08 (d, J=8.58 Hz, 1 H) 7.30 - 7.34 (m, 2 H) 7.46 (d, J=2.09 Hz, 1 H) 7.59 - 7.62 (m, 2 H) 7.65 (dd, J=7.65, 1.62 Hz, 1H) 7.76 - 7.80 (m, 1H) 8.26 (dd, J=4.87, 1.62 Hz, 1H) 9.98 (s, 1H) | + |
| 23 | 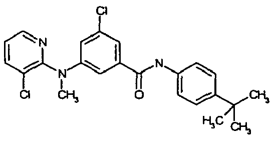 | 428.36 | (400 MHz, DMSO-D6) 1.27 (s, 9 H) 3.41 (s, 3 H) 6.97 -7.00 (m, 1H) 7.10 - 7.20 (m, 1H) 7.27 - 7.38 (m, 3 H) 7.52 - 7.55 (m, 1 H) 7.58 - 7.66 (m. 2 H) 8.02 (dd, J=7.88, 1.62 Hz, 1H) 8.47 (dd, J=4.64, 1.62 Hz, 1 H) 10.21 (s. 1H) | ++ |
| 24 | 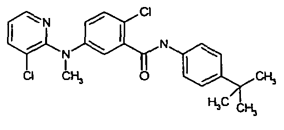 | 428.36 | (400 MHz, DMSO-D6) 1.27 (s, 9 H) 3.37 (s, 3 H) 6.79 (dd, J=8.81, 3.01 Hz, 1 H) 6.95 (d, J=2.78 Hz, 1 H) 7.27 (dd, J=7.88. 4.64 Hz, 1 H) 7.31- 7.41 (m, 3 H) 7.56 - 7.66 (m, 2 H) 7.97 (dd, J=7.88, 1.62 Hz, 1 H) 8.43 (dd, J=4.64, 1.62 Hz, 1 H) 10.37 (s, 1 H) | + |
| 25 | 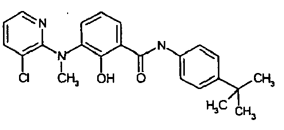 | 409.91 | (400 MHz, DMSO-D6) 1.29 (s, 9 H) 3.26 (s, 3 H) 6.86 - 6.97 (m. 2 H) 7.07 - 7.11 (m, 1 H) 7.38 - 7.44 (m, 2 H) 7.58 - 7.63 (m, 2 H) 7.66 (dd, J=7.65, 1.62 Hz, 1 H) 7.89 (dd, J=8,12, 1.62 Hz. 1 H) 8.25 (dd, J=4.87, 1.62 Hz, 1H) 10.44 (s, 1H) 12.64 (s, 1H) | + |
| 26 | 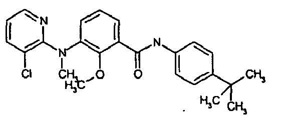 | 423.94 | (400 MHz, DMSO-D6) 1.27 (s, 9 H) 3.37 (s, 3 H) 3.71 (s, 3 H) 7.00 - 7.05 (m, 2 H) 7.13 (t, J=7.77 Hz. 1 H) 7.30 (dd. J=7.65, 1.62 Hz, 1 H) 7.32-7.38 (m, 2 H) 7.60 - 7.69 (m, 2 H) 7.75 (dd. J=7.77, 1.51 Hz, 1 H) 8.30 (dd. J=4.75, 1.51 Hz, 1 H) 10.20 (s, 1 H) | ++ |
| 27 | 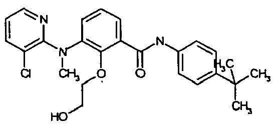 | 453.97 | (400 MHz, DMSO-D6) 1.28 (s, 9 H) 3.32 (s, 3 H) 3.49 (q, J=5.18 Hz, 2 H) 4.11 (t, J=5.18 Hz, 2 H) 5.00 (t, J=5.18 Hz, 1H) 6.97 - 7.08 (m, 2 H) 7.15 (t, J=7.88 Hz, 1 H) 7.30 - 7.37 (m, 2 H) 7.54 (dd, J=7.65, 1.66 Hz, 1 H) 7.60 - 7.67 (m, 2 H) 7.75 (dd, J=7.65, 1.62 Hz. 1H) 8.31 (dd, J=4.64, 1.62 Hz, 1 H) 10.32 (s, 1 H) | ++ |

**[Table 5]**

| Ex. | Molucular Structure | m.w. | NMR | in vitro IC50 |
|---|---|---|---|---|
| 28 | 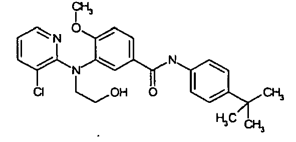 | 453.97 | (400 MHz, DMSO-D6) 1.27 (m, 9 H) 3.55 - 3.66 (m, 2 H) 3.77 (s, 3H) 3.91 (t, J=7.07 Hz, 2 H) 4,76 (t, J=5.45 Hz, 1 H) 6.91 (dd, J=7.77, 4.75 Hz, 1 H) 7.16 (d, J=8.58 Hz, 1 H) 7.27 - 7.37 (m, 2 H) 7.61-7.68 (m, 4 H) 7.87 (dd, J=8.58, 2.32 Hz, 1 H) 8.23 (dd, J=4.64, 1.62 Hz, 1H) 10.02 (s, 1H) | ++ |
| 29 | 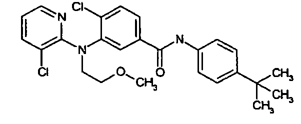 | 472.4133 | (400MHz,DMSO-d6) 1.28 (s, 9H), 3.64 (t, J = 6.5 Hz, 2H), 4.05 (t, J = 6.5 Hz, 2H), 7.02 (dd, J = 7.7, 4.9 Hz, 1H), 7.36 (d, J = 8.8 Hz, 2H), 7.64-7.66 (m, 3H), 7.73 (dd, J = 7.9,1.4 Hz, 1 H), 7.80 (d, J = 1.9 Hz, 1H), 7.85 (dd, J = 8.3, 2.3 Hz, 1H), 8.31 (dd, J = 4.9, 1.6 Hz, 1H), 10.27 (s, 1H). | ++ |
| 30 | 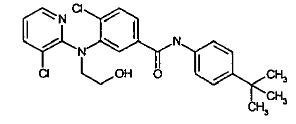 | 458.3865 | (400MHz,DMSO-d6) 1.28 (s, 9H), 3.64-3.70 (m, 2H), 3.96 (t, 2H, J = 7.0 Hz), 4.81 (t, 1H, J = 5.6 Hz), 6.97-7.03 (m, 1H), 7.37 (t. 2H, J = 4.4 Hz), 7.65 (dd, 3H, J = 8.6, 6.7 Hz), 7.73 (dd, 1H, J = 7.7, 1.6 Hz), 7.82-7.84 (m. 2H). 8.30 (q. 1H, J=2.2 Hz), 10.24 (s, 1H). | ++ |
| 31 | 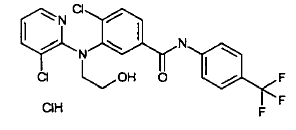 | 506.7427 | (400MHz, DMSO-d6) 3.67 (t, 2H, J =7.0 Hz), 3.97 (1, 2H, J = 6.8 Hz), 7.01 (dd, 1H, J = 7.7, 4.8 Hz), 7.67-7.73 (m, 4H), 7.87 (d. 2H, J = 7.0 Hz), 7.99 (d, 2H, J = 8.4 Hz), 8.29 (dd, 1H, J = 4.8, 1.5 Hz), 10.67 (s, 1H), | ++ |
| 32 | 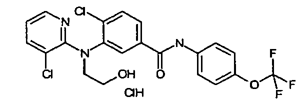 | 522.732 | (400 MHz, DMSO-d6) 3.63-3.68 (m, 2H). 3.94 (dd, J = 9.0, 4.9 Hz, 2H), 7.00 (q, J = 4.2 Hz, 1H), 7.36 (d, J = 8.3 Hz, 2H), 7.66 (d, J = 8.3 Hz, 1H), 7.72 (dd, J = 7.9, 1.9 Hz, 1H), 7.83 (tt, J = 7.7, 2.6 Hz, 4H), 8.28 (dd, J=4.6, 1.4Hz, 1H), 10.48 (s, 1H). | ++ |
| 33 | 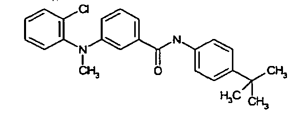 | 392.93 | (300 MHz, CDCl3) 1.32 (s, 9 H) 3.31 (s, 3 H) 6.68 (dd, *J*=8.25, 2.75 Hz, 1 H) 7.12 - 7.43 (m, 6 H) 7.49-7.58 (m, 4H) 7.70 (s, 1 H) | ++ |
| 34 | 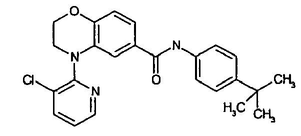 | 421.93 | (300 MHz, DMSO-D6 1.26 (s, 9 H) 3.78 - 3.83 (m, 2 H) 4.34 - 4.40 (m, 2 H) 6.97 (d, J=8.44 Hz, 1 H) 7.07 (d, J=1.83 Hz, 1H) 7.29-7.34 (m, 3 H) 7.50 (dd, J=8.44, 2.20 Hz, 1H) 7.59 (d, J=8.80 Hz, 2H) 8.06 (dd, J=8.07, 1.47 Hz, 1H) 8.43 (dd, J=4.59, 1.65 Hz, 1H) 9.90 (s, 1H) | |
| 35 | 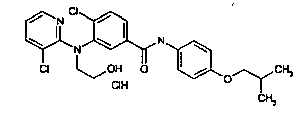 | 510.8464 | (400 MHz, DMSO-d6) 0.96 (d, J = 13.8 Hz, 6H), 1.99 (dt, J = 14.8, 5.4 Hz, 1H), 3.65 (dd, J = 8.8, 5.1 Hz, 2H), 3.70 (d, J = 15.8 Hz, 2H), 3.93 (t, J = 7.0 Hz, 1H), 6.90 (dt, J = 10.0, 2.7 Hz, 2H), 6.99 (dd, J = 7.7, 4.9 Hz, 1H), 7.57-7.82 (m, 6H), 8.28 (q, J = 2.2 Hz, 1H), 10.16 (s, 1H). | ++ |

**[Table 6]**

| Ex. | Molucular Structure | m.w. | NMR | in vitro IC50 |
|---|---|---|---|---|
| 36 | 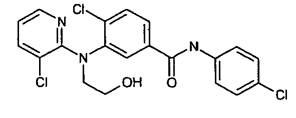 | 436.7284 | (400 MHz, DMSO-D6) 3.62 - 3.72 (m, 2 H) 3.89-3.99 (m, 2H) 4.80 (t, J=5.45 Hz, 1 H) 7.01 (dd, J=7.77, 4.75 Hz, 1 H) 7.38 - 7.49 (m, 2 H) 7.67 (d, J=8.12 Hz, 1 H) 7.72 (dd, J=7.65, 1.62 Hz, 1H) 7.75 7.84 (m, 4H) 8.30 (dd, J=4.64, 1.62 Hz, 1 H) 10.41 (s, 1H) | ++ |
| 37 | 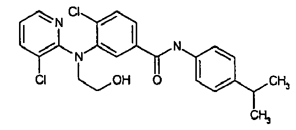 | 444.354 | (400MHz, DMSO-d6) 1.18 (d, J = 46.8 Hz, 6H), 2.79-2.90 (m, 1H), 3.58-3.68 (m, 2H), 3.92 (t, J = 15.5 Hz, 2H), 4.77 (t, J = 7.3 Hz, 1H), 6.97-7.01 (m, 1H), 7.17-7.22 (m, 2H), 7.60-7.64 (m, 3H), 7.70-7.71 (m, 1H). 7.79-7.81 (m, 2H), 8.27-8.29 (m, 1H), 10.21 (t, J = 16.5 Hz, 1H). | ++ |
| 38 | 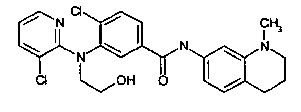 | 471.379 | (CHLOROFORM-D) 1.83 - 1.91 (m, 2H), 2.65 (t. J = 6.2 Hz, 2H), 2.81 (s, 3H), 3.18 (t, J = 5.7 Hz, 2H), 3.67 (q, J = 6.5 Hz, 2H), 3.95 (t, J = 7.0 Hz, 2H). 4.80 (t, J = 5.5 Hz, 1H), 6.83 (d, J = 8.1 Hz, 1H), 6.93 (dd, J = 5.0, 2.5 Hz, 1H), 6.98 - 7.02 (m, 2H), 7.63 (d, J = 8.4 Hz, 1H), 7.71 (dd, J = 7.9, 1.7 Hz, 1H), 7.78 (d, J = 1.8 Hz, 1H), 7.82 (dd, J = 8.3, 2.0 Hz, 1H), 8.29 (dd. J = 4.8, 1.5 Hz, 1H). 10.00 (s, 1 H). | ++ |
| 39 | 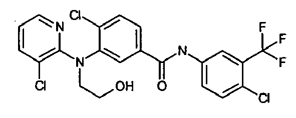 | 504.7215 | (300 MHz, DMSO-D6) 3.67 (q. J = 6.2 Hz, 2H), 3.96 (t. J = 6.2 Hz, 2H), 4.79 (t, J = 6.2 Hz, 1H), 7.01 (dd. J = 7.7. 4.8 Hz. 1H), 7.68-7.74 (m. 3H), 7.83-7.87 (m, 2H), 8.07 (dd, J = 8.8, 2.6 Hz, 1H), 8.29-8.32 (m, 2H), 10.66 (s, 1H). | ++ |
| 40 | 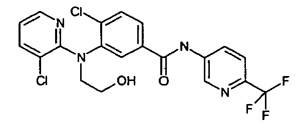 | 471.26 | (300 MHz, DMSO-d6) 3.66 (q. J = 6.3 Hz, 2H), 3.92-3.96 (m, 2H), 4.79 (t, J = 5.3 Hz, 1H), 7.00 (dd, J = 7.7, 4.9 Hz, 1H), 7.68-7.73 (m, 2H), 7.70 (s, 2H), 7.85-7.90 (m, 3H), 8.28 (dd, J = 4.6, 1.4 Hz, 1H), 8.42 (dd, J = 8.8, 2.3 Hz, 1H), 9.05 (d, J = 2.3 Hz, 1H), 10.82 (q, J = 1.7 Hz, 1H), | ++ |
| 41 | 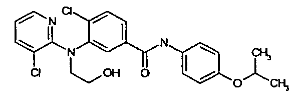 | 460.353 | (300 MHz. CHLOROFORM-D) 1.32 (d, J = 6.2 Hz, 6H). 3.72 (t, J = 4.6 Hz, 2H), 4.17 (t, J = 4.6 Hz, 2H), 4.47 - 4.55 (m, 1H), 6.03 (s, 1H), 6,87 - 6.93 (m, 3H), 7.50 - 7.55 (m, 5H), 7.68 (dd. J = 8.3, 2.0 Hz, 1H), 7.91 (s, 1H), 8.19 (dd, J = 4.8, 1.5 Hz, 1H). | ++ |
| 42 | 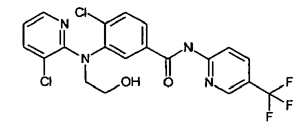 | 471.26 | (300 MHz, CHLOROFORM-D) 1.83 (s, 1H), 3.72 (t, J = 4.6 Hz, 2H), 4.17 (t, J = 4.6 Hz, 2H), 5.85 (s, 1H), 6.95 (dd, J = 7.9, 5.0 Hz, 1H), 7.56 - 7.66 (m, 3H), 7.73 (dd, J = 8.4, 2.2 Hz, 1H), 7.97 (dd, J = 8.8, 2.6 471.26 1H). 8.21 (dd, J = 5.0.1.7 8.8 Hz, 1H). 8.45 (d, J = Hz. 1H). 8.55 (d, J = 0.7 Hz, 1H), 8.82 (s, 1H). | |
| 43 | 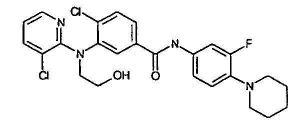 | 8.29 | (400 MHz, DMSO-d6) 1.51-1.53 (m, 2H), 1.63-1.66 (m, 4H), 2.92-2.93 (m, 4H). 3.67 (q, J = 6.4 Hz. 2H), 3.94 (L J = 7.0 Hz. 2H), 4.79 (t, J = 5.3 Hz, 1H), 6.98-7.04 (m, 2H), 7.40 (dd, J = 6.6.1.5 Hz, 1H). 7.60-503.396 (m. 2H). 7.69-7.73 (m, 1H). 7.79-7.82 (m, 2H), (dd, J = 4.8, 1.5 Hz, 1H), 10.27 (s, 1H). | ++ |

**[Table 7]**

| Ex. | Molucular Structure | m.w. | NMR | in vitro |
|---|---|---|---|---|
| 44 | 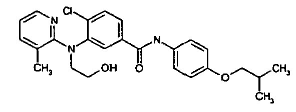 | 453.961 | (400 MHz, CHLOROFORM-D) 1.02 (d, J = 6.5 Hz, 6H), 2.05 - 2.11 (m, 1H), 2.14 (s. 3H), 3.72 (d, J = 6.5 Hz, 2H), 3.88 (1, J = 3.9 Hz, 2H), 5.88 (s, 1H), 6.21 (s. 1H). 6.56 (d, J = 5.1 Hz, 1H), 6.90 (d, J = 8.8 Hz, 2H), 7.51 (d, J = 8.8 Hz, 2H), 7.67 (d, J = 8.3 Hz, 1H), 7.77 (s. 1H), 7.85 (d, J = 8.3 Hz, 2H), 8.01 (d, J = 5.1 Hz, 1H). | |
| 45 | 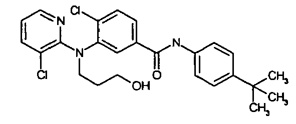 | 472.407 | (400MHz,DMSO-d6) 1.25 (s, 9H), 1.76-1.83 (m, 2H), 3.43-3.48 (m, 2H), 3.88 (dd, J = 8.8, 6.5 Hz, 2H), 4.47 (t, J = 5.3 Hz, 1H), 7.00 (q, J = 4.2 Hz, 1H), 7.34 (dd, J = 6.7, 4.9 Hz, 2H), 7.60-7.72 (m, 5H), 7.82 (dd, J = 8.3, 1.9 Hz, 1H), 8.28-8.30 (m, 1H), 10.21 (s, 1H). | ++ |
| 46 | 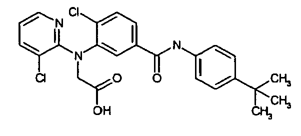 | 472.3697 | (300 MHz, DMSO-D6) 1.28 (s, 9H), 4.46 (s, 2H), 7.02 (dd, J = 7.7, 4.8 Hz, 1H), 7.34-7.38 (m, 2H), 7.60-7.64 (m, 2H), 7.68 (d, J = 8.4 Hz, 1H), 7.73 (dd, J = 7.7, 1.5 Hz, 1H), 7.78 (d, J = 1.8 Hz, 1H), 7.87 (dd, J = 8.3, 2.0 Hz, 1H), 8.26 (dd, J = 4.8, 2.1 Hz, 1H), 10,23 (s, 1H), 12.54 (brs, 1H), | ++ |
| 47 | 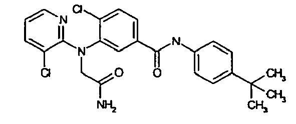 | 471.3856 | (300 MHz, DMSO-D6) 1.27 (s, 9H), 4.39 (s, 2H), 6.97-7.03 (m, 2H), 7.26 (brs, 1H), 7.36 (d, J = 8.8 Hz, 2H), 7.60-7.68 (m, 3H), 7.72 (dd, J = 7.9, 1.7 Hz, 1H), 7.81-7.86 (m, 2H), 8.25 (dd, J = 4.8, 1.5 Hz, 1H), 10.22 (s, 1H). | ++ |
| 48 | 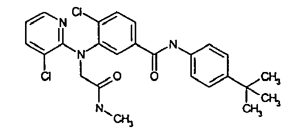 | 485.4124 | (300 MHz, DMSO-D6) 1.27 (s, 9H), 2.59 (d, J = 4.4 Hz. 3H), 4.42 (s, 2H), 6.99 (dd, J = 7.9, 4.6 Hz, 1H), 7.36 (d, J = 8.8 Hz, 2H), 7.61-7.66 (m, 3H), 7.71 (dd, J = 7.7, 1.8 Hz, 1H), 7.77 (q, J = 4.4 Hz. 1H), 7.81-7.86 (m, 2H), 8.23 (dd, J = 4.6, 1.8 Hz, 1H), 10.22 (s, 1H). | ++ |
| 49 | 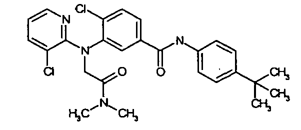 | 499.4392 | (300 MHz, DMSO-D6) 1.27 (s, 9H), 2.81 (s. 3H). 3.02 (s, 3H), 4.71 (s, 2H), 6.95 (dd, J = 7.9, 4.6 Hz, 1H), 7.35 (d, J = 8.8 Hz, 2H), 7.62-7.71 (m, 4H), 7.81-7.86 (m, 2H), 8.21 (dd, J = 4.6, 2.2 Hz. 1H). 10.21 (s, 1H). | + |
| 50 | 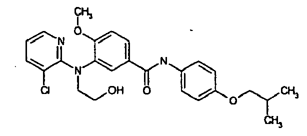 | 469.961 | (400 MHz, DMSO-d6) 0.99 (I, J = 7.9 Hz, 6H), 1.95-2.05 (m, 1H), 3.61 (q. J = 6.2 Hz, 2H). 3.72 (d, J = 6.5 Hz, 2H), 3.77 (s, 3H), 3.91 (t, J = 7.0 Hz, 2H), 4.75 (t. J = 5.1 Hz, 1H), 6.87-6.93 (m, 3H), 7.16 (d, J = 8.3 Hz. 1H), 7.59-7.66 (m, 4H), 7.86 (d. J = 8.3 Hz, 1H), 8.23 (d, J = 4.6 Hz, 1H), 9.94 (s, 1H). | ++ |
| 51 | 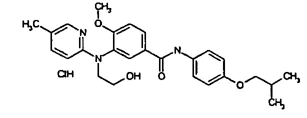 | 486.003 | (400 MHz, DMSO-d6) 0.95 (d, J = 6.5 Hz, 6H), 1.94-2.01 (m, 1H), 2.19 (s, 3H), 3.61 (t, J = 5.8 Hz, 2H), 3.70 (d, J = 6.5 Hz, 2H), 3.82 (s, 3H), 3.88-3.91 (m, 2H), 6.90 (d, J = 6.3 Hz, 3H), 7.36 (d, J = 8.8 Hz, 1H), 7.61 (d, J = 8.8 Hz, 3H), 7.80 (s, 1H), 0.08 (s, 1H), 8.12 (d, J = 8.8 Hz, 1H), 10.06 (s, 1H), | ++ |

**[Table 8]**

| Ex. | Molucular Structure | m.w. | NMR | in vitro IC50 |
|---|---|---|---|---|
| 52 | 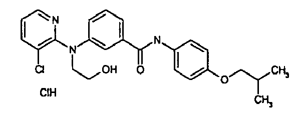 | 476.395 | (300 MHz, DMSO-d6) 1.06 (d, J = 82.2 Hz, 6H), 1.99 (dq, J = 26.7, 6.7 Hz, 1H), 3.62 (t, J = 6.4 Hz, 2H), 3.72 (d, J = 6.6 Hz, 2H), 4.05 (t, J = 6.4 Hz, 2H), 6.90 (d, J = 8.8 Hz, 2H), 7.01 (dd, J = 8.1, 1.8 Hz, 1H), 7.19 (dd, J = 7.9, 4.6 Hz, 1H), 7.35-7.38 (m, 2H), 7.54 (d, J = 7.7 Hz, 1H), 7.62 (d, J = 9.2 Hz, 2H), 7.87 (dd, J = 7.9, 1.7 Hz, 1H), 8.41 (dd, J = 4.4, 1.5 Hz, 1H), 10.07 (s. 1H). | ++ |
| 53 | 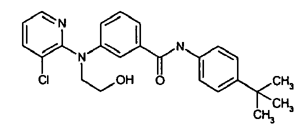 | 423.935 | (300 MHz, DMSO-d6) 1.25 (br s, 9H), 3.62 (dd, J = 11.9, 6.8 Hz, 2H), 4.03 (dt, J = 12.3, 4.9 Hz, 2H), 4.84 (t, J = 5.3 Hz, 1H), 7.02 (dd, J = 9.5, 4.8 Hz, 1H), 7.19 (dd, J = 7.7, 4.8 Hz, 1H), 7.33-7.40 (m, 4H), 7.54 (d, J = 8.1 Hz, 1H), 7.62-7.66 (m, 2H), 7.87 (dd, J= 7.9, 1.7 Hz, 1H), 8.41 (dd, J = 4.8, 1.5 Hz, 1H). 10.12 (s, 1H). | ++ |
| 54 | 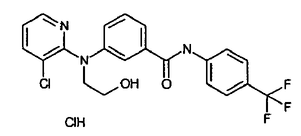 | 472.288 | (300 MHz, DMSO-d6) 3.63 (t, J = 6.6 Hz, 2H), 4.07 (t, J = 6.6 Hz, 2H), 7.04 (dd, J = 7.3, 3.7 Hz, 1H), 7.20 (dd, J = 8.1, 4.8 Hz, 1H). 7.40 (dd, J = 10.6, 5.1 Hz, 2H), 7.57 (d, J = 8.1 Hz, 1H), 7.71 (d, J = 8.8 Hz, 2H), 472.288 7.89 (dd, J = 7.7, 1.5 Hz, 1H), 8.00 (d, J = 8.4 Hz, 2H). 8.42 (dd, J = 4.8, 1.5 Hz, 1H), 10.56 (s, 1H). | ++ |
| 55 | 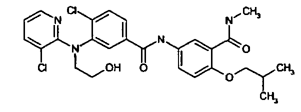 | 531.431 | (300 MHz, DMSO-d6) 1.00 (d, J = 7.0 Hz, 7H), 2.07-2.15 (m, 1H), 2.83 (d, J = 4.4 Hz, 3H), 3.67 (q, J = 6.5 Hz, 2H), 3.88-3.97 (m, 4H), 4.77 (t, J = 5.3 Hz, 1H), 7.00 (dd, J = 7.7, 4.8 Hz, 1H), 7.13 (d, J = 8.8 Hz, 1H), 7.64 (d, J = 8.4 Hz, 1H), 7.71 (dd, J = 7.7, 1.5 Hz, 1H), 7.80-7.92 (m, 3H), 8.00-8.10 (m, 2H), 8.29 (dd, J = 4.6, 1.7 Hz. 1H), 10.28 (s, 1H). | ++ |
| 56 | 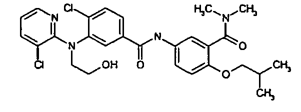 | 545.458 | (300 MHz, DMSO-d6) 0.96 (d. J = 6.6 Hz, 6H), 1.90-2.05 (m, 1H), 2.77 (s, 3H), 2.97 (s, 3H), 3.67 (q, J = 6.6 Hz, 2H), 3.77 (d, J = 6.6 Hz, 2H), 3.94 (t, J = 6.8 Hz, 2H), 4.77 (t, J = 5.3 Hz, 1H), 6.97-7.08 (m, 2H), 7.52 (d, J = 2.6 Hz, 1 H), 7.62-7.74 (m, 3H), 7.78-7.85 (m, 2H), 8.28-8.31 (m, 1H), 10.21 (s, 1H). | ++ |
| 57 | 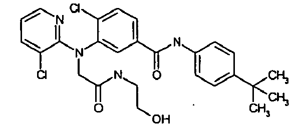 | 515.432 | (300 MHz, DMSO-d6) 1.27 (s, 9H), 3.13 (q, J = 5.7 Hz, 2H), 3.37 (q, J = 5.6 Hz, 2H), 4.43 (s, 2H), 4.63 (t, J = 5.3 Hz, 1H), 7.00 (dd, J = 7.9, 4.6 Hz, 1H). 7.35-7.37 (m, 2H), 7.62-7.65 (m, 3H), 7.72 (dd, J = 7.7, 1.5 Hz, 1H), 7.83-7.85 (m, 3H), 8.23 (dd, J = 4.6, 2.2 Hz, 1H), 10.21 (s, 1H). | ++ |
| 58 | 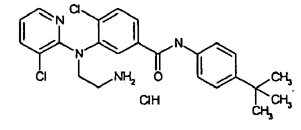 | 493.856 | (300 MHz, DMSO-d6) 1.27 (s, 9H), 3.11-3.12 (m, 2H), 4.10-4.12 (m, 2H), 7.07 (dd, J = 7.9, 4.6 Hz, 1H), 7.35 (d, J = 8.4 Hz, 2H), 7.69-7.76 (m, 4H), 7.91-7.93 (m, 2H), 8.16 (brs, 2H), 8.32-8.33 (m, 1H), 10.41 (s, 1H). | |
| 59 | 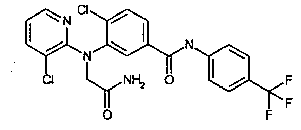 | 483.271 | (300 MHz, DMSO-d6) 4.41 (s, 2H), 6.98-7.03 (m, 2H), 7.26 (s, 1H), 7.69-7.72 (m, 4H), 7.85-7.88 (m, 2H), 7.96 (d, J = 8.4 Hz, 2H), 8.23-8.25 (m, 1H), 10.60 (s, 1H). | ++ |

**[Table 9]**

| Ex. | Molucular Structure | m.w. | NMR | in vitro IC50 |
|---|---|---|---|---|
| 60 | 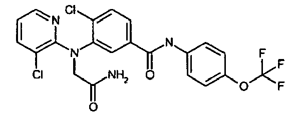 | 499.27 | (400 MHz, DMSO-d6) 4.39 (s, 2H), 6.95-7.05 (m, 2H), 7.27 (brs, 1H), 7.36 (d, J = 8.8 Hz, 2H), 7.65-7.73 (m, 2H), 7.77-7.83 (m, 4H), 8.25 (dd, J = 4.8, 1.5 Hz, 1H), 10.47 (s, 1H). | ++ |
| 61 | 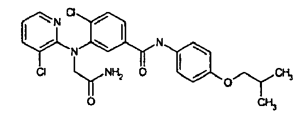 | 487.378 | (400 MHz, DMSO-d6) 0.97 (d, J = 6.8 Hz, 6H), 1.94-2.04 (m, 1H). 3.72 (d, J = 6.8 Hz, 2H), 4.39 (s, 2H), 6.92 (d, J = 8.8 Hz, 2H), 6.97-7.05 (m, 2H), 7.26 (brs, 1H), 7.58 (d, J = 8.8 Hz, 2H), 7.65 (d, J = 7.9 Hz, 1H), 7.72 (d, J = 7.9 Hz, 1H), 7.80-7.83 (m, 2H), 8.25 (dd, J=4.8, 1.5 Hz, 1H), 10.16 (s, 1H). | ++ |
| 62 | 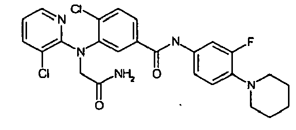 | 516.395 | (400 MHz, DMSO-d6) 1.45-1.55 (m, 2H), 1.58-1.68 (m, 4H), 2.87-2.98 (m, 4H), 4.39 (s, 2H), 6.92-7.05 (m, 3H), 7.26 (brs, 1H), 7.39 (d, J = 7.9 Hz, 1H), 7.55-7.78 (m, 3H), 7.78-7.85 (m, 2H), 8.25 (dd, J = 4.8, 1.5 Hz, 1 H), 10.28 (s, 1H). | ++ |
| 63 | 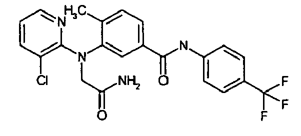 | 462.852 | (300 MHz, DMSO-d6) 2.28 (s, 3H), 4.28 (s, 2H). 6.94-6.97 (m. 2H), 7.28 (s, 1H), 7.43 (d, J = 8.1 Hz, 1H), 7.56 (d, J = 1.8 Hz, 1 H), 7.66-7.71 (m, 3H), 7.79 (dd, J = 7.9, 1.7 Hz, 1H), 7.96 (d, J = 8.4 Hz, 2H). 8.23 (dd, J = 4.8, 1.5 Hz, 1H), 10.46 (s, 1H), | ++ |
| 64 | 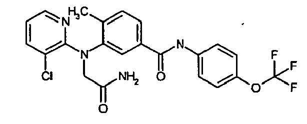 | 478.852 | (300 MHz, DMSO-d6) 2.28 (s, 3H), 4.28 (s, 2H), 6.92-6.98 (m, 2H), 7.27 (br s, 1H), 7.34 (d, J = 8.4 Hz, 2H), 7.42 (d, J = 8.1 Hz, 1H), 7.54 (d, J = 1.5 Hz, 1H), 7.67 (dd, J = 7.7, 1.5 Hz, 1H), 7.77 (dd, J = 8.1, 1.8 Hz, 1H), 7.82-7.86 (m, 2H), 8.23 (dd, J = 4.6. 1.7 Hz, 1H), 10.31 (s, 1H). | ++ |
| 65 | 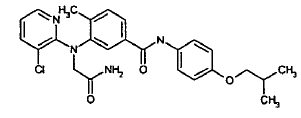 | 466.96 | (300 MHz, DMSO-d6) 0.97 (d, J = 6.6 Hz, 6H), 1.98 (dq, J = 26.4, 6.6 Hz, 1H), 2.27 (s, 3H), 3.71 (d, J = 6.6 Hz, 2H), 4.27 (s, 2H), 6.90-6.94 (m, 4H), 7.26 (br s, 1H), 7.38 (d, J = 8.1 Hz, 1H), 7.52 (d, J = 1.5 Hz, 1H), 7.58 (d, J = 9.2 Hz, 2H), 7.67 (dd, J = 7.7, 1.8 Hz, 1H), 7.75 (dd, J = 7,9,1,7 Hz, 1H), 8.22 (dd, J = 4.8, 1.5 Hz, 1 1H), 9.99 (s, 1H). | ++ |
| 66 | 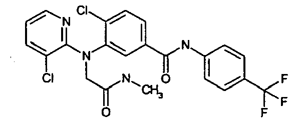 | 497.297 | (400 MHz, CDCl₃) 2.78 (d, J = 4.6 Hz. 3H), 4.43 (s, 2H), 6.96 (dd, J = 7.9, 5.1 Hz, 1H), 7.09 (d, J = 5.1 Hz, 1H), 7.50 (d, J = 8.3 Hz, 1H), 7.57-7.59 (m, 3H), 7.63-7.68 (m, 2H), 7.79 (d, J = 8.3 Hz, 2H), 8.21 (q, J = 2.2 Hz, 1H), 8.92 (s, 1H). | ++ |
| 67 | 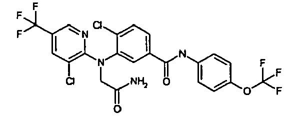 | 567.268 | (400 MHz, DMSO-d6) 4.48 (s, 2H), 7.04 (brs, 1 H), 7.37 (d, J = 8.4 Hz, 2H), 7.42 (brs, 1H), 7.74 (d, J = 8.4 Hz, 2H), 7.85 (d, J = 9.2 Hz, 2H), 7.94 (dd, J = 8.4, 1.8 Hz, 2H), 8.02 (d, J = 1.8 Hz, 1H), 8.11 (d, J = 1.8 Hz, 1H), 8.60 (d, J = 1.8 Hz, 1H), 10.49 (s, 1H | ++ |

**[Table 10]**

| Ex. | Molucular Structure | m.w. | NMR | IC50 |
|---|---|---|---|---|
| 68 | 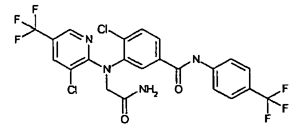 | 551.269 | (400 MHz, DMSO-d6) 4.48 (s, 2H), 7.05 (brs. 1H), 7.43 (brs, 1H), 7.73-7.75 (m, 3H), 7.92-7.97 (m, 3H), 8.08 (d. J = 1.8Kz, 1H). 8.12 (d. J = 1.8 Hz, 1H), 8.60 (d, J = 1.8 Hz. 1H). 10.65 (s, 1H). | ++ |
| 69 | 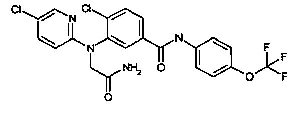 | 499.27 | (400 MHz, DMSO-d6) 4.36 (s, 2H), 6.23 (d, J = 8.8 Hz, 1 H), 7.09 (s,1H), 7.38 (d, J = 8.8 Hz, 2H), 7.45 (s, 1H), 7,57 (dd, J=8.8, 2.8 Hz, 1 H), 7.81 (d, J = 8,3 Hz, 1H). 7.86 (dd, J = 7.0, 2.3 Hz, 2H), 7.99 (dd, J = 8,6,2.1 Hz, 1H), 8.14 (d, J = 2.8 Hz, 1H), 8.26 (d, J = 2.3 Hz, 1H), 10.53 (s, 1H). | ++ |
| 70 | 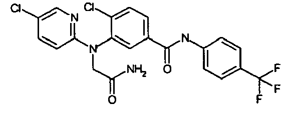 | 483.271 | (400 MHz, DMSO-d6) 4.35 (s, 2H), 6.24 (d, J = 8.8 Hz. 1H), 7.10 (s, 1H), 7.46 (s, 1H), 7.57 (dd, J = 8.8, 2.8 Hz, 1H), 7.74 (d, J = 8.8 Hz, 2H), 7.82 (d, J = 8.3 Hz. 1H). 7.96-8.03 (m, 3H), 8.14 (d. J = 2.3 Hz, 1H), 8.28 (d, J = 2.3 Hz, 1H), 10.68 (s, 1H). | ++ |
| 71 | 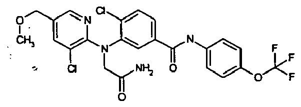 | 543.323 | (400 MHz, DMSO-d6) 3.29 (s, 3H), 4.37 (s, 2H), 4.40 (s, 2H), 6.99 (brs, 1H), 7.27 (brs, 1H), 7.36 (d, J = 8.4 Hz, 2H), 7.65-7.67 (m, 2H), 7.81-7.85 (m, 4H), 8.20 (d, J = 1.8 Hz. 1H), 10.47 (s. 1H). | ++ |
| 72 | 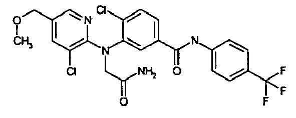 | 527.323 | (400 MHz, DMSO-d6) 3.29 (s. 3H), 4.37 (s, 2H), 4.41 (s, 2H), 6.99 (brs, 1H), 7.27 (brs, 1H), 7.67-7.73 (m, 4H), 7.84-7.88 (m, 2H), 7.95 (d, J = 8.8 Hz, 2H). 8.20 (d. J = 1.8 Hz, 1H), 10.61 (s, 1H). | ++ |
| 73 | 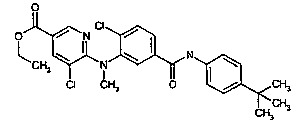 | 500.417 | (400 MHz, DMSO-d6) 1.27 (s. 9H), 1.31 (t. J = 7.0 Hz, 3H), 3.44 (s, 3H), 4.31 (q, J = 7.0 Hz, 2H), 7.36 (d, J = 8.8 Hz, 2H), 7.63 (d, J = 8.8 Hz, 2H), 7.74 (d, J = 8.3 Hz, 1H), 7,85 (d, J = 2.3 Hz. 1H), 7.93 (dd, J 8.3. 2.3 Hz, 1H). 8.04 (d, J = 2.3 Hz, 1H), 8.80 (d, J = 2.3 Hz, 1H), 10.21 (s, 1H). | + |
| 74 | 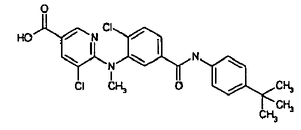 | 472.364 | (400 MHz, DMSO-d6) 1.27 (s. 9H), 3.43 (s. 3H), 7.36 (dd, J = 9.0, 2.1 Hz, 2H), 7.63 (d, J = 8.8 Hz, 2H), 7.74 (d, J = 8.3 Hz, 1H), 7.84 (d, J = 1.9 Hz. 1H). 7.93 (dd, J = 8.3, 2.3 Hz, 1H), 8.02 (d, J = 1.9 Hz, 1H), 8.78 (d, J = 1.9 Hz, 1H), 10.21 (s. 1H). 13.20 (brs, 1H). | |
| 75 | 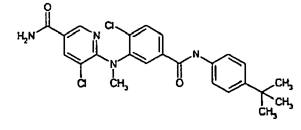 | 471.379 | (400 MHz, DMSO-d6) 1.27 (s. 9H), 3.40 (s. 3H), 7.36 (d, J = 7.0 Hz, 2H), 7.49 (brs, 1H), 7.62 (d, J = 7.0 Hz, 2H), 7.73 (d, J = 8.3 Hz, 1H), 7.79 (d, J = 1.9 Hz, 1H). 7.91 (dd, J = 8.3, 1.9 Hz, 1H), 8.02 (brs, 1H), 8.11 (d, J = 1.9 Hz, 1H), 8.77 (d, J = 1.9 Hz, 1H), 10.21 (s, 1H). | ++ |

**[Table 11]**

| Ex. | Molucular Structure | m.w. | NMR | in vitro IC50 |
|---|---|---|---|---|
| 76 | 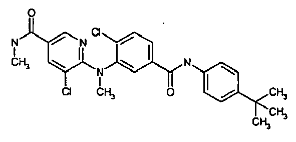 | 485.406 | (400 MHz, DMSO-d₆) δ:1.27 (s, 9H), 2.78 (d, J = 4.6 Hz, 3H), 3.40 (s, 3H), 7.36 (d, J = 8.8 Hz, 2H), 7.62 (d, J = 8.3 Hz. 2H), 7.72 (d. J = 8.3 Hz. 1H), 7.78 (d, J = 1.9 Hz. 1H), 7.91 (dd, J = 8.3. 2.3 Hz, 1H), 8.08 485.406 (d, J = 2.3 Hz. 1H), 8.49-8.50 (m, 1H), 8.74 (d, J = 9Hz, 1H) 10.21 (s, 1H), | + |
| 77 | 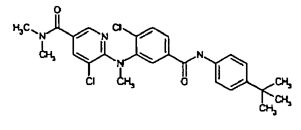 | 499.432 | (400MHz, DMSO-d6) 1.27 (s, 9H), 2.99 (s, 6H), 3.38 (s. 3H), 7.36 (d, J = 8.8 Hz, 2H), 7.63 (d, J = 8.8 Hz, 2H), 7.72 (d, J = 8.3 Hz, 1H), 7.78 (d, J = 1.9 Hz, 1H), 7.81 (d, J = 1.9 Hz, 1H), 7.89 (dd, J = 8.3. 2.3 Hz, 1H), 8.39 (d. J = 1.9 Hz. 1H). 10.22 (s, 1H). | + |
| 78 | 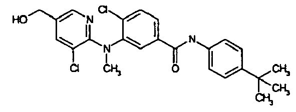 | 458.38 | (300 MHz, DMSO-d6) 1.27 (s, 9H), 3.31 (s, 3H), 4.48 (d, J = 5.5 Hz, 2H), 5.28 (t, J = 5.5 Hz, 1H), 7.35 (d, J = 9.3 Hz, 2H), 7.60-7.69 (m, 5H), 7.83 (dd, J = 8.4, 2.2 Hz, 1H), 8.24 (d, J = 1.8 Hz, 1H), 10.20 (s, 1H). | ++ |
| 79 | 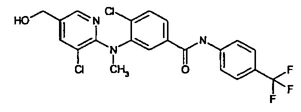 | 470.272 | (300 MHz, DMSO-d6) 3.33 (s, 3H), 4.48 (d, J = 5.5 Hz, 2H), 5.29 (t, J = 5.5 Hz, 1H), 7.65-7.73 (m, 5H), 7.85 (dd, J = 8.3, 2.0 Hz, 1H), 7.96 (d, J = 8.8 Hz, 2H), 8.25 (d, J = 1.8 Hz, 1H), 10.59 (s, 1H). | **++** |
| 80 | 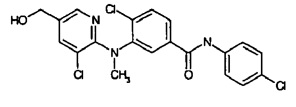 | 436.719 | (400 MHz, CDCl3) 3.40 (d, J = 2.3 Hz, 3H), 4.67 (s, 2H), 7.32 (dd, J = 9.0, 2.1 Hz, 2H), 7.51-7.61 (m, 6H). 7.69 (s, 1H), 8.23 (s, 1H). | ++ |
| 81 | 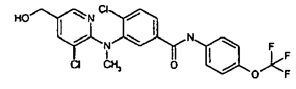 | 486.271 | (300 MHz, DMSO-d6) 3.33 (s, 3H), 4.48 (s, 2H), 5.97 (br s, 1H), 7.35 (d, J = 8.4 Hz, 2H), 7.66-7.69 (m, 3H), 7.84-7.87 (m, 3H), 8.24 (d, J = 1.8 Hz, 1H), 10.51 (s, 1H). | ++ |
| 82 | 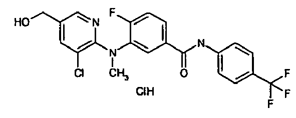 | 490.278 | (300 MHz, DMSO-d6) 3.38 (s. 3H), 4.50 (s, 2H), 5.06 (br s, 1H), 7.38 (dd, J = 11.4. 8.8 Hz, 1H), 7.71-7.75 (m. 4H), 7.80-7.84 (m, 1H), 7.99 (d, J = 8.8 Hz, 2H), 8.25 (d, J = 1.8 Hz, 1H), 10.60 (s, 1H). | ++ |
| 83 | 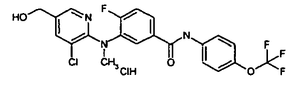 | 506.278 | (300 MHz, DMSO-d6) 3.38 (s, 3H), 4.50 (s, 2H), 5.79 (br s, 1H), 7.35-7.39 (m, 3H), 7.71-7.74 (m, 2H), 7-81-7.86 (m, 3H), 8.25 (d, J = 1.8 Hz, 1H), 10.47 (s, 1H). | ++ |

### INDUSTRIAL APPLICABILITY

The 3-aminobenzamide compound of the present invention effectively inhibits vanilloid receptor subtype 1 (VR1) activity, and therefore it is effective in the medical treatment and/or prevention of diseases such as pain, acute pain, chronic pain, neuropathic pain, rheumatoid arthritis pain, neuralgia, neuropathy, hyperalgesia, migraine, joint pain, acute postherpetic neuralgia, postherpetic neuralgia, chronic postherpetic neuralgia, postoperative pain, cancer pain, inflammatory pain, interstitial cystitis, posttraumatic neuralgia, diabetic neuropathy, neurodegenerative disease, cerebral apoplexy, ischemic symptom, nerve injury, neurogenic skin disorder, inflammatory disease, pruritus, allergic rhinitis, apoplexy, irritable bowel syndrome, asthma, chronic obstructive pulmonary disease, dermatitis, mucositis, stomach and duodenal ulcer and inflammatory bowel disease, bladder hypersensitivity, and overactive bladder type frequent urination and urinary incontinence.

## Claims

1. A 3-aminobenzamide compound represented by the following general formula [1] or a pharmaceutically acceptable salt thereof: 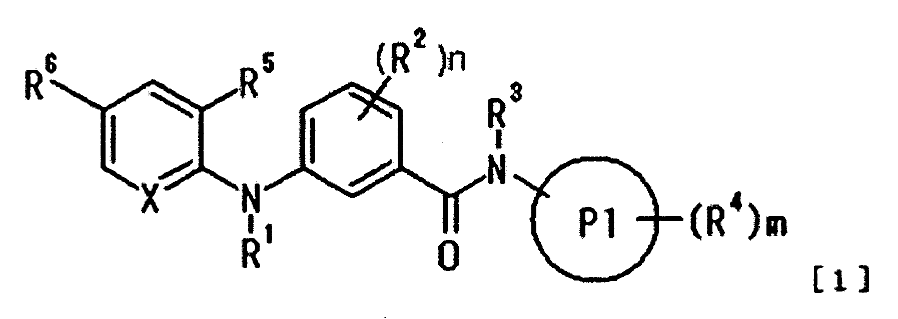 [wherein
R¹ is
a C1-6 alkyl group which may be substituted with one or more substituents, which may be the same or different, selected from the following Group A
[Group A]
1) a halogen atom,
2) a hydroxyl group,
3) a C1-6 alkoxy group,
4) a halo C1-6 alkoxy group,
5) -NR⁷R⁸ (wherein R⁷ and R⁸ are the same or different and each represents
(a) a hydrogen atom, or
(b) a C1-6 alkyl group which may be substituted with a hydroxyl group, or
(c) anitrogen-containing saturated heterocyclic group composed of a monocyclic ring formed by R⁷ and R⁸ together with the adjacent nitrogen atom),
6) -CONR⁷R⁸ (wherein R⁷ and R⁸ are the same as above),
7) -COR⁹ (wherein R⁹ is
(a) a hydrogen atom,
(b) a hydroxyl group,
(c) a C1-6 alkyl group, or
(d) a C1-6 alkoxy group),
8) -NR⁷¹COR⁹ (wherein R⁹ is the same as above and R⁷¹ is
(a) a hydrogen atom, or
(b) a C1-6 alkyl group),
9) -NR⁷¹CONR⁷R⁸ (wherein R⁷, R⁸ and R⁷¹ are the same as above),
10) -NR⁷¹SO₂R¹⁰ (wherein R⁷¹ is the same as above and R¹⁰ is a C1-6 alkyl group),
11) -SO₂R¹⁰ (wherein R¹⁰ is the same as above);
[wherein the C1-6 alkyl group, C1-6 alkoxy group, halo C1-6 alkoxy group and nitrogen-containing saturated heterocyclic group composed of a monocyclic ring in the above 1) to 11) may be further substituted with one or more substituents selected from the Group A.]
R² is
one or more substituents, which may be the same or different, selected from the following Group B
[Group B]
1) a halogen atom,
2) a hydroxyl group,
3) a C1-6 alkyl group which may be substituted with one or more substituents, which may be the same or different, selected from said Group A,
4) a C1-6 alkoxy group which may be substituted with one or more substituents, which may be the same or different, selected from said Group A,
5) a cycloalkylalkoxy group (said cycloalkylalkoxy group may be substituted with
(a) one or more substituents, which may be the same or different, selected from said Group A, or
(b) a C1-6 alkyl group which may be substituted with one or more substituents, which may be the same or different, selected from said Group A),
6) an aralkyl group (said aralkyl group may be substituted with
(a) one or more substituents, which may be the same or different, selected from said Group A, or
(b) a C1-6 alkyl group which may be substituted with one or more substituents, which may be the same or different, selected from said Group A),
7) an aralkoxy group (said aralkoxy group may be substituted with
(a) one or more substituents, which may be the same or different, selected from said Group A, or
(b) a C1-6 alkyl group which may be substituted with one or more substituents, which may be the same or different, selected from said Group A),
8) -COR¹¹ (wherein R¹¹ is
(a) a hydroxyl group,
(b) a C1-6 alkyl group which may be substituted with one or more substituents, which may be the same or different, selected from said Group A,
(c) a C1-6 alkoxy group which may be substituted with one or more substituents, which may be the same or different, selected from said Group A or a C1-6 alkyl group which may be substituted with said one or more substituents,
(d) a cycloalkyl group having 3 to 8 carbon atoms which may be substituted with one or more substituents, which may be the same or different, selected from said Group A or a C1-6 alkyl group which may be substituted with said one or more substituents,
(e) a cycloalkylalkoxy group which may be substituted with one ormore substituents, whichmaybe the same or different, selected from said Group A or a C1-6 alkyl group which may be substituted with said one or more substituents,
(f) an aralkyl group which may be substituted with one or more substituents, which may be the same or different, selected from said Group A or a C1-6 alkyl group which may be substituted with said one or more substituents,
(g) an aralkoxy group which may be substituted with one or more substituents, which may be the same or different, selected from said Group A or a C1-6 alkyl group which may be substituted with said one or more substituents,
(h) a saturated or unsaturated carbocyclic group having 3 to 14 carbon atoms which may be substituted with one or more substituents, which may be the same or different, selected from said Group A or a C1-6 alkyl group which may be substituted with said one or more substituents),
9) -NR¹²R¹³ (wherein R¹² and R¹³ are the same or different and each represents
(a) a hydrogen atom,
(b) a C1-6 alkyl group which may be substituted with one or more substituents, which may be the same or different, selected from said Group A, or
(c) a nitrogen-containing saturated heterocyclic group composed of a monocyclic ring and is formed by R¹² and R¹³ together with an adjacent nitrogen atom),
10) -CONR¹²R¹³ (wherein R¹² and R¹³ are the same as above),
11) -NR¹²¹COR¹¹ (wherein R¹²¹ is
(a) a hydrogen atom,
(b) a C1-6 alkyl group which may be substituted with one or more substituents, which may be the same or different, selected from said Group A, and R¹¹ is the same as above),
12) -NR¹²¹CONR¹²R¹³ (wherein R¹², R¹³ and R¹²¹ are the same as above),
13) -SR¹⁴ (wherein R¹⁴ is
(a) a C1-6 alkyl group which may be substituted with one or more substituents, which may be the same or different, selected from said Group A, or
(b) a cycloalkyl group having 3 to 8 carbon atoms which may be substituted with one or more substituents, which may be the same or different, selected from said Group A),
14) -SOR¹⁴ (wherein R¹⁴ is the same as above),
15) -SO₂R¹⁴ (wherein R¹⁴ is the same as above),
16) -SO₂NR¹²R¹³ (wherein R¹² and R¹³ are the same as above),
17) a saturated or unsaturated carbocyclic group having 3 to 14 carbon atoms (wherein the carbocyclic group may be substituted with
(a) one or more substituents, which may be the same or different, selected from said Group A, or
(b) a C1-6 alkyl group which may be substituted with one or more substituents, which may be the same or different, selected from said Group A),
18) a saturated or unsaturated heterocyclic group having at least one hetero atom selected from a nitrogen atom, an oxygen atom and a sulfur atom (wherein the heterocyclic group may be substituted with
(a) one or more substituents, which may be the same or different, selected from said Group A, or
(b) a C1-6 alkyl group which may be substituted with one or more substituents, which may be the same or different, selected from said Group A), and
19) an aryloxy group (wherein the aryloxy group may be substituted with
(a) one or more substituents, which may be the same or different, selected from said Group A, or
(b) a C1-6 alkyl group which may be substituted with one or more substituents, which may be the same or different, selected from said Group A);
or R¹ and R² may together form a -CH₂-CH₂-O- bond between the adjacent nitrogen atom and carbon atom;
R³ is
(1) a hydrogen atom, or
(2) a C1-6 alkyl group which may be substituted with one or more substituents, which may be the same or different, selected from said Group A;
m is an integer of 1 to 5;
n is an integer of 0, 1 to 4; and
R⁴ is one or more substituents, which may be the same or different, selected from said Group B, and
when m is two or more
(1) two R⁴ groups may together form =0, or
(2) two R⁴ groups together with a carbon atom adjacent to a P1 ring may form 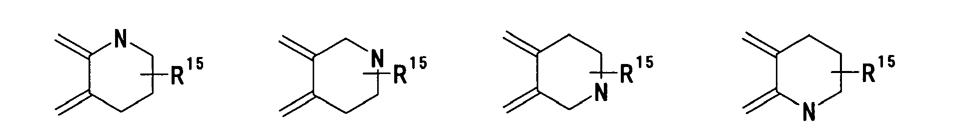 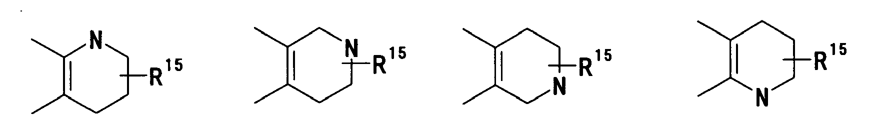 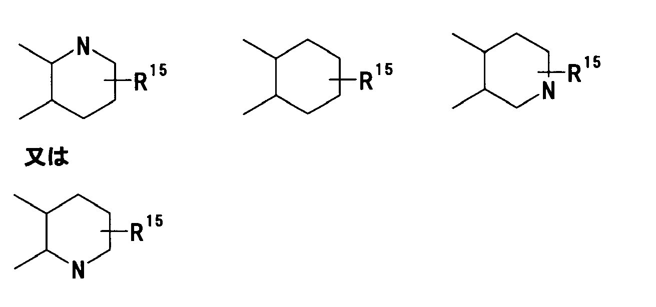
(wherein R¹⁵ is 1 to 4 substituents which are selected from said Group B);
R⁵and R⁶ are the same or different and each is
(1) a hydrogen atom, or
(2) a substituent selected from said Group B; or
R² and R⁵ may form a -0- bond together with an adjacent carbon atom;
X is
(1) CH or
(2) N; and
P1 ring is
(1) a saturated or unsaturated carbocyclic group having 3 to 14 carbon atoms, or
(2) a saturated or unsaturated heterocyclic group having at least one hetero atom selected from a nitrogen atom, an oxygen atom and a sulfur atom].

2. The 3-aminobenzamide compound or a pharmaceutically acceptable salt thereof according to Claim 1 wherein R¹ and R² do not form a -CH₂-CH₂-O- together with the adjacent nitrogen atom and carbon atom and R² and R⁵ do not form a -0- together with the adjacent carbon atom in said general formula [1].

3. The 3-aminobenzamide compound or a pharmaceutically acceptable salt thereof according to Claim 1 wherein the compound is represented by the following general formula [2] : 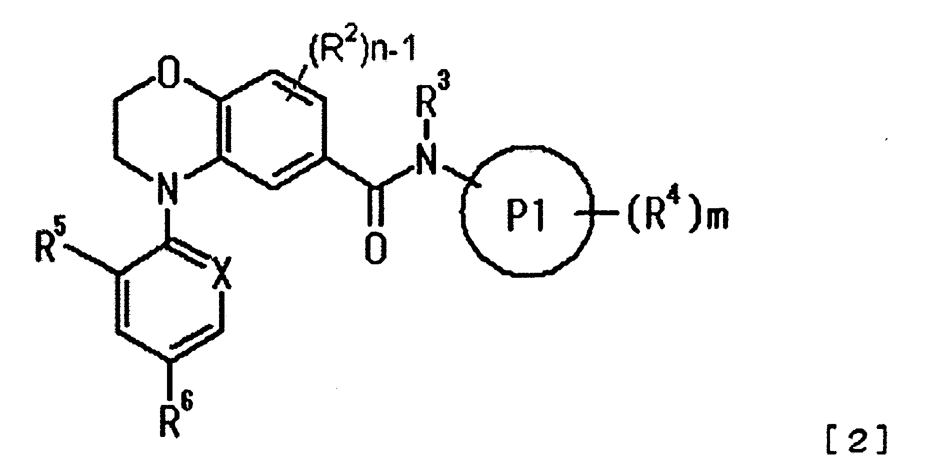 [wherein R², R³, R⁴, R⁵, R⁶, m, n, X, and P1 ring are the same as above provided that n is not 0 in this case.]

4. The 3-aminobenzamide compound or a pharmaceutically acceptable salt thereof according to Claim 1 wherein the compound is represented by the following general formula [3]: 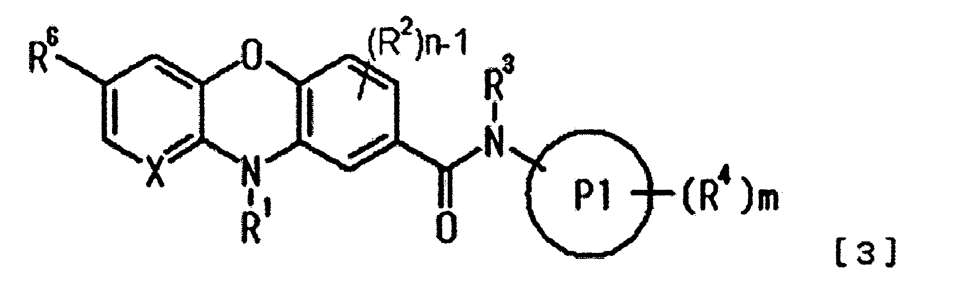 [wherein R¹, R², R³, R⁴, R⁶, m, n, X, and P1 ring are the same as above provided that n is not 0 in this case.]

5. The 3-aminobenzamide compound or a pharmaceutically acceptable salt thereof according to Claims 1 to 4 wherein the carbocyclic group or heterocyclic group of P1 ring is a monocyclic ring.

6. The 3-aminobenzamide compound or a pharmaceutically acceptable salt thereof according to Claims 1, 2, 4 or 5 wherein R¹ is a C1-6 alkyl group which may be substituted with one or more substitution groups selected from a hydroxyl group, a C1-6 alkoxy group, -CONR⁷R⁸, and -COR⁹; n is 0 or n is an integer of 1 to 4 and R² is a halogen atom, a hydroxyl group, a C1-6 alkyl group defined in said Group B, a C1-6 alkoxy group defined in said Group B, a carbocyclic group defined in said Group B, or an aralkoxy group defined in said Group B; and R³ is a hydrogen atom.

7. The 3-aminobenzamide compound or a pharmaceutically acceptable salt thereof according to Claim 6 wherein n is 0 or n is an integer of 1 to 4 and R² is a halogen atom, a hydroxyl group, a C1-6 alkyl group, a phenyl group, a phenoxy group or a C1-6 alkoxy group which may be substituted with a substituent selected from a hydroxyl group, a C1-6 alkoxy group, -CONR¹²R¹³ (wherein R¹² and R¹³ are the same as above) and -COR¹¹ (wherein R¹¹ is the same as above).

8. The 3-aminobenzamide compound or a pharmaceutically acceptable salt thereof according to Claims 1 to 7 wherein R⁴ is a halogen atom, a C1-6 alkyl group, a halo C1-6 alkyl group, a C1-6 alkoxy group, a halo C1-6 alkoxy group, or a saturated monocyclic heterocyclic group having a nitrogen atom as a hetero atom.

9. The 3-aminobenzamide compound or a pharmaceutically acceptable salt thereof according to Claim 1 wherein X is CH or N; and R¹ is a C1-6 alkyl group which may be substituted with one or more substituents selected from a hydroxyl group, a C1-6 alkoxy group, -NR⁷R⁸ (wherein R⁷ and R⁸ are the same or different and each is a hydrogen atom or a C1-6 alkyl group which may be substituted with a hydroxyl group), -CONR⁷R⁸ (wherein R⁷ and R⁸ are the same or different and each is a hydrogen atom or a C1-6 alkyl group which may be substituted with a hydroxyl group) and -COR⁹ (wherein R⁹ is a hydroxyl group or a C1-6 alkoxy group); n is an integer of 0, 1 to 2, and R² is a halogen atom, a hydroxyl group, a C1-6 alkyl group, an aryl group, an aryloxy group or a C1-6 alkoxy group (wherein the alkoxy group may be substituted with one or two substituents selected from a hydroxyl group, a C1-6 alkoxy group, -CONR⁷R⁸ (wherein R⁷ and R⁸ are the same or different and each is a hydrogen atom or a C1-6 alkyl group which may be substituted with a hydroxyl group) and -COR⁹ (wherein R⁹ is a hydroxyl group or a C1-6 alkoxy group); R³ is a hydrogen atom; m is an integer of 1 to 3; R⁴ is a halogen atom, a C1-6 alkyl group, a halo C1-6 alkyl group, a C1-6 alkoxy group, a halo C1-6 alkoxy group, -CONR¹²R¹³ (wherein R¹² and R¹³ are the same or different and each is a hydrogen atom or a C1-6 alkyl) or a saturated monocyclic heterocyclic group having a nitrogen atom as a hetero atom; R⁵ and R⁶ are the same or different and each is a hydrogen atom, a halogen atom, a C1-6 alkoxy group, a C1-6 alkyl group which may be substituted with a hydroxyl group or a C1-6 alkoxy group, a halo C1-6 alkyl group, -CONR¹²R¹³ (wherein R¹² and R¹³ are the same or different and each is a hydrogen atom or a C1-6 alkyl) or -COR¹¹ (wherein R¹¹ is a hydroxyl group or a C1-6 alkoxy group) ; and P1 ring is a phenyl group or a pyridyl group.

10. The 3-aminobenzamide compound or a pharmaceutically acceptable salt thereof according to Claim 1 selected from the following group:
(1) N-(4-tert-butylphenyl)-3-[N-(3-chloropyridin-2-yl)-N-methyl-amino]benzamide hydrochloride,
(2) N-(4-tert-butylphenyl)-3-[N-(pyridin-2-yl)-N-methyl-amino]benzamide hydrochloride,
(3) N-(4-tert-butylphenyl)-3-[N-(3-chloropyridin-2-yl)-N-ethyl-amino]benzamide,
(4) N-(4-tert-butylphenyl)-3-[N-(3-chloropyridin-2-yl)-N-methyl]amino-4-methoxybenzamide,
(5) N-(4-tert-butylphenyl)-3-[N-(3-chloropyridin-2-yl)-N-methyl]amino-4-phenoxybenzamide,
(6) N-(4-tert-butylphenyl)-3-[N-(3-chloropyridin-2-yl)-N-methyl-amino]-2-methylbenzamide,
(7) N-(4-tert-butylphenyl)-3-[N-(3-chloropyridin-2-yl)-N-methyl-amino]-4-methylbenzamide,
(8) N-(4-tert-butylphenyl)-3-[N-(3-chloropyridin-2-yl)-N-methyl-amino]-4-phenylbenzamide,
(9) N-(4-tert-butylphenyl)-3-[N-(3-chloropyridin-2-yl)-N-methyl-amino]-4-fluorobenzamide,
(10) N-(4-tert-butylphenyl)-3-[N-(3-chloropyridin-2-yl)-N-ethyl]amino-4-methoxybenzamide,
(11) N-(4-tert-butylphenyl)-3-[N-(3-chloropyridin-2-yl)-N-isopropyl]amino-4-methoxybenzamide,
(12) N-(4-tert-butylphenyl)-4-chloro-3-[N-(3-chloropyridin-2-yl)-N-methyl-amino]benzamide,
(13) N-(4-tert-butylphenyl)-4-chloro-3-[N-(pyridin-2-yl)-N-methyl-amino]benzamide,
(14) N-(4-tert-butylphenyl)-3-[N-(3-chloropyridin-2-yl)-N-methyl-amino]-4-methoxymethyloxybenzamide,
(15) N-(4-tert-butylphenyl)-3-[N-(3-chloropyridin-2-yl)-N-methyl-amino]-4-hydroxybenzamide,
(16) N-(4-tert-butylphenyl)-3-[N-(3-chloropyridin-2-yl)-N-methyl-amino]-4-isopropoxybenzamide,
(17) N-(4-tert-butylphenyl)-10-methyl-10H-benzo[b]pyrido[2,3-e][1,4]oxazine-8-carboxamide,
(18) N-(4-tert-butylphenyl)-3-[N-(3-chloropyridin-2-yl)-N-methyl-amino]-4-(2-hydroxyethyl)oxybenzamide,
(19) {4-(4-tert-butylphenyl)aminocarbonyl-2-[N-(3-chloropyridin-2-yl)-N-methyl]amino-phenoxy}acetic acid,
(20) N-(4-tert-butylphenyl)-4-carbamoylmethyloxy-3-[N-(3-chloropyridin-2-yl)-N-methyl-amino]benzamide,
(21) N-(4-tert-butylphenyl)-3-[N-(3-chloropyridin-2-yl)-N-methyl-amino]-4-(N-methylcarbamoylmethyl)oxybenzamide,
(22) N-(4-tert-butylphenyl)-3-[N-(3-chloropyridin-2-yl)-N-methyl-amino]-4-(N,N-dimethylcarbamoylmethyl)oxybenzamide,
(23) N-(4-tert-butylphenyl)-5-chloro-3-[N-(3-chloropyridin-2-yl)-N-methyl]aminobenzamide,
(24) N-(4-tert-butylphenyl)-2-chloro-5-[N-(3-chloropyridin-2-yl)-N-methyl]aminobenzamide,
(25) N-(4-tert-butylphenyl)-3-[N-(3-chloropyridin-2-yl)-N-methyl]amino-2-hydroxybenzamide,
(26) N-(4-tert-butylphenyl)-3-[N-(3-chloropyridin-2-yl)-N-methyl]amino-2-methoxybenzamide,
(27) N-(4-tert-butylphenyl)-3-[N-(3-chloropyridin-2-yl)-N-methyl-amino]-2-(2-hydroxyethyl)oxybenzamide,
(28) N-(4-tert-butylphenyl)-3-[N-(3-chloropyridin-2-yl)-N-(2-hydroxyethyl)]amino-4-methoxybenzamide,
(29) N-(4-tert-butylphenyl)-4-chloro-3-[N-(3-chloropyridin-2-yl)-N-(2-methoxyethyl)amino]benzamide,
(30) N-(4-tert-butylphenyl)-4-chloro-3-[N-(3-chloropyridin-2-yl)-N-(2-hydroxyethyl)amino]benzamide,
(31) 4-chloro-3-[N-(3-chloropyridin-2-yl)-N-(2-hydroxyethyl)amino]-N-(4-trifluoromethylphenyl)-benzamide hydrochloride,
(32) 4-chloro-3-[N-(3-chloropyridin-2-yl)-N-(2-hydroxyethyl)amino]-N-(4-trifluoromethoxyphenyl)-benzamide hydrochloride,
(33) N-(4-tert-butylphenyl)-3-[N-(2-chlorophenyl)-N-methyl-ami no]benzamide, and
(34) N-(4-tert-butylphenyl)-4-(3-chloropyridin-2-yl)-3,4-dihydro-2H-benzo[1,4]oxazine-6-carboxamide; and
(35) 4-chloro-3-[N-(3-chloropyridin-2-yl)-N-(2-hydroxyethyl)amino]-N-(4-isobutyloxyphenyl)-benzamide hydrochloride,
(36) 4-chloro-N-(4-chlorophenyl)-3-[N-(3-chloropyridin-2-yl)-N-(2-hydroxyethyl)amino]-benzamide,
(37) 4-chloro-3-[N-(3-chloropyridin-2-yl)-N-(2-hydroxyethyl)amino]-N-(4-isopropylphenyl)-benzamide,
(38) 4-chloro-3-[N-(3-chloropyridin-2-yl)-N-(2-hydroxyethyl)amino]-N-(1-methyl-1,2,3,4-tetrahydroisoquinolin-7-yl)-benzamide,
(39) 4-chloro-3-[N-(3-chloropyridin-2-yl)-N-(2-hydroxyethyl)amino]-N-(4-chloro-3-trifluoromethylphenyl)-benzamide,
(40) 4-chloro-3-[N-(3-chloropyridin-2-yl)-N-(2-hydroxyethyl)amino]-N-(2-trifluoromethylpyridin-5-yl)-benzamide,
(41) 4-chloro-3-[N-(3-chloropyridin-2-yl)-N-(2-hydroxyethyl)amino]-N-(4-isopropyloxyphenyl)-benzamide,
(42) 4-chloro-3-[N-(3-chloropyridin-2-yl)-N-(2-hydroxyethyl)amino]-N-(5-trifluoromethylpyridin-2-yl)-benzamide,
(43) 4-chloro-3-[N-(3-chloropyridin-2-yl)-N-(2-hydroxyethyl)amino]-N-(3-fluoro-4-piperidinylphenyl)-benzamide,
(44) 4-chloro-3-[N-(3-chloropyridin-2-yl)-N-(2-hydroxyethyl)amino]-N-(4-isobutyloxyphenyl)-benzamide,
(45) 4-chloro-3-[N-(3-chloropyridin-2-yl)-N-(3-hydroxypropyl)amino]-N-(4-tert-butylphenyl)-benzamide,
(46) N-(3-chloropyridin-2-yl)-N-{2-chloro-5-[N-(4-tert-butylphenyl)carbamoyl]phenyl}-aminoacetic acid,
(47) 4-chloro-3-[N-(3-chloropyridin-2-yl)-N-(carbamoylmethyl)amino]-N-(4-tert-butylphenyl)-benzamide,
(48) 4-chloro-3-[N-(3-chloropyridin-2-yl)-N-(N-methylcarbamoylmethyl)amino]-N-(4-tert-butylphenyl)-benzamide,
(49) 4-chloro-3-[N-(3-chloropyridin-2-yl)-N-(N,N-dimethylcarbamoylmethyl)amino]-N-(4-tert-butylphenyl)-benzamide,
(50) 3-[N-(3-chloropyridin-2-yl)-N-(2-hydroxyethyl)amino]-N-(4-isobutyloxyphenyl)-4-methoxy-benzamide,
(51) N-(4-isobutyloxyphenyl)-4-methoxy-3-[N-(5-methylpyridin-2-yl)-N-(2-hydroxyethyl)amino]-benzamide,
(52) 3-[N-(3-chloropyridin-2-yl)-N-(2-hydroxyethyl)amino]-N-(4-isobutyloxyphenyl)-benzamide,
(53) N-(4-tert-butylphenyl)-3-[N-(3-chloropyridin-2-yl)-N-(2-hydroxyethyl)amino]-benzamide,
(54) 3-[N-(3-chloropyridin-2-yl)-N-(2-hydroxyethyl)amino]-N-(4-trifluoromethylphenyl)-benzamide,
(55) 4-chloro-3-[N-(3-chloropyridin-2-yl)-N-(2-hydroxyethyl)amino]-N-(4-isobutyloxy-3-methylcarbamoylphenyl)-benzamide,
(56) 4-chloro-3-[N-(3-chloropyridin-2-yl)-N-(2-hydroxyethyl)amino]-N-(3-dimethylcarbamoyl-4-isobutyloxyphenyl)-benzamide,
(57) N-(4-tert-butylphenyl)-4-chloro-3-{N-(3-chloropyridin-2-yl)-N-[N-(2-hydroxyethyl)carbamoylmethyl]amino}-benzamide,
(58) 3-[N-(2-aminoethyl)-N-(3-chloropyridin-2-yl)amino]-4-chloro-N-(4-tert-butylphenyl)-benzamide,
(59) 4-chloro-3-[N-(3-chloropyridin-2-yl)-N-(carbamoylmethyl)amino]-N-(4-trifluoromethylphenyl)-benzamide,
(60) 4-chloro-3-[N-(3-chloropyridin-2-yl)-N-(carbamoylmethyl)amino]-N-(4-trifluoromethoxyphenyl)-benzamide,
(61) 4-chloro-3-[N-(3-chloropyridin-2-yl)-N-(carbamoylmethyl)amino]-N-(4-isobutyloxyphenyl)-benzamide,
(62) 4-chloro-3-[N-(3-chloropyridin-2-yl)-N-(carbamoylmethyl)amino]-N-(3-fluoro-4-piperidinophenyl)-benzamide,
(63) 3-[N-(3-chloropyridin-2-yl)-N-(carbamoylmethyl)amino]-4-methyl-N-(4-trifluoromethylphenyl)-benzamide,
(64) 3-[N-(3-chloropyridin-2-yl)-N-(carbamoylmethyl)amino]-4-methyl-N-(4-trifluoromethoxyphenyl)-benzamide,
(65) 3-[N-(3-chloropyridin-2-yl)-N-(carbamoylmethyl)amino]-N-(4-isobutyloxyphenyl)-4-methoxy-benzamide,
(66) 4-chloro-3-[N-(3-chloropyridin-2-yl)-N-(N-methylcarbamoylmethyl)amino]-N-(4-trifluoromethylphenyl)-benzamide,
(67) 4-chloro-3-[N-(3-chloro-5-trifluoromethylpyridin-2-yl)-N-(carbamoylmethyl)amino]-N-(4-trifluoromethoxyphenyl)-benzamide,
(68) 4-chloro-3-[N-(3-chloro-5-trifluoromethylpyridin-2-yl)-N-(carbamoylmethyl)amino]-N-(4-trifluoromethylphenyl)-benzamide,
(69) 4-chloro-3-[N-(3-chloropyridin-2-yl)-N-(carbamoylmethyl)amino]-N-(trifluoromethoxyphenyl)-benzamide,
(70) 4-chloro-3-[N-(3-chloropyridin-2-yl)-N-(carbamoylmethyl)amino]-N-(trifluoromethylphenyl)-benzamide,
(71) 4-chloro-3-[N-(3-chloro-5-methoxymethylpyridin-2-yl)-N-(carbamoylmethyl)amino]-N-(trifluoromethoxyphenyl)-benzamide,
(72) 4-chloro-3-[N-(3-chloro-5-methoxymethylpyridin-2-yl)-N-(carbamoylmethyl)amino]-N-(trifluoromethylphenyl)-benzamide
(73) ethyl 6-{[5-(4-tert-butylphenylcarbamoyl)-2-chlorophenyl]-methyl-amino}-5-chloronicotinate,
(74) 6-{[5-(4-tert-butylphenylcarbamoyl)-2-chlorophenyl]-methyl-amino}-5-chloronicotinic acid,
(75) 6-{[5-(4-tert-butylphenylcarbamoyl)-2-chlorophenyl]-methyl-amino}-5-chloronicotinamide,
(76) 6-{[5-(4-tert-butylphenylcarbamoyl)-2-chlorophenyl]-methyl-amino}-5-chloro-N-methylnicotinamide,
(77) 6-{[5-(4-tert-butylphenylcarbamoyl)-2-chlorophenyl]-methyl-amino}-5-chloro-N,N-dimethylnicotinamide,
(78) N-(4-tert-butylphenyl)-4-chloro-3-[N-(3-chloro-5-hydroxymethylpyridin-2-yl)-N-methyl-amino]benzamide,
(79) 4-chloro-3-[N-(3-chloro-5-hydroxymethylpyridin-2-yl)-N-methyl-amino]-N-(4-trifluoromethylphenyl)-benzamide,
(80) 4-chloro-3-[N-(3-chloro-5-hydroxymethylpyridin-2-yl)-N-methyl-amino]-N-(4-chlorophenyl)-benzamide,
(81) 4-chloro-3-[N-(3-chloro-5-hydroxymethylpyridin-2-yl)-N-methyl-amino]-N-(4-trifluoromethoxyphenyl)-benzamide,
(82) 3-[N-(3-chloro-5-hydroxymethylpyridin-2-yl)-N-methyl-amino]-4-fluoro-N-(4-trifluoromethylphenyl)-benzamide, and
(83) 3-[N-(3-chloro-5-hydroxymethylpyridin-2-yl)-N-methyl-amino]-4-fluoro-N-(4-trifluoromethoxyphenyl)-benzamide.

11. A pharmaceutical composition comprising a 3-aminobenzamide compound or a pharmaceutically acceptable salt thereof according to any of Claims 1 to 10 and a pharmaceutically acceptable carrier.

12. A pharmaceutical composition comprising a 3-aminobenzamide compound or a pharmaceutically acceptable salt thereof according to any of Claims 1 to 10 and a pharmaceutically acceptable carrier for treating and/or preventing a disease selected from algia, acute pain, chronic pain, neuropathic pain, rheumatoid arthritis pain, neuralgia, neuropathy, hyperalgesia, migraine, joint pain, acute postherpetic neuralgia, postherpetic neuralgia, chronic postherpetic neuralgia, postoperative pain, cancer pain, inflammatory pain, interstitial cystitis, posttraumatic neuralgia, diabetic neuropathy, neurodegenerative disease, brain apoplexy, ischemic symptom, nerve injury, neurogenic skin disorders, inflammatory disease, pruritus, allergic rhinitis, apoplexy, irritable bowel syndrome, asthma, chronic obstructive pulmonary disease, dermatitis, mucositis, stomach and duodenal ulcer and inflammatory bowel disease, bladder hypersensitivity, and overactive bladder type frequent urination and urinary incontinence.

13. A pharmaceutical composition for treating and/or preventing pain comprising a 3-aminobenzamide compound or a pharmaceutically acceptable salt thereof according to any of Claims 1 to 10 and a pharmaceutically acceptable carrier.

14. The pharmaceutical composition according to Claim 13 wherein the pain is algia, acute pain, chronic pain, neuropathic pain, rheumatoid arthritis pain, neuralgia, neuropathy, hyperalgesia, migraine, joint pain, acute postherpetic neuralgia, postherpetic neuralgia, chronic postherpetic neuralgia, postoperative pain, cancer pain, inflammatory pain, interstitial cystitis, posttraumatic neuralgia, diabetic neuropathy or neurodegenerative disease.

15. An inhibitor of vanilloid receptor subtype 1 (VR1) activity comprising a 3-aminobenzamide compound or a pharmaceutically acceptable salt thereof according to any of Claims 1 to 10 and a pharmaceutically acceptable carrier.

16. A method for treating and/or preventing a disease selected from algia, acute pain, chronic pain, neuropathic pain, rheumatoid arthritis pain, neuralgia, neuropathy, hyperalgesia, migraine, joint pain, acute postherpetic neuralgia, postherpetic neuralgia, chronic postherpetic neuralgia, postoperative pain, cancer pain, inflammatory pain, interstitial cystitis, posttraumatic neuralgia, diabetic neuropathy, neurodegenerative disease, brain apoplexy, ischemic symptom, nerve injury, neurogenic skin disorders, inflammatory disease, pruritus, allergic rhinitis, apoplexy, irritable bowel syndrome, asthma, chronic obstructive pulmonary disease, dermatitis, mucositis, stomach and duodenal ulcer and inflammatory bowel disease, bladder hypersensitivity, and overactive bladder type frequent urination and urinary incontinence **characterized in that** the method comprises administering a pharmaceutically effective amount of a 3-aminobenzamide compound or a pharmaceutically acceptable salt thereof according to any of Claims 1 to 10.

17. A method for treating and/or preventing pain **characterized in that** the method comprises administering a pharmaceutically effective amount of a 3-aminobenzamide compound or a pharmaceutically acceptable salt thereof according to any of Claims 1 to 10.

18. The treating and/or preventing method according to Claim 17 wherein the pain is algia, acute pain, chronic pain, neuropathic pain, rheumatoid arthritis pain, neuralgia, neuropathy, hyperalgesia, migraine, joint pain, acute postherpetic neuralgia, postherpetic neuralgia, chronic postherpetic neuralgia, postoperative pain, cancer pain, inflammatory pain, interstitial cystitis, posttraumatic neuralgia, diabetic neuropathy or neurodegenerative disease.

19. A commercial package comprising a pharmaceutical composition according to Claims 11 to 14 and written instructions about this pharmaceutical composition stating that said composition can be used or should be used for treating and/or preventing a disease selected from algia, pain, acute pain, chronic pain, neuropathic pain, rheumatoid arthritis pain, neuralgia, neuropathy, hyperalgesia, migraine, joint pain, acute postherpetic neuralgia, postherpetic neuralgia, chronic postherpetic neuralgia, postoperative pain, cancer pain, inflammatory pain, interstitial cystitis, posttraumatic neuralgia, diabetic neuropathy, neurodegenerative disease, brain apoplexy, ischemic symptom, nerve injury, neurogenic skin disorders, inflammatory disease, pruritus, allergic rhinitis, apoplexy, irritable bowel syndrome, asthma, chronic obstructive pulmonary disease, dermatitis, mucositis, stomach and duodenal ulcer and inflammatory bowel disease, bladder hypersensitivity, and overactive bladder type frequent urination and urinary incontinence.

20. Use of a 3-aminobenzamide compound or a pharmaceutically acceptable salt thereof according to any of Claims 1 to 10 for preparing a pharmaceutical composition according to Claim 12 for treating and/or preventing a disease selected from algia, acute pain, chronic pain, neuropathic pain, rheumatoid arthritis pain, neuralgia, neuropathy, hyperalgesia, migraine, joint pain, acute postherpetic neuralgia, postherpetic neuralgia, chronic postherpetic neuralgia, postoperative pain, cancer pain, inflammatory pain, interstitial cystitis, posttraumatic neuralgia, diabetic neuropathy, neurodegenerative disease, brain apoplexy, ischemic symptom, nerve injury, neurogenic skin disorders, inflammatory disease, pruritus, allergic rhinitis, apoplexy, irritable bowel syndrome, asthma, chronic obstructive pulmonary disease, dermatitis, mucositis, stomach and duodenal ulcer and inflammatory bowel disease, bladder hypersensitivity, and overactive bladder type frequent urination and urinary incontinence.

21. Use of a 3-aminobenzamide compound or a pharmaceutically acceptable salt thereof according to any of Claims 1 to 10 for preparing a pharmaceutical composition for treating and/or preventing pain according to Claim 13.

22. The use of a 3-aminobenzamide compound or a pharmaceutically acceptable salt thereof according to Claim 21 wherein the pain is algia, acute pain, chronic pain, neuropathic pain, rheumatoid arthritis pain, neuralgia, neuropathy, hyperalgesia, migraine, joint pain, acute postherpetic neuralgia, postherpetic neuralgia, chronic postherpetic neuralgia, postoperative pain, cancer pain, inflammatory pain, interstitial cystitis, posttraumatic neuralgia, diabetic neuropathy or neurodegenerative disease.

23. A pharmaceutical composition comprising a 3-aminobenzamide compound or a pharmaceutically acceptable salt thereof according to any of Claims 1 to 10 and a pharmaceutically acceptable carrier wherein the composition is to be used in combination with one or more agents selected from the group consisting of an anti-virus agent, an antidepressant, an anticonvulsant, an antiarrhythmic drug, a local analgesic, an anesthetic drug, anN-methyl-D-aspartate receptor antagonist, adrenal-cortex steroid, a nerve block, a nonsteroidal antiinflammatory analgesic, narcotics, an antagonist analgesic, ?₂ adrenaline-receptor agonist, a medicine for external application, a calcium channel antagonist, and a potassium channel opening drug.

24. Use of a 3-aminobenzamide compound or a pharmaceutically acceptable salt thereof according to any of Claims 1 to 10 for preparing a pharmaceutical composition according to Claim 23.

25. A method for treating and/or preventing a disease selected from algia, acute pain, chronic pain, neuropathic pain, rheumatoid arthritis pain, neuralgia, neuropathy, hyperalgesia, migraine, joint pain, acute postherpetic neuralgia, postherpetic neuralgia, chronic postherpetic neuralgia, postoperative pain, cancer pain, inflammatory pain, posttraumatic neuralgia, diabetic neuropathy, neurodegenerative disease, brain apoplexy, ischemic symptom, nerve injury, neurogenic skin disorders, inflammatory disease, interstitial cystitis, pruritus, allergic rhinitis, apoplexy, irritable bowel syndrome, asthma, chronic obstructive pulmonary disease, dermatitis, mucositis, stomach and duodenal ulcer and inflammatory bowel disease, bladder hypersensitivity, and overactive bladder type frequent urination and urinary incontinence **characterized in that** one or more agents selected from the group consisting of an anti-virus agent, an antidepressant, an anticonvulsant, an antiarrhythmic drug, a local analgesic, an anesthetic drug, an N-methyl-D-aspartate receptor antagonist, adrenal-cortex steroid, a nerve block, a nonsteroidal antiinflammatory analgesic, narcotics, an antagonist analgesic, ?₂ adrenaline-receptor agonist, a medicine for external application, a calcium channel antagonist, and a potassium channel opening drug are used in combination with a pharmaceutically effective amount of an inhibitor of vanilloid receptor subtype 1 (VR1) activity.

26. The medical treatment method and/or the prevention method according to Claim 25 wherein the inhibitor of vanilloid receptor 1 type (VR1) activity is a 3-aminobenzamide compound or a pharmaceutically acceptable salt thereof according to any of Claims 1 to 10.

27. A method for treating and/or preventing pain **characterized in that** the method uses administration of an inhibitor of vanilloid receptor subtype 1 (VR1) activity in combination with stimulation-produced analgesia selected from acupuncture, transcutaneous electroacupuncture stimulation therapy, transcutaneous electrical nerve stimulation therapy, silver spike point (SSP) therapy, peripheral nerve stimulation therapy, spinal cord electrical stimulation therapy, electroconvulsive therapy, laser therapy and low frequency therapy.

28. The treating and/or preventing method according to Claim 27 wherein the inhibitor of vanilloid receptor subtype 1 (VR1) activity is a 3-aminobenzamide compound or a pharmaceutically acceptable salt thereof according to any of Claims 1 to 10.

29. A method for treating and/or preventing postoperative pain **characterized in that** an inhibitor of vanilloid receptor subtype 1 (VR1) activity is administered after performing a surgical operation selected from cicatrectomy, nerve freezing solidification, peripheral nerve excision, spinal cord dorsal root excision, sympathectomy, spinal cord dorsal root entry zone destruction, cordotomy, and frontal lobe excision.

30. The treating and/or preventing method according to Claim 29 wherein the inhibitor of vanilloid receptor subtype 1 (VR1) activity is a 3-aminobenzamide compound or a pharmaceutically acceptable salt thereof according to any of Claims 1 to 10.
